(19) Europäisches Patentamt
European Patent Office
Office européen des brevets

(11) Numéro de publication: **0 102 291**
**B1**

(12) # FASCICULE DE BREVET EUROPEEN

(45) Date de publication du fascicule du brevet: **08.01.86**

(51) Int. Cl.⁴: **C 07 D 501/20**, A 61 K 31/545

(21) Numéro de dépôt: **83401653.7**

(22) Date de dépôt: **12.08.83**

(54) **Nouveaux dérivés de la céphalosporine, leur préparation et les médicaments qui les contiennent.**

(30) Priorité: **13.08.82 FR 8214092**

(43) Date de publication de la demande: **07.03.84 Bulletin 84/10**

(45) Mention de la délivrance du brevet: **08.01.86 Bulletin 86/2**

(84) Etats contractants désignés: **AT BE CH DE FR GB IT LI LU NL SE**

(56) Documents cités:
**FR - A - 2 206 085**
**FR - A - 2 381 050**
**FR - A - 2 381 051**

(73) Titulaire: **RHONE-POULENC SANTE, Les Miroirs 18 Avenue d'Alsace, F-92400 Courbevoie Cedex (FR)**

(72) Inventeur: **Berger, Christian, 26 chemin de la Forestière Tour 3, F-69130 Ecully (FR)**
Inventeur: **Farge, Daniel, 30 rue des Pins Sylvestres, F-94320 Thiais (FR)**
Inventeur: **Moutonnier, Claude, 3 rue Auguste Rodin, F-92350 Le Plessis-Robinson (FR)**
Inventeur: **Peyronel, Jean-Francois, 36 parc d'Ardenay, F-91120 Palaiseau (FR)**
Inventeur: **Plau, Bernard, 82 avenue Carnot, F-94100 Saint-Maur (FR)**

(74) Mandataire: **Gaumont, Robert et al, RHONE-POULENC RECHERCHES Service Brevets Pharma 25, Quai Paul Doumer, F-92408 Courbevoie Cedex (FR)**

EP 0 102 291 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention concerne de nouveaux dérivés de thiazolylcéphalosporines de formule générale:

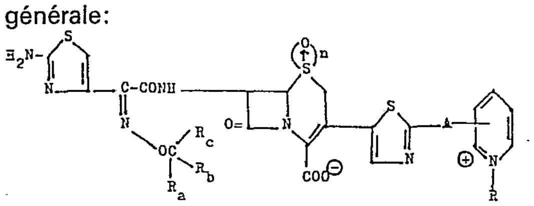

(I)

leurs sels, leur préparation et les médicaments qui les contiennent.

La demande de brevet FR-A N° 2206085 décrit de nombreuses hétérocyclylcéphalosporines et notamment des thiazolyl-3 céphalosporines.

La publication de T. Sugawara et coll., «Chem. Pharm. Bull.», *28*, (7), 2116 (1980) fait état de thiadiazolylcéphalosporines dont l'intérêt principal est une bonne activité sur germes gram-négatifs.

Les demandes de brevets FR-A N$^{os}$ 2381050 et 2381051 décrivent des thiadiazolyl-3 céphalosporines actives sur germes gram-positifs et gram-négatifs.

Dans la formule générale I, le symbole A représente une liaison simple ou un radical bivalent choisi parmi $-CH_2-$, $-NH-$ ou $-NHCO-$ fixé en position -3 ou -4 du radical pyridinio, le symbole R représente un radical méthyle carboxyméthyle, carbamoylméthyle, benzyle ou allyle et les symboles $R_a$, $R_b$ et $R_c$ représentent chacun un atome d'hydrogène, ou bien le symbole $R_a$ représente un radical carboxy et les symboles $R_b$ et $R_c$ sont identiques ou différents et représentent des atomes d'hydrogène, ou des radicaux alcoyle contenant 1 à 4 atomes de carbone, ou forment ensemble un radical alcoylène contenant 2 à 5 atomes de carbone et n est égal à 0 ou 1.

Il est entendu que les produits de formule générale I existent sous forme de sel interne (l'un des groupements carboxy présent dans la molécule se trouvant à l'état de radical carboxylato), ou sous forme de solvant acide de cette bétaïne, et que toutes ces formes entrent dans le cadre de la présente invention.

Il est également entendu que dans la formule générale I et dans les formules générales employées ci-après, le groupement $-O-C-R_aR_bR_c$ se trouve en position syn.

De plus, lorsque dans la formule générale I, les symboles $R_b$ $R_c$ sont différents, il existe des diastéréo-isomères; il est entendu que ces formes isomères et leurs mélanges entrent dans le cadre de la présente invention.

A. Selon l'invention, les produits de formule générale I peuvent être préparés par action d'un acide de formule générale :

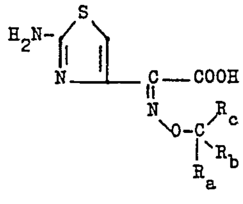

(II)

dans laquelle $R_a$, $R_b$ et $R_c$ sont définis comme précédemment, et dont la fonction amine et le cas échéant le radical carboxy représenté par $R_a$ sont préalablement protégés, ou par action d'un dérivé réactif de cet acide, sur une amino-7 céphalosporine ou sur un de ses sels, de formule générale:

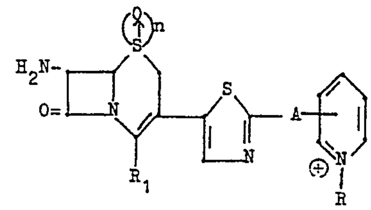

(III)

dans laquelle les symboles A et n sont définis comme précédemment, le symbole R est défini comme précédemment ou représente un radical carboxyméthyle protégé, et le symbole $R_1$ représente un radical carboxylato ou un radical carboxy libre ou protégé (étant entendu que lorsque $R_1$ est autre que carboxylato le produit de formule générale III se trouve à l'état d'halogénure ou de sulfonate), puis on réduit éventuellement le sulfoxyde et élimine les radicaux protecteurs.

La fonction amine de l'acide de formule générale II peut être protégée par tout groupement facilement éliminable utilisé en chimie peptidique, dont la mise en place et l'élimination n'affectent pas le reste de la molécule.

On peut utiliser notamment des groupements tels que t-butoxycarbonyle, vinyloxycarbonyle, trichloro-2,2,2 éthoxycarbonyle, chloracétyle, trichloracétyle, trifluoracétyle, trityle, benzyle, benzyloxycarbonyle, p-nitrobenzyloxycarbonyle, p-méthoxybenzyloxycarbonyle ou formyle, ou encore un radical diphénylphosphinoyle ou un radical tel que défini ci-après par la formule générale XIV qui peuvent être introduits par application de la méthode décrite par A. Morimoto et coll., «J. Chem. Soc. Perkin I», 1109 (1980).

Le cas échéant le radical carboxy représenté par $R_a$ et le radical carboxy contenu par R peuvent être protégés par des groupements protecteurs d'acide facilement éliminables, par exemple t-butyle, trichloro-2,2,2 éthyle, benzhydryle, p-nitrobenzyle ou p-méthoxybenzyle.

Lorsque le symbole $R_1$ dans la formule générale III représente un radical carboxy protégé, le radical protecteur peut être par exemple un groupement tel que cité ci-dessus.

Lorsque, dans la formule générale III, $R_1$ est autre que carboxylato, l'halogénure ou le sulfonate utilisés peuvent être par exemple l'iodure, le bromure ou le chlorure, un alcoylsulfonate, dont la partie alcoyle qui contient 1 à 4 atomes de carbone peut être substituée par un ou plusieurs atomes d'halogène, un phénylsulfonate, dont le radical phényle peut être éventuellement substitué par un ou plusieurs substituants choisis parmi les atomes d'halogène ou les radicaux alcoyle ou nitro.

Par ailleurs, lorsque l'on fait réagir un sel de l'amino-7 céphalosporine de formule générale III, on utilise par exemple un chlorhydrate, un bromhydrate, un iodhydrate ou un sulfonate défini comme ci-dessus.

Lorsque l'on utilise le produit de formule générale II sous sa forme acide, on effectue la condensation sur l'amino-7 céphalosporine de formule générale III dans laquelle $R_1$ est un radical carboxy protégé, ou un radical carboxylato, en présence d'un agent de condensation tel qu'un carbodiimide (par exemple dicyclohexylcarbodiimide), le N,N'-carbonyldiimidazole ou l'éthoxy-2 éthoxycarbonyl-1 dihydro-1,2 quinoléine, à une température comprise entre −20 et 40° C, puis on élimine le cas échéant les groupements protecteurs.

Lorsque l'on veut utiliser un dérivé réactif de l'acide de formule générale II, il est possible de mettre en œuvre l'anhydride, un anhydride mixte, ou un ester réactif de formule générale:

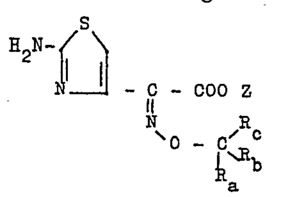

(IV)

dans laquelle, $R_a$, $R_b$ et $R_c$ étant définis comme ci-dessus, Z représente un radical succinimido, benzotriazolyle-1, nitro-4 phényle, dinitro-2,4 phényle, pentachlorophényle ou phtalimido.

Il est généralement avantageux d'utiliser un halogénure d'acide. Dans ce dernier cas, on peut faire réagir le chlorhydrate du chlorure d'acide.

Lorsque l'on met en œuvre l'anhydride, un anhydride mixte ou un halogénure d'acide (qui peuvent être préparés *in situ*), on effectue la condensation dans un solvant organique inerte tel qu'un éther (par exemple tétrahydrofuranne ou dioxanne), un solvant chloré (par exemple chloroforme ou chlorure de méthylène), un amide (par exemple diméthylformamide ou diméthylacétamide) ou une cétone (par exemple acétone) ou dans des mélanges des solvants ci-dessus, en présence d'un accepteur d'acide tel qu'un époxyde (par exemple l'oxyde de propylène) ou tel qu'une base organique azotée comme la pyridine, la diméthylaminopyridine, la N-méthylmorpholine ou une trialcoylamine (par exemple triéthylamine) ou en présence d'un agent de silylation tel que le bistriméthylsilylacétamide, ou bien dans un milieu hydroorganique en présence d'un agent alcalin de condensation tel que le bicarbonate de sodium, et l'on opère à une température comprise entre −40 et 40° C, puis on remplace les groupements protecteurs par des atomes d'hydrogène.

Lorsque l'on met en œuvre un ester réactif de formule générale IV, on opère généralement en présence d'une amine tertiaire (par exemple triéthalamine) ou d'un agent de condensation tel que cité précédemment dans un solvant organique tel que le diméthylformamide à une température comprise entre 0 et 80° C, puis on remplace les groupements protecteurs par des atomes d'hydrogène.

Le cas échéant, la réduction du sulfoxyde s'effectue dans les conditions décrites dans la demande de brevet DE-A N° 2637176.

L'élimination des groupements protecteurs d'acide peut s'effectuer par exemple:

– lorsqu'il s'agit d'un groupement t-butyle, p-méthoxybenzyle ou benzhydryle: par traitement en milieu acide, dans les conditions décrites ci-après pour l'élimination du radical trityle protecteur d'amino. Dans le cas du radical benzhydryle, on peut opérer en présence d'anisole ou par traitement par le chlorure d'aluminium dans les conditions décrites par T. Tsuji et coll., «Tet. Lett.», 30, 2793 (1979);

– lorsqu'il s'agit d'un groupement trichloro-2,2,2 éthyle ou p-nitrobenzyle: par réduction (notamment traitement par le zinc dans l'acide acétique, ou lorsqu'il s'agit du groupement p-nitrobenzyle par hydrogénolyse).

L'élimination des groupements protecteurs d'amine peut s'effectuer par exemple:

– lorsqu'il s'agit d'un radical t-butoxycarbonyle, vinyloxycarbonyle, trityle, p-méthoxybenzyloxycarbonyle ou formyle: par traitement en milieu acide. De préférence on utilise l'acide trifluoracétique en opérant à une température comprise entre 0 et 20° C, ou bien on utilise l'acide formique, phosphorique ou polyphosphorique anhydres ou aqueux à une température comprise entre 20 et 60° C, ou encore l'acide paratoluènesulfonique ou méthanesulfonique dans l'acétone ou l'acétonitrile, à une température comprise entre 20° C et la température de reflux du mélange réactionnel. Dans ces conditions le produit de formule générale I peut être obtenu sous forme de trifluoracétate, de solvate avec l'acide formique, de phosphate, de méthanesulfonate ou de paratoluènesulfonate, dont on peut libérer la fonction amine par toute méthode connue en soi pour obtenir une amine à partir de l'un de ses sels sans toucher au reste de la molécule. On opère notamment par mise en contact avec une résine échangeuse d'ions ou par action d'une base organique;

– lorsqu'il s'agit d'un radical trichloro-2,2,2 éthoxycarbonyle ou p-nitrobenzyloxycarbonyle ou d'un radical de formule générale XIV dans laquelle R' est trichloro-2,2,2 éthyle ou nitrobenzyle: par réduction (notamment traitement par le zinc dans l'acide acétique);

– lorsqu'il s'agit d'un radical chloracétyle ou trichloracétyle: par application de la méthode décrite dans le brevet FR-B N° 2243199;

– lorsqu'il s'agit d'un radical benzyle ou benzyloxycarbonyle: par hydrogénation catalytique;

– lorsqu'il s'agit d'un radical trifluoracétyle: par traitement en milieu basique;

– lorsqu'il s'agit d'un radical diphénylphosphinoyle: selon la méthode décrite par P. Haake et coll., «J. Am. Chem. Soc.», *95*, 8073 (1973);

– lorsqu'il s'agit d'un radical de formule générale XIV: selon la méthode décrite dans le brevet BE-A N° 833619.

B. Selon l'invention, les produits de formule générale I peuvent aussi être obtenus par action d'un halogénure ou d'un sulfonate de structure R−X dans laquelle R est défini comme précédemment ou représente un radical carboxyméthyle protégé, et le symbole X représente un atome d'halogène

choisi parmi l'iode, le brome et le chlore ou un radical alcoylsulfonyloxy, dont la partie alcoyle qui contient 1 à 4 atomes de carbone peut être substituée par un ou plusieurs atomes d'halogène, ou phénylsulfonyloxy dont le radical phényle peut être éventuellement substitué par un ou plusieurs substituants choisis parmi les atomes d'halogène ou les radicaux alcoyle ou nitro, sur un dérivé de céphalosporine de formule générale :

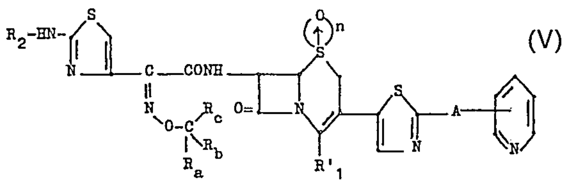

(V)

dans laquelle A, $R_a$, $R_b$, $R_c$ et n sont définis comme précédemment, étant entendu que lorsque $R_a$ est un radical carboxy il peut être libre ou protégé, $R'_1$ est un radical carboxy libre ou protégé et $R_2$ est un atome d'hydrogène ou un radical protecteur d'amino, puis éventuellement on réduit le sulfoxyde obtenu et élimine les radicaux protecteurs.

Les radicaux protecteurs d'amino représentés par $R_2$ peuvent être choisis parmi les groupements facilement éliminables utilisés en chimie peptidique, par exemple tels que définis précédemment.

Le cas échéant, les radicaux carboxy sont protégés par des groupements protecteurs tels que définis précédemment.

La réaction s'effectue généralement dans un solvant organique tel qu'un amide (par exemple diméthylformamide, hexaméthylphosphorotriamide ou diméthylacétamide), un nitrile (acétonitrile par exemple), une cétone (acétone par exemple) ou un dérivé nitré (nitrométhane, nitrobenzène par exemple) ou dans un mélange de tels solvants, à une température comprise entre 0 et 80° C.

La réduction du sulfoxyde et la libération des radicaux protégés s'effectuent dans les conditions décrites précédemment.

C. Selon l'invention, les produits de formule générale I peuvent également être obtenus par action d'une thio-urée de formule générale:

$$R_2NH-CS-NH_2 \qquad (VI)$$

dans laquelle $R_2$ est un atome d'hydrogène, ou un radical protecteur d'amino sur un dérivé de céphalosporine de formule générale:

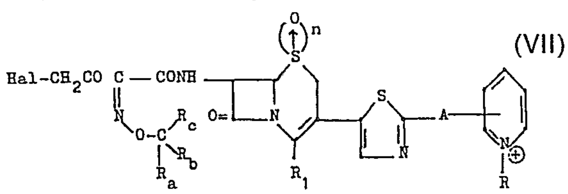

(VII)

dans laquelle les symboles A, R, $R_1$ et n sont définis comme pour la formule générale III (étant entendu que lorsque $R_1$ est autre que carboxylato, le produit se trouve à l'état d'halogénure ou de sulfonate), les symboles $R_a$, $R_b$ et $R_c$ sont définis comme précédemment ou $R_a$ représente un radical carboxy protégé et Hal est un atome d'halogène, suivie éventuellement de la réduction du sulfoxyde et le cas échéant, de l'élimination des radicaux protecteurs.

Lorsque $R_2$ est un radical protecteur d'amino, il peut être choisi parmi les radicaux cités précédemment, à l'exception de chloracétyle ou trichloracétyle.

On utilise avantageusement un produit pour lequel le symbole Hal est un atome d'halogène choisi parmi le chlore et le brome.

On opère généralement en milieu aqueux, organique ou hydroorganique par exemple dans des solvants ou des mélanges de solvants tels que des alcools (méthanol, éthanol), des cétones (acétone), des solvants chlorés (chloroforme, dichlorométhane), des nitriles (acétonitrile), des amides (diméthylformamide, diméthylacétamide), des éthers (tétrahydrofuranne, dioxanne), des esters (acétate d'éthyle) ou des acides (acide acétique, acide formique), en présence ou non d'une base telle que la soude, la potasse, des carbonates, des carbonates acides de métaux alcalins, des sels d'acides carboxyliques et de métaux alcalins (formiate de sodium, acétate de sodium) ou des amines tertiaires (triéthylamine, triméthylamine ou pyridine), à une température comprise entre −30 et +60° C.

Selon l'invention, les produits de formule générale I pour lesquels A est un radical −NHCO− peuvent également être obtenus à partir de l'amine correspondante de formule générale:

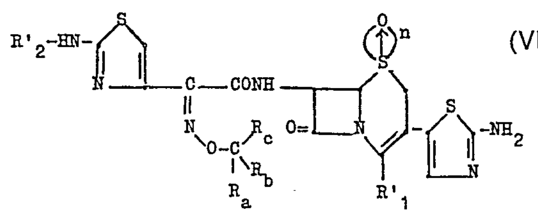

(VIII)

dans laquelle $R_a$, $R_b$, $R_c$, $R'_1$ et n sont définis comme précédemment, étant entendu que lorsque $R_a$ est un radical carboxy, il peut être libre ou protégé et $R'_2$ est un radical protecteur d'amino, par toute méthode connue en soi pour former une fonction amide, sans toucher au reste de la molécule, puis réduction éventuelle du sulfoxyde obtenu et élimination des radicaux protecteurs.

Le radical protecteur d'amino $R'_2$ peut être choisi parmi les groupements cités précédemment pour le procédé A. Il peut être éliminé après la réaction selon les méthodes citées précédemment en A.

La réaction s'effectue généralement par action d'un dérivé de l'acide nicotinique ou isonicotinique dont l'atome d'azote est quaternisé par un radical R tel que défini précédemment, étant entendu que lorsque R est carboxyméthyle, le radical carboxy est protégé.

On opère avantageusement par action d'un halogénure d'acide, dans les conditions décrites précédemment pour la réaction de l'halogénure de l'acide de formule générale II sur le dérivé d'amino-7 céphalosporine de formule générale III.

Le cas échéant, la protection et l'élimination du radical carboxy et la réduction du sulfoxyde s'effectuent dans les conditions décrites précédemment.

Les produits de formule générale II peuvent être préparés selon les méthodes décrites dans la demande de brevet EP-A N° 53961.

Les dérivés d'amino-7 céphalosporines de formule générale III peuvent être obtenus à partir d'un produit de formule générale:

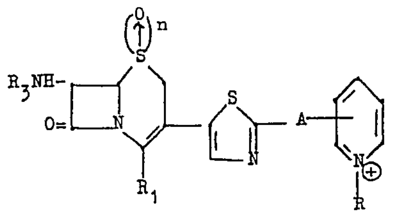

(IX)

dans laquelle A, R, $R_1$ et n sont définis comme précédemment pour la formule générale III (étant entendu que lorsque $R_1$ est autre que le radical carboxylato, le produit se trouve à l'état d'halogénure ou de sulfonate) et $R_3$ représente un radical facilement éliminable, par élimination du radical $R_3$ ou, le cas échéant, élimination successive ou simultanée du radical $R_3$ et des radicaux protecteurs contenus par $R_1$ et R.

Par radical $R_3$ facilement éliminable, on entend par exemple:

1. benzyhydryle ou trityle,
2. un radical acyle de formule générale:

$$R_4CO- \qquad (X)$$

dans laquelle $R_4$ représente:

a) un atome d'hydrogène ou un radical alcoyle contenant 1 à 7 atomes de carbone, méthyle substitué par 1 à 3 atomes d'halogène,

b) un radical phényle (pouvant être jusqu'à 3 fois substitué par des atomes d'halogène ou des radicaux hydroxy, nitro, cyano, trifluorométhyle, alcoyle ou alcoyloxy) ou un radical thiényle-2 ou -3,

c) un radical de formule générale:

$$R'_4-Y-CH_2- \qquad (X_a)$$

dans laquelle $R'_4$ est un radical phényle pouvant être substitué par un atome d'halogène ou par un radical alcoyle, alcoyloxy ou hydroxy, et Y est un atome d'oxygène ou de soufre,

d) un radical arylalcoyle de formule générale:

$$R''_4CH_2- \qquad (X_b)$$

dans laquelle $R''_4$ est un radical phényle pouvant être jusqu'à 3 fois substitué par des radicaux hydroxy, alcoyle ou alcoyloxy ou un radical hétérocyclyle tel que thiényle-2 ou -3 ou furyle-2 ou -3,

3. un radical amino-5 adipoyle dont les fonctions amine et acide sont protégées par des radicaux protecteurs tels que définis précédemment,
4. un radical de formule générale:

$$R_5OCO- \qquad (XI)$$

dans laquelle $R_5$ est un radical alcoyle ramifié non substitué, un radical alcoyle droit ou ramifié portant un ou plusieurs substituants tels que des atomes d'halogène ou des radicaux cyano, trialcoylsilyle, phényle ou phényle substitué (par un ou plusieurs atomes d'halogène ou radicaux alcoyle, alcoyloxy, nitro ou phényle) ou un radical quinolyle,

5. un radical de formule générale:

$$\underset{\underset{(O)_n}{|}}{Ar-S-} \quad (XII_a) \quad ou \quad ArSe- \quad (XII_b)$$

dans lesquelles le reste Ar est un radical phényle éventuellement substitué par un ou plusieurs atomes d'halogène ou radicaux nitro ou alcoyle et n est égal à 0 ou 1, ou bien

6. $R_3NH-$ peut être remplacé par un radical dialcoylaminométhylèneamino ou par un radical de formule générale:

$$Ar'-CH=N- \qquad (XIII)$$

dans laquelle Ar' est un radical phényle éventuellement substitué par un ou plusieurs radicaux tels que alcoyle, alcoyloxy, hydroxy ou nitro, ou encore

7. $R_3$ est un radical diphénylphosphinoyle ou un radical de formule générale:

$$(R'O)_2\underset{\underset{O}{\downarrow}}{P}- \qquad (XIV)$$

dans laquelle R' est alcoyle, trichloro-2,2,2 éthyle, phényle ou benzyle (ces deux derniers éventuellement substitués par un atome d'halogène ou par un radical alcoyle, alcoyloxy ou nitro) ou les radicaux R' forment ensemble un radical alcoylène contenant 2 ou 3 atomes de carbone.

Comme exemples de radicaux $R_3$ pouvant être utilisés, on peut citer les radicaux suivants:

formyle, acétyle, trichloracétyle, phénylacétyle,
phénoxyacétyle, benzoyle,
t-butoxycarbonyle,
chloro-2 diméthyl-1,1 éthoxycarbonyle,
trichloro-2,2,2 éthoxycarbonyle,
trichloro-2,2,2 diméthyl-1,1 éthoxycarbonyle,
cyano-2 diméthyl-1,1 éthoxycarbonyle,
triméthylsilyl-2 éthoxycarbonyle,
benzyloxycarbonyle,
p-méthoxybenzyloxycarbonyle,
diméthoxy-3,5 benzyloxycarbonyle,
p-nitrobenzyloxycarbonyle,
diphénylméthoxycarbonyle,
(biphénylyl-4)-2 isopropyloxycarbonyle,
quinolyl-8 oxycarbonyle,
o-nitrophénylthio,
p-nitrophénylthio,
diméthoxyphophoryle,
diéthoxyphosphoryle,
diphénoxyphosphoryle,
dibenzyloxyphosphoryle.

Comme exemples de radicaux méthylène-amino définis précédemment en 6, on peut citer:
diméthylaminométhylène-amino,
diméthoxy-3,4 benzylidène-amino,
nitro-4 benzylidène-amino.

L'élimination du radical protecteur peut s'effectuer par toute méthode connue pur libérer une fonction amine sans toucher au reste de la molécule.

On opère notamment dans les conditions décrites dans le brevet BE-A N° 883415 ou rappelées ci-avant en A.

Les dérivés de céphalosporine de formule générale IX peuvent être obtenus par analogie avec l'un des procédés B et D utilisés pour la préparation des produits selon l'invention, à savoir:

– soit à partir d'un produit de formule générale:

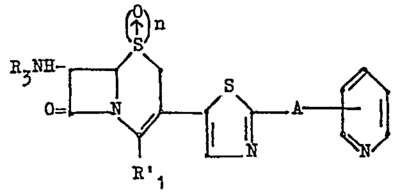

(XV)

dans laquelle A, $R'_1$, $R_3$ et n sont définis comme précédemment, en opérant par analogie avec le procédé B,

– soit, lorsque le symbole A représente un radical $-NHCO-$, à partir d'un produit de formule générale:

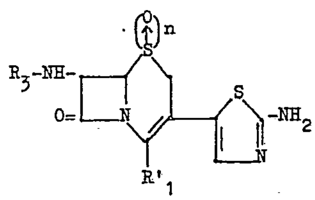

(XVI)

dans laquelle $R'_1$, $R_3$ et n sont définis comme précédemment, en opérant par analogie avec le procédé D.

Les conditions de mise en œuvre de ces procédés sont identiques à celles utilisées pour la préparation des produits de formule générale I.

Les produits de formules générales XV et XVI peuvent être préparés par action d'un produit de formule générale:

$$R_6-CS-NH_2 \qquad (XVII)$$

dans laquelle $R_6$ est un radical amino ou un radical de structure

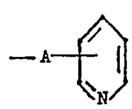

dans lequel A est défini comme précédemment, ou un radical amino, sur un dérivé de céphalosporine de formule générale:

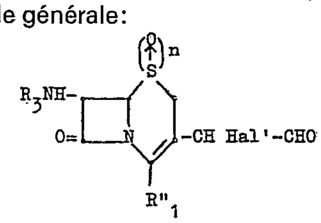

(XVIII)

dans laquelle $R_3$ et n sont définis comme précédemment, $R''_1$ est un radical carboxy protégé tel que défini pour $R'_1$ et Hal' représente un atome d'halogène, puis éventuellement action d'un agent de déshydratation, et éventuellement réduction du sulfoxyde et élimination des groupements protecteurs.

Le symbole Hal' peut représenter un atome de chlore, de brome ou d'iode.

On opère généralement en milieu organique ou hydroorganique, par exemple dans des solvants (ou des mélanges de solvants) tels que des alcools (méthanol, éthanol), des éthers (tétrahydrofuranne, dioxanne), des cétones (acétone), des nitriles (acétonitrile), des amides secondaires (diméthylformamide, diméthylacétamide), des esters (acétate d'éthyle) ou des acides (acide acétique, acide formique), en présence ou non d'une base (soude, potasse, carbonates, carbonates acides de métaux alcalins, sels d'acides carboxyliques et de métaux alcalins, amines tertiaires), à une température comprise entre $-50°$ C et la température de reflux du mélange réactionnel.

Il est parfois préférable d'introduire un agent de déshydratation.

Parmi les agents de déshydratation utilisables, on peut citer: les halogénures d'acides sulfoniques (par exemple chlorure de tosyle, chlorure de méthanesulfonyle ou un halogénure du type $R''SO_2Cl$ dans lequel R'' est alcoyle, trifluoro (ou trichloro) méthyle ou phényle éventuellement substitué par halogène méthyle ou nitro), les halogénures de phosphoryle (par exemple oxychlorure de phosphore) ou le chlorure de sulfonyle, dans un solvant basique pyridine, amide (par exemple diméthylformamide, diméthylacétamide, hexaméthylphosphorotriamide) ou dans un solvant chloré (par exemple chloroforme, chlorure de méthylène), un éther (par exemple tétrahydrofuranne), un ester, une cétone, un nitrile, un solvant aromatique, en présence d'une amine tertiaire (par exemple pyridine, quinoléine, triéthylamine).

Les produits de formule générale XVII peuvent être préparés par action d'ammoniac sur l'isothiocyanate ou le dithiocarbamate d'alcoyle ou d'aryle correspondant, ou par action de suflure d'hydrogène sur le nitrile correspondant, et notamment selon la méthode citée ci-après:

– lorsque $R_6$ représente nicotinoylamino ou isonicotinoylamino: selon la méthode de W.H. Pike, «Chem. Ber.», *6*, 755 (1873).

Les dérivés de céphalosporine de formule générale XVIII dans laquelle n = 0 peuvent être préparés par action d'un agent d'halogénation sur une énamine de formule générale:

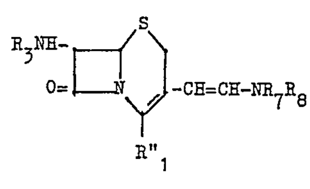

(XIX)

dans laquelle $R''_1$ et $R_3$ sont définis comme précédemment et $R_7$ et $R_8$, identiques ou différents, représentent des radicaux alcoyle (éventuellement substitués par un radical alcoyloxy ou dialcoylamino) ou phényle, ou forment ensemble avec l'atome d'azote auquel ils sont attachés, un hétérocycle à 5 ou 6 chaînons contenant éventuellement un autre hétéroatome choisi parmi l'azote, l'oxygène ou le soufre et éventuellement substitué par un radical alcoyle, suivie de l'hydrolyse du produit formé.

Par exemple on met en œuvre une énamine de formule générale XIX dans laquelle $R_7$ et $R_8$ représentent chacun un radical méthyle.

Parmi les agents d'halogénation peuvent être cités: les halogènes, les N-haloamides [par exemple N-bromo (ou N-chloro) succinimide, N-bromo (ou N-chloro) acétamide, dibromohydantoïne], les hypohalogénites d'alcoyle (par exemple l'hypochlorite de t-butyle, l'hypobromite de t-butyle ou l'hypochlorite d'éthyle).

On opère généralement dans un solvant organique tel qu'un éther (par exemple tétrahydrofuranne, dioxanne), un solvant chloré (par exemple chlorure de méthylène, chloroforme), un ester (par exemple acétate d'éthyle), un alcool (par exemple méthanol, éthanol), un amide (par exemple diméthylformamide, diméthylacétamide), un nitrile (par exemple acétonitrile) ou une cétone (par exemple acétone), ou dans un mélange de ces solvants, à une température comprise entre −70 et 0° C.

L'hydrolyse s'effectue à une température comprise entre −70 et +20° C.

Il n'est pas indispensable d'isoler ni de purifier les produits de formule générale XVIII pour les mettre en œuvre selon l'invention.

Les dérivés de la céphalosporine de formule générale XVIII dans laquelle n = 1 peuvent être obtenus par oxydation d'un produit de formule générale XVIII dans laquelle n = 0 par application des méthodes décrites dans la demande de brevet DE-A N° 2637176.

Les énamines de formule générale XIX peuvent être préparées par application de la méthode décrite dans le brevet BE-A N° 883416 à partir de dérivés de céphalosporines de formule générale:

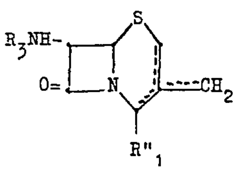

(XX)

Les dérivés de la céphalosporine de formule générale XX peuvent être obtenus comme décrit dans le brevet BE-A N° 883416 ou, lorsque $R_3$ est un radical facilement éliminable tel que défini précédemment en 7, par application de la méthode décrite par A. Morimoto et coll., «J. Chem. Soc. Perkin I», 1109 (1980), à partir de l'halogénure correspondant $R_3-Hal$ qui peut être lui-même obtenu selon l'une des méthodes décrites par [K. Sasse, «Methoden den Organischen Chemie», vol. 12, part. 2, p. 274, Houben Weyl, «Georg Thieme Verlag, Stuttgart (1964)].

Les dérivés de la céphalosporine de formules générales V et VIII peuvent être préparés par application du procédé A décrit prédécemment pour la préparation des produits selon l'invention, c'est-à-dire par acylation d'une amino-7 céphalosporine de formule générale:

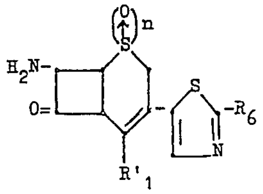

(XXI)

dans laquelle $R'_1$, $R_6$ et n sont définis comme précédemment, puis éventuellement élimination des radicaux protecteurs.

Il est entendu que lorsque $R_6$ représente un radical amino, ce dernier est de préférence protégé. La protection s'effectue par un groupement tel que défini précédemment pour $R_2$ dans les formules générales V et VIII.

L'acylation s'effectue dans les conditions décrites précédemment pour la préparation des produits selon l'invention par le procédé A.

L'élimination de radicaux protecteurs peut être effectuée dans les conditions décrites précédemment.

L'amino-7 céphalosporine de formule générale XXI peut être obtenu à partir d'une céphalosporine de formule générale XV ou XVI (selon la nature du substituant $R_6$), par application des méthodes décrites pour la préparation de l'amino-7 céphalosporine de formule générale III.

Il est entendu que lorsque $R_6$ représente un radical amino ce dernier est de préférence protégé; la protection est effectuée par un groupement différent de $R_3$, de telle manière que le radical protecteur $R_3$ soit éliminable sélectivement.

Les dérivés de la céphalosporine de formules générales V et VIII peuvent également être préparés à partir d'une céphalosporine de formule générale:

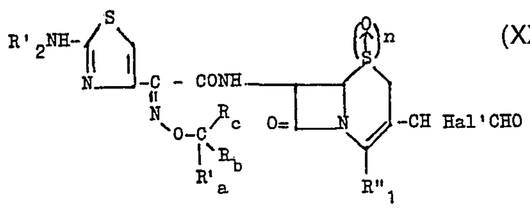

(XXII)

dans laquelle $R''_1$, $R'_2$, $R_b$, $R_c$, n et Hal' sont définis comme précédemment, et $R'_a$ est un atome d'hydrogène ou un radical carboxy protégé, par analogie avec la méthode décrite précédemment pour la préparation des produits de formules générales XV et XVI.

Le symbole $R'_2$ qui représente un radical protecteur d'amino peut être par exemple un radical t-butoxycarbonyle, trichloro-2,2,2 éthoxycarbonyle, trityle, benzyle, benzyloxycarbonyle, formyle, trifluoracétyle, p-nitrobenzyloxycarbonyle, p-méthoxybenzyloxycarbonyle, diphénylphosphinoyle ou un radical de formule générale XIV.

Les produits de formule générale XXII dans laquelle n = 0 peuvent être obtenus à partir d'une énamine de formule générale:

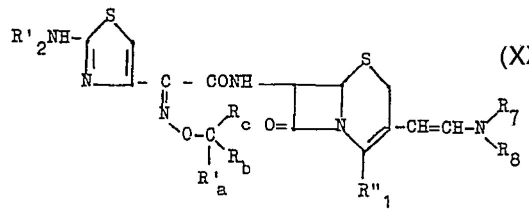

(XXIII)

dans laquelle $R''_1$, $R'_2$, $R'_a$, $R_b$, $R_c$, $R_7$ et $R_8$ sont définis comme précédemment, en opérant par analogie avec la méthode décrite pour la préparation des céphalosporines de formule générale XVIII.

Les produits de formule générale XXII dans laquelle n = 1 peuvent être obtenus par oxydation du produit correspondant de formule générale XXII dans laquelle n = 0, dans les conditions décrites dans la demande de brevet DE-A N° 2637176.

Les énamines de formule générale XXIII peuvent être obtenues selon la méthode décrite dans le brevet BE-A N° 883416 ou dans la demande de brevet EP-A N° 53961.

Les produits de formule générale VII peuvent être obtenus par action d'un halogénure d'acide de formule générale:

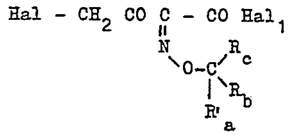

(XXIV)

dans laquelle Hal, $R_b$ et $R_c$ sont définis comme dans la formule générale VII et $R'_a$ est défini comme dans la formule générale XXII, et $Hal_1$ représente un atome de chlore ou de brome, sur une amino-7 céphalosporine de formule générale III, suivie éventuellement de la réduction du sulfoxyde et de l'élimination des radicaux protecteurs.

La réaction s'effectue généralement en milieu hydroorganique par exemple eau/éther (tétrahydrofuranne, dioxanne), eau/cétone (acétone) ou eau/solvant chloré (chloroforme, dichlorométhane), en présence d'un agent alcalin de condensation tel qu'un bicarbonate alcalin (par exemple bicarbonate de sodium) à une température comprise entre −40 et +40° C.

Il est également possible d'opérar par analogie avec la méthode décrite dans la demande de brevet FR-A N° 2399418.

Il est entendu que, lorsque le radical R de l'amino-7 céphalosporine contient un radical carboxy, ce dernier est libre ou protégé.

Les produits de formule générale XXIV peuvent être préparés comme décrit dans la demande de brevet EP-A N° 53961.

Les dérivés de la céphalosporine de formules générales V, VIII, XV, XVI et XXI dans lesquelles n = 1, peuvent être obtenus par oxydation des dérivés correspondants dans lesquels n = 0 dans les conditions décrites dans la demande de brevet DE-A N° 2637176.

Les produits selon l'invention peuvent être transformés en sels d'addition avec les acides et notamment en sels d'addition avec les acides forts. Ces sels d'addition peuvent être obtenus par action du produit sur des acides dans des solvants appropriés. Comme solvants organiques on utilise par exemple des alcools, des cétones, des éthers ou des solvants chlorés.

Les produits selon la présente invention pour lesquels $R_a$ est un radical carboxy ou R un radical carboxyméthyle peuvent être transformés en sels métalliques ou en sels d'addition avec les bases azotées selon les méthodes connues en soi. Ces sels peuvent être obtenus par action d'une base métallique (par exemple alcaline ou alcalino-terreuse), de l'ammoniac ou d'une amine sur un produit selon l'invention dans un solvant approprié tel qu'un alcool, un éther ou l'eau ou par réaction d'échange avec un sel d'un acide organique. Le sel formé précipite après concentration éventuelle de sa solution, il est séparé par filtration ou décantation. Il peut également être isolé de sa solution par évaporation du solvant, notamment par lyophilisation.

Comme exemple de sels pharmaceutiquement acceptables peuvent être cités les sels avec les métaux alcalins (sodium, potassium, lithium) ou avec les métaux alcalino-terreux (magnésium, calcium), les sels d'ammonium, les sels de bases azotées (étahnolamine, diéthanolamine, triméthylamine, triéthylamine, méthylamine, propylamine, diisopropylamine, N,N-diméthyléthanolamine, benzylamine, dicyclohexylamine, N-benzyl-β-phénéthylamine, N,N'-dibenzyléthylènediamine, N-méthylglucamine, diphénylènediamine, benzhydrylamine, quinine, choline, arginine, lysine, leucine, dibenzylamine) ou les sels d'addition avec des acides minéraux (chlorhydrates, bromhydrates, sulfates, nitrates, phosphates) ou organiques (succinates, fumarates, maléates, p-toluènesulfonates).

Les nouveaux produits selon la présente invention peuvent être éventuellement purifiés par des méthodes physiques telles que la cristallisation, l'ultrafiltration ou la chromatographie.

Les nouveaux dérivés de la céphalosporine selon la présente invention et leurs sels pharmaceutiquement acceptables présentent des propriétés antibactériennes particulièrement intéressantes. Ils manifestent une activité remarquable *in vitro* et *in vivo* sur les germes Gram-positifs et Gram-négatifs.

*In vitro*, les produits de formule générale I se sont montrés actifs à une concentration comprise entre 1 et 30 μg/cm³ sur des souches de staphylocoques sensibles à la pénicilline G (*Staphylococcus aureus* Smith) et à une concentration comprise entre 0,01 et 1 μg/cm³ sur *Escherichia coli* souche NIHJ. De plus les produits de formule générale I pour lesquels $R_a$ est un radical carboxy se sont montrés actifs à une concentration comprise entre 2 et 15 μg/cm³ sur *Pseudomonas aeruginosa* Dalgleish.

*In vivo*, les produits de formule générale I se sont montrés actifs sur les infections expérimentales de la souris à *Staphylococcus aureus* Smith (sensible à la pénicilline G) à une dose comprise entre 0,5 et 15 mg/kg/d par voie sous-cutanée.

Les produits de formule générale I se sont montrés atoxiques à une dose de 1 mg/kg par voie sous-cutanée chez la souris.

D'un intérêt particulier sont les produits de formule générale I dans laquelle le symbole A représente une liaison simple, ou un radical bivalent choisi parmi $-CH_2-$, $-NH-$ ou $-NHCO-$ fixé en position -3 ou -4 du radical pyridinio, le symbole R représente un radical méthyle, carboxyméthyle, carbamoylméthyle ou benzyle, les symboles $R_a$, $R_b$ et $R_c$ représentent chacun un atome d'hydrogène, ou bien le symbole $R_a$ représente un radical carboxy et les symboles $R_b$ et $R_c$ représentent des atomes d'hydrogène ou des radicaux méthyle, et n est égal à 0 ou 1, ainsi que leurs sels d'addition avec

les acides, leurs sels métalliques ou leurs sels d'addition avec les bases azotées lorsqu'ils existent.

Et parmi ces produits, plus spécialement actifs sont les produits de formule générale I dans laquelle le symbole A est une liaison simple ou un radical -NH-, fixé en position -3 du radical pyridinio, le symbole R représente un radical méthyle, carboxyméthyle ou carbamoylméthyle, les symboles $R_a$, $R_b$ et $R_c$ représentent des atomes d'hydrogène, ou bien le symbole $R_a$ représente un radical carboxy et les symboles $R_b$ et $R_c$ représentent des atomes d'hydrogène ou des radicaux méthyle et n égale 0, ainsi que leurs sels d'addition avec les acides, leurs sels métalliques ou leurs sels d'addition avec les bases azotées lorsqu'ils existent.

Les exemples suivants, donnés à titre non limitatif, illustrent la présente invention.

*Exemple 1 :*

Une solution de 14,9 g de benzhydryloxycarbonyl-2 [méthoxyimino-2 (tritylamino-2 thiazolyl-4)-2 acétamido]-7 oxo-8 oxyde-5 [(pyridyl-3)-2 thiazolyl-5]-3 thia-5 aza-1 bicyclo-[4.2.0]-octène-2 isomère syn et de 1,07 $cm^3$ d'iodure de méthyle dans 60 $cm^3$ de N,N-diméthylformamide est agitée 24 h à 20° C, filtrée et versée sur 600 $cm^3$ d'acétate d'éthyle. Le précipité formé est essoré et lavé 4 fois par 50 $cm^3$ d'acétate d'éthyle et 2 fois par 50 $cm^3$ d'oxyde d'isopropyle. Le solide est cristallisé dans 70 $cm^3$ de dichlorométhane pour donner 10,4 g d'iodure de benzhydryloxycarbonyl-2 [méthoxyimino-2 (tritylamino-2 thiazolyl-4)-2 acétamido]-7 [(méthyl-1 pyridinio-3)-2 thiazolyl-5]-3 oxo-8 oxyde-5 thia-5 aza-1 bicyclo-[4.2.0]-octène-2 isomère syn sous la forme d'une poudre cristalline jaune.

Spectre infrarouge (KBr, bandes caractéristiques en $cm^{-1}$) 1800, 1740, 1520, 1500, 1450, 1220, 1040, 760, 705, 675.

Spectre RMN du proton (350 MHz, DMSO $d_6$, δ en ppm, J en Hz) 3,86 (S, 3H, $=N-O-CH_3$); 3,87 et 4,50 (2D, 2H, $-S-CH_2-$); 4,47 (S, 3H, $\geq \overset{+}{N}-CH_3$); 5,13 (D, J = 5, 1H, -H en 6); 5,97 (DD, J = 7 et 5, 1H, H en 7); 6,72 (S, 1H, H en 5 du thiazole); 6,96 [S, 1H, $-COO-\underline{CH}(C_6H_5)_2$]; 6,90 à 7,4 (Mt, 25H, aromatiques); 8,05 (S, 1H, -H en 4 du thiazole); 8,23 (DD, J = 8 et 6, 1H, -H en 5 du pyridinio); 8,73 (D large, J = 8, 1H, H en 4 du pyridinio); 8,79 [S, 1H, $-\underline{NH}-C(C_6H_5)_3$]; 9,04 (D, J = 6, 1H, H en 6 du pyridinio); 9,21 (D, J = 7, 1H, -CO-NH-); 9,40 (S large, 1H, -H en 2 pyridinio).

Une solution de 10,4 g d'iodure de benzhydryloxycarbonyl-2 [méthoxyimino-2-(tritylamino-2 thiazolyl-4)-2 acétamido]-7 [(méthyl-1 pyridinio-3)-2 thiazolyl-5]-3 oxo-8 oxyde-5 thia-5 aza-1 bicyclo-[4.2.0]-octène-2 isomère syn dans un mélange de 200 $cm^3$ de dichlorométhane et de 50 $cm^3$ de N,N-diméthylacétamide refroidie à -10° C est traitée par 1,8 $cm^3$ de trichlorure de phosphore puis agitée à -5° C pendant 40 min, filtrée et versée sur 1 l d'acétate d'éthyle. Le précipité est essoré, lavé par 2 fois 30 $cm^3$ d'acétate d'éthyle et 2 fois 30 $cm^3$ d'oxyde d'isopropyle, repris dans 50 $cm^3$ de dichlorométhane et reprécipité par 250 $cm^3$ d'acétate d'éthyle. Le solide est essoré, lavé à nouveau comme ci-dessus et séché pour donner 11,7 g d'iodure de benzhydryloxycarbonyl-2 [méthoxyimino-2 (tritylamino-2 thiazolyl-4)-2 acétamido]-7 [(méthyl-1 pyridinio-3)-2 thiazolyl-5]-3 oxo-8 thia-5 aza-1 bicyclo-[4.2.0.]-octène-2 isomère syn sous la forme d'un solide jaune.

Spectre RMN du proton (250 MHz, DMSO $d_6$, δ en ppm, J en Hz) 3,9 (S, 3H, $=N-OCH_3$); 3,93 et 4,06 (2D, J = 19, 2H, $-S-CH_2-$); 4,47 (S, 3H, $\geq \overset{+}{N}-CH_3$); 5,32 (D, J = 5, 1H, -H en 6); 5,93 (DD, J = 8 et 5, 1H, -H en 7); 6,81 (S, 1H, -H en 5 du thiazole); 6,93 S, 1H, $-COO-\underline{CH}(C_6H_5)_2$]; 6,90 à 7,4 (Mt, 25H, aromatiques); 8,08 (S, 1H, -H en 4 du thiazole); 8,24 (DD, J = 8 et 6, 1H, -H en 5 du pyridino); 8,73 (D, J = 8, 1H, H en 4 du pyridinio); 9,09 (D, J = 6, 1H, H en 6 du pyridinio); 9,42 (S large, 1H, -H en 2 du pyridinio); 9,48 [Mf, 1H, $-\underline{NH}-C(C_6H_5)_3$]; 9,78 (D, J = 8, 1H, -CO-NH-).

Une solution de 11,6 g d'iodure de benzhydryloxycarbonyl-2 [méthoxyimino-2 (tritylamino-2 thiazolyl-4)-2 acétamido]-7 [(méthyl-1 pyridinio-3)-2 thiazolyl-5]-3 oxo-8 thia-5 aza-1 bicyclo-[4.2.0]-octène-2 isomère syn dans un mélange de 20 $cm^3$ d'anisole et de 150 $cm^3$ d'acide formique est chauffée 30 min à 50° C puis diluée par 50 $cm^3$ d'eau distillée et chauffée encore 30 min à 50° C. La solution est concentrée à sec sous pression réduite (1 mm de mercure; 0,13 kPa) à 40° C. Le résidu est repris par 50 $cm^3$ d'éthanol et la solution est concentrée à sec sous pression réduite (30 mm de mercure; 4 kPa) à 40° C, cette solution étant répétée encore 2 fois. Le résidu est concrété par 50 $cm^3$ d'éthanol. Le solide est essoré, lavé 4 fois par 20 $cm^3$ d'éthanol, 3 fois par 20 $cm^3$ d'acétone, 2 fois par 20 $cm^3$ d'acétate d'éthyle et 2 fois par 20 $cm^3$ d'éther éthylique et séché. On obtient 7,6 g d'iodhydrate d'[(amino-2 thiazolyl-4)-2 méthoxyimino-2 acétamido]-7 carboxylato-2 [(méthylpyridinio-3)-2 thiazolyl-5]-3 oxo-8 thia-5 aza-1 bicyclo-[4.2.0.]-octène-2 isomère syn brut sous la forme d'un solide brun clair que l'on reprend par 500 $cm^3$ d'eau distillée et 250 $cm^3$ d'acétate d'éthyle; après filtration et décantation de la phase aqueuse, celle-ci est lavée par 150 $cm^3$ d'acétate d'éthyle et traitée par de la résine IR 45 basique jusqu'à atteindre un pH de 4,7. La résine est éliminée par filtration et la solution aqueuse est lyophilisée. Le lyophilisat est concrété par 50 $cm^3$ d'eau distillée pour donner 3,15 g d'[(amino-2 thiazolyl-4)-2 méthoxyimino-2 acétamido]-7 carboxylato-2 [(méthyl-1 pyridinio-3)-2 thiazolyl-5]-3 oxo-8 thia-5 aza-1 bicyclo-[4.2.0]-octène-2 isomère syn sous la forme d'une poudre orangée.

Spectre infrarouge (KBr, bandes caractéristiques en $cm^{-1}$) 3600-2500, 1765, 1670, 1610, 1530, 1380, 1030, 770, 675.

Spectre RMN du proton (250 MHz, DMSO $d_6$, δ en ppm, J en Hz) 353,71 et 3,84 (2D, J = 17, 2H, $-S-CH_2-$); 3,86 (S, 3H, $=N-OCH_3$); 4,43 (S,

3H, ⩾ $\overset{+}{N}-CH_3$); 5,15 (D, J = 5, 1H, –H en 6); 5,65 (DD, J = 8 et 5, 1H, –H en 7); 6,76 (S, 1H, –H en 5 du thiazole); 7,27 (Mf, 2H, $-NH_2$); 8,08 (S, 1H, –H en 4 du thiazole); 8,14 (DD, J = 8 et 6, 1H, –H en 5 du pyridinio); 8,83 (D, J = 8, 1H, –H en 4 du pyridinio); 8,98 (D, J = 6, 1H, –H en 6 du pyridinio); 9,48 (S, 1H, –H en 2 du pyridinio); 9,64 (D, J = 8, 1H, –CONH–).

Le benzhydryloxycarbonyl-2 [méthoxyimino-2 (tritylamino-2 thiazolyl-4)-2 acétamido]-7 oxo-8 oxyde-5 [(pyridyl-3)-2 thiazolyl-5]-3 thia-5 aza-1 bicyclo-[4.2.0]-octène-2 isomère syn peut être préparé de la manière suivante:

A une solution de 25 g d'acide méthoxyimino-2 (tritylamino-2 thiazolyl-4)-2 acétique forme syn dans un mélange de 500 $cm^3$ de dichlorométhane sec de 5,25 $cm^3$ de N,N-diméthylacétamide refroidie à –10° C on ajoute goutte à goutte 32,6 $cm^3$ d'une solution toluénique 2M de phosgène. Le mélange réactionnel et agité 3½ h entre –5 et 0° C, puis on ajoute à –5° C, 15,8 $cm^3$ de N,N-diméthylacétamide et une solution de 26 g d'amino-7 benzhydryloxycarbonyl-2 oxo-8 oxyde-5 [(pyridyl-3)-2 thiazolyl-5]-3 thia-5 aza-1 bicyclo-[4.2.0]-octène-2 dans 200 $cm^3$ de dichlorométhane. Le mélange réactionnel est agité 1½ h entre 0 et +5° C puis 16 h à 20° C, puis traité par 500 $cm^3$ de solution à 5% de bicarbonate de sodium et filtré. La phase organique est lavée par 500 $cm^3$ de solution demi-saturée de chlorure de sodium, séchée sur sulfate de magnésium et concentrée à sec sous pression réduite (30 mm de mercure; 4 kPa) à 30° C. Le résidu est chromatographié sur une colonne (hauteur = 52 cm, diamètre = 7 cm) de gel de silice (0,06-0,2 mm) en éluant par de l'acétate d'éthyle et en recueillant des fractions de 1 l. Les fractions 6 à 13 sont réunies et concentrées à sec sous pression réduite (30 mm de mercure; 4 kPa) à 30° C pour donner 20,2 g de benzhydryloxycarbonyl-2 [méthoxyimino-2 (tritylamino-2 thiazolyl-4)-2 acétamido]-7 oxo-8 oxyde-5 [(pyridyl-3)-2 thiazolyl-5]-3 thia-5 aza-1 bicyclo-[4.2.0]-octène-2 isomère syn sous la forme d'un solide crème (par reprise de la meringue obtenue dans 80 $cm^3$ de dichlorométhane et précipitation par 300 $cm^3$ d'oxyde d'isopropyle).

Spectre infrarouge (KBr, bandes caractéristiques en $cm^{-1}$) 1800, 1730, 1685, 1495, 1450, 1420, 1220, 1050, 755, 700.

Spectre RMN du proton (250 MHz, $CDCl_3$, δ en ppm, J en Hz) 3,38 et 3,99 (D large et D, J = 19, 2H, $-SCH_2-$); 4,09 (S, 3H, $=N-O-CH_3$); 4,67 (D large, J = 5, 1H, –H en 6); 6,26 (DD, J = 10 et 5, 1H, –H en 7); 6,74 (S, 1H, –H en 5 du thiazole); 6,96 [S, 1H, $-COO-\underline{CH}(C_6H_5)_2$]; 6,95 à 7,45 [Mt, aromatiques et $-\underline{NH}-C(C_6H_5)_3$]; 7,37 (DD, J = 8 et 5, –H en 5 de la pyridine); 7,58 (D, J = 10, 1H, –CO–NH–); 7,60 (S, 1H, –H en 4 du thiazole); 8,02 (DDD, J = 8, 2 et 1,5, 1H, –H en 4 de la pyridine); 8,67 (DD, J = 5 et 1,5, 1H, –H en 6 de la pyridine); 8,93 (D, J = 2, 1H, –H en 2 de la pyridine).

*Exemple 2:*

Une solution de 3,5 g de benzhydryloxycarbonyl-2 [t-butoxycarbonylméthoxyimino-2 (tritylamino-2 thiazolyl-4)-2 acétamido]-7 oxo-8 oxyde-5 [(pyridyl-3)-2 thiazolyl-5]-3 thia-5 aza-1 bicyclo-[4.2.0]-octène-2 isomère syn et de 0,23 $cm^3$ d'iodure de méthyle est laissée 16 h à 25° C à l'abri de la lumière puis versée dans un mélange de 100 $cm^3$ d'acétate d'éthyle et 150 $cm^3$ d'oxyde d'isopropyle. Le précipité est essoré, lavé 3 fois par 30 $cm^3$ d'oxyde d'isopropyle, repris dans 30 $cm^3$ de dichlorométhane et reprécipité par un mélange d'acétate d'éthyle et d'éther éthylique pour donner 3,7 g d'iodure de benzhydryloxycarbonyl-2 [t-butoxycarbonylméthoxyimino-2 (tritylamino-2 thiazolyl-4)-2 acétamido]-7 [(méthyl-1 pyridinio-3)-2 thiazolyl-5]-3 oxo-8 oxyde-5 thia-5 aza-1 bicyclo-[4.2.0]-octène-2 isomère syn sous la forme d'un solide jaune.

Spectre infrarouge (KBr, bandes caractéristiques en $cm^{-1}$) 3410, 1800, 1730, 1680, 1515, 1495, 1450, 1370, 1225, 1155, 750, 700, 670.

Spectre RMN du proton (250 MHz, DMSO $d_6$, δ en ppm, J en Hz) 1,44 [S, 9H, $-C(CH_3)_3$]; 3,88 et 4,41 (2D, J = 19, 2H, $-S-CH_2-$); 4,47 (S, 3H, ⩾ $\overset{+}{N}-CH_3$); 4,56 (S, 2H, $=N-O-CH_2-$); 5,13 (D, J = 5, 1H, –H en 6); 6,03 (DD, J = 8 et 5, 1H, –H en 7); 6,88 (S, 1H, –H en 5 du thiazole); 6,96 [S, 1H, $-COO-\underline{CH}(C_6H_5)_2$]; 6,9 à 7,4 (Mt, 25H, aromatiques); 8,05 (S, 1H, –H en 4 du thiazole); 8,22 (DD, J = 8 et 6, 1H, –H en 5 du pyridinio); 8,73 (D large, J = 8, 1H, –H en 4 du pyridinio); 8,84 [S, 1H, $-NH-C(C_6H_5)_3$]; 8,95 (D, J = 8, 1H, –CO–NH–); 9,05 (D, J = 6, 1H, –H en 6 du pyridinio); 9,42 (S large, 1H, –H en 2 du pyridinio).

Ce produit est redissous dans un mélange de 50 $cm^3$ de dichlorométhane et de 1,15 $cm^3$ de N,N-diméthylacétamide refroidi à –10° C, auquel on ajoute 0,54 $cm^3$ de trichlorure de phosphore. Après 30 min à –5° C la solution réactionnelle est versée dans 300 $cm^3$ d'acétate d'éthyle. Le précipité est essoré, lavé 4 fois par 30 $cm^3$ d'acétate d'éthyle puis par 50 $cm^3$ d'oxyde d'isopropyle pour donner 3,6 g d'iodure de benzhydryloxycarbonyl-2 [t-butoxycarbonylméthoxyimino-2 (tritylamino-2 thiazolyl-4)-2 acétamido]-7 [(méthyl-1 pyridinio-3)-2 thiazolyl-5]-3 oxo-8 thia-5 aza-1 bicyclo-[4.2.0]-octène-2 isomère syn sous la forme d'un solide jaune.

Spectre infrarouge (KBr, bandes caractéristiques en $cm^{-1}$) 3420, 1790, 1730, 1690, 1520, 1500, 1450, 1230, 1160, 760, 705, 670.

Spectre RMN du proton (250 MHz, DMSO $d_6$, δ en ppm, J en Hz) 1,46 [S, 9H, $-C(CH_3)_3$]; 3,89 et 4,05 (2D, J = 19, 2H, $-S-CH_2-$); 4,48 (S, 3H, ⩾ $\overset{+}{N}-CH_3$); 4,56 (S, 2H, $=N-OCH_2-$); 5,32 (D, J = 5, 1H, –H en 6); 5,94 (DD, J = 8 et 5, 1H, –H en 7); 6,81 (S, 1H, –H en 5 du thiazole); 6,93 [S, 1H, $-COO-\underline{CH}(C_6H_5)_2$]; 6,90 à 7,4 (Mt, 25H, aromatiques); 8,06 (S, 1H, –H en 4 du thiazole); 8,23 (DD, J = 8 et 6, 1H, –H en 5 du pyridinio); 8,74 (D large, J = 8, 1H, –H en 4 du pyridinio);

9,04 [S, 1H, $-N\underline{H}-C(C_6H_5)_3$]; 9,06 (D, J = 6, 1H, $-H$ en 6 du pyridinio); 9,40 (S, 1H, $-H$ en 2 du pyridinio); 9,68 (D, J = 8, 1H, $-CO-NH-$).

Une solution de 3,6 g d'iodure de benzhydryloxycarbonyl-2 [t-butoxycarbonylméthoxyimino-2 (tritylamino-2 thiazolyl-4)-2 acétamido]-7 [(méthyl-1 pyridinio-3)-2 thiazolyl-5]-3 oxo-8 thia-5 aza-1 bicyclo-[4.2.0]-octène-2 isomère syn dans un mélange de 50 $cm^3$ d'acide formique et 6 $cm^3$ d'anisole est agitée 30 min à 50° C, puis encore 15 min à la même température après addition de 15 $cm^3$ d'eau distillée. Après concentration à sec sous pression réduite (0,1 mm de mercure; 13 Pa) à 40° C, le résidu est repris par 50 $cm^3$ d'éthanol absolu que l'on évapore sous pression réduite (30 mm de mercure; 4 kPa) à 40° C. Cette opération est répétée encore 2 fois puis le résidu est concrété par 30 $cm^3$ d'éthanol. Le précipité est encore lavé 3 fois par 10 $cm^3$ d'éthanol, 3 fois par 10 $cm^3$ d'acétone et 3 fois par 10 $cm^3$ d'éther éthylique puis séché pour donner 2,6 g d'iodhydrate d'{(amino-2 thiazolyl-4)-2 carboxyméthoxyimino-2 acétamido}-7 carboxylato-2 [(méthylpyridinio-3)-2 thiazolyl-5]-3 thia-5 aza-1 bicyclo-[4.2.0]-octène-2 isomère syn brut sous la forme d'un solide jaune. Le produit brut obtenu est repris dans un mélange de 100 $cm^3$ d'acétate d'éthyle et 250 $cm^3$ d'eau distillée en agitant 20 min à 20° C; après filtration et décantation de la phase aqueuse celle-ci est relavée par 100 $cm^3$ d'acétate d'éthyle puis traitée par 30 $cm^3$ de résine IR 45 basique jusqu'à ce que le pH atteigne une valeur de 3,5. La résine est éliminée par filtration et la solution est lyophilisée. Le solide obtenu est concrété par 10 $cm^3$ d'eau, le solide est filtré et lavé 2 fois par 1 $cm^3$ d'eau pour donner 0,64 g d'[(amino-2 thiazolyl-4)-2 carboxyméthoxyimino-2 acétamido]-7 carboxylato-2 [(méthyl-1 pyridinio-3)-2 thiazolyl-5]-3 thia-5 aza-1 bicyclo-[4.2.0]-octène-2 isomère syn sous la forme d'un solide jaune.

Spectre infrarouge (KBr, bandes caractéristiques en $cm^{-1}$) 3600-2000, 1770, 1675, 1615, 1530, 1360, 1040, 670.

Spectre RMN du proton (350 MHz, DMSO $d_6$, δ en ppm, J en Hz) 3,63 et 3,76 (2D, J = 19, 2H, $-S-CH_2-$); 4,43 (S, 3H, $\geqslant \overset{+}{N}-CH_3$); 4,56 (S, 2H, $=N-O-CH_2-$); 5,15 (D, J = 5, 1H, $-H$ en 6); 5,78 (DD, J = 9 et 5, 1H, $-H$ en 7); 6,83 (S, 1H, $-H$ en 5 du thiazole); 7,25 (Mf, 2H, $-NH_2$); 7,97 (S, 1H, $-H$ en 4 du thiazole); 8,08 (DD, J = 7,5 et 5, 1H, $-H$ en 5 du pyridinio); 8,78 (D, J = 7,5, 1H, $-H$ en 4 du pyridinio); 9,09 (D, J = 5, 1H, H en 6 du pyridinio); 9,43 (S, 1H, $-H$ en 2 du pyridinio); 10,19 (Mf, 1H, $-CO-NH-$).

Le benzhydryloxycarbonyl-2 [t-butoxycarbonylméthoxyimino-2 (tritylamino-2 thiazolyl-4)-2 acétamido]-7 oxo-8 oxyde-5 [(pyridyl-3)-2 thiazolyl-5]-3 thia-5 aza-1 bicyclo-[4.2.0]-octène-2 isomère syn peut être obtenu de la manière suivante:

Une solution de 6 g de benzhydryloxycarbonyl-2 t-butoxycarbonylamino-7 oxo-8 oxyde-5 [(pyridyl-3)-2 thiazolyl-5]-3 thia-5 aza-1 bicyclo-[4.2.0]-octène-2 dans 60 $cm^3$ d'acétonitrile contenant 6 $cm^3$ d'acide méthane sulfonique est agitée 6 min à 20° C. Le mélange réactionnel est versé dans 800 $cm^3$ de solution saturée de bicarbonate de sodium et extrait par 400 $cm^3$ d'acétate d'éthyle. La phase organique est lavée par 400 $cm^3$ de solution saturée de chlorure de sodium, séchée sur sulfate de sodium et concentrée à sec sous pression réduite (30 mm de mercure; 4 kPa) à 40° C pour donner 4,2 g d'amino-7 benzhydryloxycarbonyl-2 oxo-8 oxyde-5 [(pyridyl-3)-2 thiazolyl-5]-3 thia-5 aza-1 bicyclo-[4.2.0]-octène-2 brut sous la forme d'un solide beige (Rf = 0,5, chromatoplaque de gel de silice éluée par un mélange de dichlorométhane et d'éthanol 90/10 en volumes). Ce produit est repris dans 120 $cm^3$ de dichlorométhane auquel on ajoute 5,15 g d'acide syn t-butoxycarbonylméthoxyimino-2 (tritylamino-2 thiazolyl-4)-2 acétique puis 30 $cm^3$ de N,N-diméthylformamide et 0,06 g de diméthylamino-4 pyridine. La solution est refroidie à +5° C puis on ajoute 1,96 g de N,N'-dicyclohexylcarbodiimide. On agite 1½ h à 5° C puis 1½ h à 20° C, puis encore 5 min à 20° C après addition de 0,2 $cm^3$ d'acide acétique. Le dichlorométhane est évaporé sous pression réduite (30 mm de mercure; 4 kPa) à 30° C et le résidu est repris par 100 $cm^3$ d'acétate d'éthyle. Après filtration, lavage successivement par 200 $cm^3$ d'eau, 200 $cm^3$ de solution de bicarbonate de sodium à 5% et 200 $cm^3$ de solution saturée de chlorure de sodium, la phase organique est séchée sur sulfate de sodium et concentrée à sec sous pression réduite (30 mm de mercure; 4 kPa) à 30° C. Le résidu est chromatographié sur une colonne de 400 $cm^3$ de silice (0,06-0,2 mm) de diamètre 4 cm en éluant par des mélanges de cyclohexane et d'acétate d'éthyle: successivement 4 l de mélange 50/50, 1 l de mélange 40/60, 1 l de mélange 30/70, 1 l de mélange 20/80 (en volumes) puis par 3 l d'acétate d'éthyle et en recueillant des fractions de 250 $cm^3$. Les fractions 29 à 38 sont réunies et concentrées à sec sous pression réduite (30 mm de mercure; 4 kPa) à 40° C. La meringue obtenue est concrétée par de l'éther éthylique pour donner 3,6 g de benzhydryloxycarbonyl-2 [t-butoxycarbonylméthoxyimino]-2 (tritylamino-2 thiazolyl-4)-2 acétamido]-7 oxo-8 oxyde-5 [(pyridyl-3)-2 thiazolyl-5]-3 thia-5 aza-1 bicyclo-[4.2.0]-octène-2 isomère syn sous la forme d'un solide crème.

Spectre infrarouge (KBr, bandes caractéristiques en $cm^{-1}$) 3400, 1805, 1730, 1690, 1520, 1500, 1450, 1370, 1230, 1160, 755, 705.

Spectre RMN du proton (250 MHz, DMSO $d_6$, δ en ppm, J en Hz) 1,45 [S, 9H, $-C(CH_3)_3$]; 3,85 et 4,40 (2D, J = 19, 2H, $-S-CH_2-$); 4,57 (S, 2H, $=N-OCH_2-$); 5,13 (D, J = 4,5, 1H, $-H$ en 6); 6,03 (DD, J = 7,5 et 4,5, 1H, $-H$ en 7); 6,92 (S, 1H, $-H$ en 5 du thiazole); 6,99 [S, 1H, $-COO-C\underline{H}(C_6H_5)_2$]; 6,95 à 7,45 (Mt, 25H, aromatiques); 7,57 (DDD, J = 8,5 et 0,5, 1H, $-H$ en 5 de la pyridine); 7,95 (S, 1H, $-H$ en 4 du thiazole); 8,1 (DDD, J = 8, 2 et 1,5, 1H, H en 4 de la pyridine); 8,71 (DD, J = 5 et 1,5, 1H, $-H$ en 6 de la pyridine); 8,89 [S, 1H, $-N\underline{H}-C(C_6H_5)_3$]; 8,93 (DD, J = 2 et 0,5, 1H, H en 2 de la pyridine); 8,99 (D, J = 7,5, 1H, $-CONH-$).

Le benzhydryloxycarbonyl-2 t-butoxycarbonylamino-7 oxo-8 oxyde-5 [(pyridyl-3)-2 thiazolyl-5]-3 thia-5 aza-1 bicyclo-[4.2.0]-octène-2 peut être préparé de la manière suivante:

A une solution refroidie à −10° C de 31,3 g de benzhydryloxycarbonyl-2 t-butoxycarbonylamino-7 oxo-8 [(pyridyl-3)-2 thiazolyl-5]-3 thia-5 aza-1 bicyclo-[4.2.0]-octène-2 dans 300 cm³ de dichlorométhane est ajoutée en 40 min une solution de 8,65 g d'acide méthachloroperbenzoïque dans 300 cm³ de dichlorométhane. On ajoute 500 cm³ de solution de bicarbonate de sodium à 5%. La phase organique est lavée par 500 cm³ de solution saturée de chlorure de sodium, séchée sur sulfate de magnésium et concentrée à sec sous pression réduite (30 mm de mercure; 4 kPa) à 30° C. Le résidu est concrété par de l'éther éthylique pour donner 27,7 g de benzhydryloxycarbonyl-2 t-butoxycarbonylamino-7 oxo-8 oxyde-5 [(pyridyl-3)-2 thiazolyl-5]-3 thia-5 aza-1 bicyclo-[4.2.0]-octène-2 sous la forme d'un solide crème.

Spectre infrarouge ($CHBr_3$, bandes caractéristiques en $cm^{-1}$) 3410, 1800, 1720, 1505, 1370, 1235, 1050, 760, 745.

Spectre RMN du proton (250 MHz, $CDCl_3$, δ en ppm, J en Hz) 1,5 [S, 9H, $-C(CH_3)_3$]; 3,35 et 4,02 (DD et D, J = 19 et 1 et J = 19, 2H, $-S-CH_2-$); 4,58 (D large, J = 5, 1H, −H en 6); 5,85 (D, J = 10, 1H, −CO−NH−); 5,94 (DD, J = 10 et 5, 1H, −H en 7); 6,96 [S, 1H, $-COO-\underline{CH}(C_6H_5)_2$]; 6,90 à 7,40 (Mt, aromatiques); 7,36 (DD, J = 8 et 5, 1H, −H en 5 de la pyridine); 7,61 (S, 1H, −H en 4 du thiazole); 8,02 (DDD, J = 8, 2 et 1,5, 1H, −H en 4 de la pyridine); 8,67 (DD, J = 5 et 1,5, 1H, −H en 6 de la pyridine); 8,93 (D, J = 2, 1H, −H en 2 de la pyridine).

Le benzhydryloxycarbonyl-2 t-butoxycarbonylamino-7 oxo-8 [(pyridyl-3)-2 thiazolyl-5]-3 thia-5 aza-1 bicyclo-[4.2.0]-octène-2 peut être préparé de la manière suivante:

On ajoute à une solution de 120 g de benzhydryloxycarbonyl-2 (bromo-1 oxo-2 éthyl)-3 t-butoxycarbonylamino-7 oxo-8 thia-5 aza-1 bicyclo-[4.2.0]-octène-2 (mélange des diastéréo-isomères) dans 1200 cm³ de tétrahydrofuranne, 28,2 g de thionicotinamide et 16,5 cm³ de pyridine. On agite le mélange réactionnel à 50° C pendant 80 min. Après refroidissement à +5° C on ajoute successivement en 5 min 15,8 cm³ de chlorure de méthanesulfonyle et 57,3 cm³ de triéthylamine. Le mélange réactionnel est maintenu 30 min à +5° C puis réchauffé à 20° C et versé dans un mélange de 1 l d'acétate d'éthyle et 2 l de solution de bicarbonate de sodium à 5%. La phase organique est lavée par 2 l de solution de bicarbonate de sodium à 5% puis par 2 l de solution demi-saturée de chlorure de sodium. Après séchage sur sulfate de magnésium et évaporation à sec du solvant sous pression réduite (4 kPa) à 30° C on chromatographie le résidu obtenu sur une colonne de 9 cm de diamètre contenant 3,4 l de gel de silice (0,06-0,2 mm), en recueillant des fractions de 1 l. On élue successivement par: 11 l de dichlorométhane puis des mélanges de dichlorométhane et d'acétate d'éthyle 94/6 (16 l), 88/12 (4 l) 75/25 (12 l) et 70/30 (10 l) (proportions en volumes). Les fractions 32 à 52 sont réunies, concentrées à sec et le résidu concrété par de l'oxyde d'isopropyle pour donner 37,1 g de benzhydryloxycarbonyl-2 t-butoxycarbonylamino-7 oxo-8 [(pyridyl-3)-2 thiazolyl-5]-3 thia-5 aza-1 bicyclo-[4.2.0]-octène-2 sous la forme d'un solide beige.

Spectre infrarouge ($CHBr_3$, bandes caractéristiques en $cm^{-1}$) 3430, 1780, 1725, 1590, 1575, 1510, 1460, 1420, 1370, 1240, 810, 765, 750.

Spectre RMN du proton (250 MHz, $CDCl_3$, δ en ppm, J en Hz) 1,49 [S, 9H, $-C(CH_3)_3$]; 3,61 et 3,78 (2D, J = 19, 2H, $-SCH_2-$); 5,1 (D, J = 5, 1H, −H en 6); 5,48 (D, J = 10, 1H, −CO−NH−); 5,76 (DD, J = 10 et 5, 1H, −H en 7); 6,95 [S, 1H, $-COO\underline{CH}(C_6H_5)_2$]; 6,90 à 7,4 (Mt, aromatiques); 7,38 (DDD, J = 7,5, 5 et 0,5, −H en 5 de la pyridine); 7,59 (S, 1H, H en 4 du thiazole); 8,04 (DDD, J = 7,5, 2 et 1,5, 1H, −H en 4 de la pyridine); 8,67 (DD, J = 5 et 1,5, 1H, −H en 6 de la pyridine); 8,94 (D, J = 2, 1H, −H en 2 de la pyridine).

Le benzhydryloxycarbonyl-2 (bromo-1 oxo-2 éthyl)-3 t-butoxycarbonylamino-7 oxo-8 thia-5 aza-1 bicyclo-[4.2.0]-octène-2 (mélange des diastéréo-isomères peut être préparé de la manière suivante:

A une solution refroidie à −55° C de 2 g de benzhydryloxycarbonyl-2 t-butoxycarbonylamino-7 (diméthylamino-2 vinyl)-3 oxo-8 thia-5 aza-1 bicyclo-[4.2.0]-octène-2 (isomère E) dans 11 cm³ de tétrahydrofuranne sec on ajoute goutte à goutte, en 5 min, une solution de 0,2 cm³ de brome dans 2 cm³ de chlorure de méthylène sec. Le mélange réactionnel est agité pendant 1 h à −60° C puis versé dans un mélange de 200 cm³ d'acétate d'éthyle et 200 cm³ d'eau glacée. La couche organique est lavée par 100 cm³ de solution demi-saturée de bicarbonate de sodium puis par 100 cm³ d'eau et 100 cm³ de solution demi-saturée de chlorure de sodium et séchée sur sulfate de sodium en présence de noir décolorant. Après filtration, le solvant est évaporé sous pression réduite (30 mm de mercure; 4 kPa) à 30° C. Le résidu est repris par 50 cm³ d'oxyde d'isopropyle que l'on évapore sous pression réduite (30 mm de mercure; 4 kPa) à 30° C. On obtient 1,8 g de benzhydryloxycarbonyl-2 (bromo-1 oxo-2 éthyl)-3 t-butoxycarbonylamino-7 oxo-8 thia-5 aza-1 bicyclo-[4.2.0]-octène-2 sous la forme d'un solide beige clair (mélange des deux épimères du bromoaldéhyde).

Spectre infrarouge ($CHBr_3$), bandes caractéristiques ($cm^{-1}$) 3420, 1790, 1725, 1505, 1455, 1390, 1370, 1245, 1225, 760, 745.

Spectre de RMN du proton ($CDCl_3$, 350 MHz, δ en ppm, J en Hz)

*Epimère A:*

1,42 [s, 9H, $(CH_3)_3C-$]; 3,71 et 3,55 (AB, J = 17,5, 2H, $-SCH_2-$); 5,06 (d, J = 4, 1H, H en 6); 5,22 (d, J = 9, 1H, −NH−); 5,65 (dd, J = 4 et 9, 1H, H en 7); 6,01 (s, 1H, $-\underline{CH}Br-$); 6,99 (s, 1H, $-CHAr_2$); 7,3 à 7,5 (m, 10H, aromatiques); 9,31 (s, 1H, −CHO).

*Epimère B:*

1,42 [s, 9H, $(CH_3)_3C-$]; 3,35 et 3,65 (AB, J = 17,5, 2H, $-SCH_2-$); 5,01 (d, J = 4, 1H, H en 6); 5,29 (d, J = 9, 1H, $-NH-$); 5,72 (dd, J = 4 et 9, 1H, H en 7); 6,00 (s, 1H, $-CHBr-$); 6,92 (s, 1H, $-CHAr_2$); 7,3 à 7,5 (m, 10H, aromatiques); 9,30 (s, 1H, $-CHO$).

Le benzhydryloxycarbonyl-2 t-butoxycarbonylamino-7 (diméthylamino-2 vinyl)-3 oxo-8 thia-5 aza-1 bicyclo-[4.2.0]-octène-2 (isomère E) peut être préparé comme décrit dans le brevet belge N° 883415.

*Exemple 3:*

Une solution de 8,5 g de benzhydryloxycarbonyl-2 [(t-butoxycarbonyl-2 propyl-2)-oxyimino-2 (tritylamino-2 thiazolyl-4)-2 acétamido]-7 oxo-8 oxyde-5 [(pyridyl-3)-2 thiazolyl-5]-3 thia-5 aza-1 bicyclo-[4.2.0]-octène-2 isomère syn dans 40 $cm^3$ de N,N-diméthylformamide contenant 0,55 $cm^3$ d'iodure de méthyle est traitée selon le mode opératoire décrit dans l'exemple 2, pour donner 8 g d'iodure de benzhydryloxycarbonyl-2 [(t-butoxycarbonyl-2 propoxy-2)-imino-2 (tritylamino-2 thiazolyl-4)-2 acétamido]-7 [(méthyl-pyridinio-3)-2 thiazolyl-5]-3 oxo-8 oxyde-5 thia-5aza-1 bicyclo-[4.2.0]-octène-2 isomère syn sous la forme d'un solide jaune.

Spectre infrarouge (KBr, bandes caractéristiques en $cm^{-1}$) 3380, 1800, 1730, 1685, 1515, 1495, 1450, 1370, 1140, 755, 700, 670.

Spectre RMN du proton (350 MHz, DMSO $d_6$, δ en ppm, J en Hz) 1,37 [S, 9H, $-C(CH_3)_3$]; 1,46 [2S, 6H, $> C(CH_3)_2$]; 3,91 et 4,5 (2D, J = 19, 2H, $-S-CH_2-$); 4,47 (S, 3H, $\geqslant \overset{+}{N}-CH_3$); 5,16 (D, J = 4,5, 1H, −H en 6); 6,07 (DD, J = 8 et 5, 1H, −H en 7); 6,80 (S, 1H, −H en 5 du thiazole); 6,97 [S, 1H, $-COO-CH(C_6H_5)_2$]; 6,90 à 7,4 (Mt, aromatiques); 8,1 (S, 1H, −H en 4 du thiazole); 8,25 (DD, J = 8 et 6, 1H, −H en 5 du pyridinio); 8,4 (D, J = 8, 1H, $-CO-NH-$); 8,74 (D, J = 8, 1H, −H enn 4 du pyridinio); 8,76 [S, 1H, $-NH-C(C_6H_5)_3$]; 9,06 (D, J = 6, 1H, −H en 6 du pyridinio); 9,42 (S, 1H, −H en 2 du pyridinio).

Une solution de 8 g d'iodure de benzhydryloxycarbonyl-2 [(t-butoxycarbonyl-2 propyl-2)-oxyimino-2 (tritylamino-2 thiazolyl-4)-2 acétamido]-7 [(méthyl-1 pyridinio-3)-2 thiazolyl-5]-3 oxo-8- oxyde-5 thia-5 aza-1 bicyclo-[4.2.0]-octène-2 isomère syn dans un mélange de 100 $cm^3$ de dichlorométhane et de 2,26 $cm^3$ de N,N-diméthylacétamide est traité par 0,96 $cm^3$ de trichlorure de phosphore, selon le mode opératoire de l'exemple 2 pour donner 8 g de benzhydryloxycarbonyl-2 [(t-butoxycarbonyl-2 propyl-2)-oxyimino-2 (tritylamino-2 thiazolyl-4)-2 acétamido]-7 [(méthyl-1 pyridinio-3)-2 thiazolyl-5]-3 oxo-8 thia-5 aza-1 bicyclo-[4.2.0]-octène-2 isomère syn sous la forme d'un solide jaune.

Spectre infrarouge (KBr, bandes caractéristiques en $cm^{-1}$) 1790, 1730, 1680, 1520, 1495, 1450, 1370, 1140, 755, 700, 670.

Spectre RMN du proton (350 MHz, DMSO $d_6$, δ en ppm, J en Hz) 1,4 [S, 9H, $-C(CH_3)_3$]; 1,47 et 1,49 [2S, 6H, $> C(CH_3)_2$]; 3,92 et 4,09 (2D, J = 19, 2H, $-S-CH_2-$); 4,48 (S, 3H, $\geqslant \overset{+}{N}-CH_3$); 5,34 (D, J = 5, 1H, −H en 6); 5,95 (DD, J = 8 et 5, 1H, −H en 7); 6,80 (S, 1H, −H en 5 du thiazole); 6,94 [S, 1H, $-COO-CH(C_6H_5)_2$]; 6,9 à 7,4 (Mt, aromatiques); 8,10 (S, 1H, −H en 4 du thiazole); 8,24 (DD, J = 8 et 6, 1H, H en 5 du pyridinio); 8,73 (D, J = 8, 1H, −H en 4 du pyridinio); 9,09 (D, J = 6, 1H, −H en 6 du pyridinio); 9,42 (S, 1H, −H en 2 du pyridinio); 9,61 (D, J = 8, 1H, $-CO-NH-$); 9,75 [Mf, 1H, $-NH-C(C_6H_5)_3$].

Une solution de 8 g de benzhydryloxycarbonyl-2 [(t-butoxycarbonyl-2 propyl-2)-oxyimino-2 (tritylamino-2 thiazolyl-4)-2 acétamido]-7 [(méthyl-1 pyridinio-3)-2 thiazolyl-5]-3 oxo-8 thia-5 aza-1 bicyclo-[4.2.0]-octène-2 isomère syn dans 160 $cm^3$ d'acide formique contenant 16 $cm^3$ d'anisole est traitée selon le mode opératoire décrit dans l'exemple 2 pour donner 4,5 g d'[(amino-2 thiazolyl-4)-2 (carboxy-2 propyl-2) oxyimino-2 acétamido]-7 carboxylato-2 [(méthyl-1 pyridinio-3)-2 thiazolyl-5]-3 oxo-8 thia-5 aza-1 bicyclo-[4.2.0]-octène-2 isomère syn à l'état d'iodhydrate brut. Le produit brut obtenu est traité par de la résine IR 45 basique selon le mode opératoire de l'exemple 2 pour obtenir 1,4 g d'[(amino-2 thiazolyl-4)-2 (carboxy-2 propyl-2) oxyimino-2 acétamido]-7 carboxylato-2 [(méthyl-1 pyridinio-3)-2 thiazolyl-5]-3 oxo-8 thia-5 aza-1 bicyclo-[4.2.0]-octène-2 isomère syn sous la forme d'un solide jaune.

Spectre infrarouge (KBr, bandes caractéristiques en $cm^{-1}$) 3380, 3250, 3100-2300, 1770, 1680, 1610, 1520, 1360, 1160, 670.

Spectre RMN du proton (250 MHz, DMSO $d_6$, δ en ppm, J en Hz) 1,47 et 1,52 [2S, 6H, $> C(CH_3)_2$]; 3,60 à 3,90 (Mt, $-S-CH_2-$); 4,4 (S, 3H, $\geqslant \overset{+}{N}-CH_3$); 5,16 (D, J = 5, 1H, −H en 6); 5,79 (DD, J = 9 et 5, 1H, −H en 7); 6,76 (S, 1H, −H en 5 du thiazole); 7,32 (Mf, 2H, $-NH_2$); 7,98 (S, 1H, −H en 4 du thiazole); 8,07 (Mt, 1H, −H en 5 du pyridinio); 8,75 (D, J = 7,5, 1H, −H en 4 du pyridinio); 9,06 (Mf, 1H, −H en 6 du pyridinio); 9,39 (S large, 1H, −H en 2 du pyridinio); 9,89 (Mf, 1H, $-CO-NH-$).

Le benzhydryloxycarbonyl-2 [(t-butoxycarbonyl-2 propyl-2) oxyimino-2 (tritylamino-2 thiazolyl-4)-2 acétamido]-7 oxo-8 oxyde-5 [(pyridyl-3)-2 thiazolyl-5]-3 thia-5 aza-1 bicyclo-[4.2.0]-octène-2 isomère syn peut être préparé de la manière suivante.

On ajoute lentement une solution de 1,08 g d'amino-7 benzhydryloxycarbonyl-2 oxo-8 oxyde-5 [(pyridyl-3)-2 thiazolyl-5]-3 thia-5 aza-1 bicyclo-[4.2.0]-octène-2 dans 20 $cm^3$ de dichlorométhane à une solution de 2,2 mmol de chlorure de l'acide (t-butoxycarbonyl-2 propyl-2) oxyimino-2 (tritylamino-2 thiazolyl-4)-2 acétique isomère syn (préparé à partir de l'acide correspondant et de pentachlorure de phosphore selon le mode opératoire décrit dans le brevet belge N° 876538) dans 20 $cm^3$ de dichlorométhane contenant 0,6 $cm^3$ de triéthylamine et refroidi à

$-10°$ C. Après 45 min à $-5°$ C le mélange réactionnel est versé sur 100 $cm^3$ de solution saturée de bicarbonate de sodium et la phase organique est lavée par 50 $cm^3$ de solution demi-saturée de bicarbonate de sodium, 50 $cm^3$ d'acide chlorhydrique 0,1 N et 2 fois par 50 $cm^3$ de solution saturée de chlorure de sodium. Le résidu obtenu après séchage et concentration à sec sous pression réduite (30 mm de mercure; 4 kPa) à 30° C de la solution organique est chromatographié sur une colonne (diamètre: 2,2 cm) de 100 $cm^3$ de gel de silice (0,2-0,06 mm) en éluant par de l'acétate d'éthyle et en recueillant des fractions de 50 $cm^3$. Les fractions 7 à 12 sont réunies et concentrées à sec sous pression réduite (30 mm de mercure; 4 kPa) à 30° C pour donner 1,7 g de benzhydryloxycarbonyl-2 [(t-butoxycarbonyl-2 propyl-2) oxyimino-2 (tritylamino-2 thiazolyl-4)-2 acétamido]-7 oxo-8 oxyde-5 [(pyridyl-3)-2 thiazolyl-5]-3 thia-5 aza-1 bicyclo-[4.2.0]-octène-2 isomère syn sous la forme d'une meringue jaune.

Spectre infrarouge (KBr, bandes caractéristiques en $cm^{-1}$) 1805, 1730, 1690, 1510, 1500, 1450, 1370, 1145, 760, 700.

Spectre RMN du proton (250 MHz, $CDCl_3$, δ en ppm, J en Hz) 1,44 [S, 9H, $-C(CH_3)_3$]; 1,6 et 1,63 [2S, 6H, $>C(CH_3)_2$]; 3,37 et 3,98 (2D, J = 18, 2H, $-S-CH_2-$); 4,71 (D large, J = 5, 1H, −H en 6); 6,33 (DD, J = 10 et 5, 1H, −H en 7); 6,73 (S, 1H, −H en 5 du thiazole); 6,98 [S, 1H, $-COO-\underline{CH}(C_6H_5)_2$]; 7 à 7,5 [Mt, aromatiques et $-\underline{NH}-C(C_6H_5)_3$]; 7,4 (DD, J = 8 et 5, −H en 5 de la pyridine); 7,61 (S, 1H, −H en 4 du thiazole); 8,01 (D, J = 10, 1H, −CO−NH−); 8,05 (DT, J = 8 et 1,5, 1H, −H en 4 de la pyridine); 8,68 (DD, J = 5 et 1,5, 1H, −H en 6 de la pyridine); 8,94 (D, J = 1,5, 1H, −H en 2 de la pyridine).

*Exemple 4:*

Une solution de 3,1 g de benzhydryloxycarbonyl-2 [méthoxyimino-2 (tritylamino-2 thiazolyl-4)-2 acétamido]-7 oxo-8 oxyde-5 [(pyridyl-3)-2 thiazolyl-5]-3 thia-5 aza-1 bicyclo-[4.2.0]-octène-2 isomère syn et de 0,58 $cm^3$ de bromoacétate de t-butyle dans 15 $cm^3$ de N,N-diméthylformamide est agitée pendant 48 h à 20° C, puis 16 h à 35° C et versé dans 300 $cm^3$ d'acétate d'éthyle. Le précipité est essoré, lavé 5 fois par 20 $cm^3$ d'acétate d'éthyle et 3 fois par 20 $cm^3$ d'éther éthylique pour donner 3,2 g de bromure de benzhydryloxycarbonyl-2 [(t-butoxycarbonylméthyl-1 pyridinio-3)-2 thiazolyl-5]-3 [méthoxyimino-2 (tritylamino-2 thiazolyl-4)-2 acétamido]-7 oxo-8 oxyde-5 thia-5 aza-1 bicyclo-[4.2.0]-octène-2 isomère syn sous la forme d'un solide jaune.

Spectre infrarouge (KBr, bandes caractéristiques en $cm^{-1}$) 1800, 1740, 1685, 1515, 1495, 1450, 1375, 1155, 1040, 755, 705.

Spectre RMN du proton (250 MHz, DMSO $d_6$, δ en ppm, J en Hz) 1,52 [S, 9H, $-C(CH_3)_3$]; 3,87 (S, 3H, $=NOCH_3$); 3,90 et 4,41 (2D, J = 19, 2H, $-S-CH_2-$); 5,14 (D, J = 5, 1H, H en 6); 5,72 (S, 2H, $\geqslant \overset{+}{N}-CH_2-COO-$); 5,97 (DD, J = 8 et 5, 1H, H en 7); 6,82 (S, 1H, H en 5 du thiazole); 6,95 [S, 1H, $-COO-\underline{CH}(C_6H_5)_2$]; 6,9 à 7,45 (Mt, 25H, aromatiques); 8,05 (S, 1H, H en 4 du thiazole); 8,35 (DD, J = 8,5 et 6, 1H, H en 5 du pyridinio); 8,81 [S, 1H, $-\underline{NH}-C(C_6H_5)_3$]; 8,86 (D, J = 8,5, 1H, H en 4 du pyridinio); 9,16 (D, J = 6, 1H, H en 6 du pyridinio); 9,23 (D, J = 8, 1H, −CO−NH−); 9,63 (S, 1H, H en 2 du pyridinio).

Une solution de 3,1 g de bromure de benzhydryloxycarbonyl-2 [(t-butoxycarbonylméthyl-1 pyridinio-3)-2 thiazolyl-5]-3 [méthoxyimino-2 (tritylamino-2 thiazolyl-4)-2 acétamido]-7 oxo-8 oxyde-5 thia-5 aza-1 bicyclo-[4.2.0]-octène-2 isomère syn dans un mélange de 25 $cm^3$ de dichlorométhane et de 1,06 $cm^3$ de N,N-diméthylacétamide est refroidie à $-5°$ C et traitée par 0,47 $cm^3$ de trichlorure de phosphore puis traitée selon le mode opératoire de l'exemple 1 pour donner 3 g de bromure de benzhydryloxycarbonyl-2 [(t-butoxycarbonylméthyl-1 pyridinio-3)-2 thiazolyl-5]-3 [méthoxyimino-2 (tritylamino-2 thiazolyl-4)-2 acétamido]-7 oxo-8 thia-5 aza-1 bicyclo-[4.2.0]-octène-2 isomère syn sous la forme d'un solide jaune.

Spectre infrarouge (KBr, bandes caractéristiques en $cm^{-1}$) 1790, 1740, 1680, 1520, 1500, 1450, 1375, 1155, 1045, 750, 705.

Spectre RMN du proton (250 MHz, DMSO $d_6$, δ en ppm, J en Hz) 1,52 [s, 9H, $-C(CH_3)_3$]; 3,91 et 4,07 (2D, J = 17,5, 2H, $-S-CH_2-$); 3,93 (S, 3H, $=N-O-CH_3$); 5,34 (D, J = 5, 1H, H en 6); 5,75 (S, 2H, $\geqslant \overset{+}{N}-CH_2-COO-$); 5,94 (DD, J = 8 et 5, 1H, H en 7); 6,85 (S, 1H, −H en 5 du thiazole); 6,93 [S, 1H, $-COO-\underline{CH}(C_6H_5)_2$]; 6,9 à 7,45 (Mt, 25H, aromatiques); 8,09 (S, 1H, H en 4 du thiazole); 8,36 (DD, J = 8 et 6, 1H, H en 5 du pyridinio); 8,87 (D, J = 8, 1H, H en 4 du pyridinio); 9,19 (D, J = 6, 1H, H en 6 du pyridinio); 9,63 (S, 1H, H en 2 du pyridinio); 9,83 (D, J = 8, 1H, −CO−NH−); 9,90 [Mf, 1H, $-\underline{NH}-C(C_6H_5)_3$].

Une solution de 3,1 g de bromure de benzhydryloxycarbonyl-2 [(t-butoxycarbonylméthyl-1 pyridinio-3]-2 thiazoyl-5]-3 [méthoxyimino-2 (tritylamino-2 thiazolyl-4)-2 acétamido]-7 oxo-8 thia-5 aza-1 bicyclo-[4.2.0]-octène-2 isomère syn dans un mélange de 50 $cm^3$ d'acide formique et 5 $cm^3$ d'anisole est traitée selon le mode opératoire de l'exemple 8 pour donner 1,55 g de bromhydrate d'[(amino-2 thiazolyl-4)-2 méthoxyimino-2 acétamido]-7 carboxylato-2 [(carboxyméthyl-1 pyridinio-3)-2 thiazolyl-5]-3 oxo-8 thia-5 aza-1 bicyclo-[4.2.0]-octène-2 isomère syn brut sous la forme d'un solide jaune. Le produit brut obtenu est repris dans un mélange de 200 $cm^3$ d'eau distillée et de 100 $cm^3$ d'acétate d'éthyle. L'insoluble est filtré, lavé par 4 fois 25 $cm^3$ d'eau distillée, les filtrats sont rassemblés, décantés et la phase aqueuse est lavée par 100 $cm^3$ d'acétate d'éthyle puis agitée avec 30 $cm^3$ de résine IR 45 basique jusqu'à atteindre un pH de 3,8. La résine est éliminée par filtration et lavée 4 fois par 10 $cm^3$ d'eau distillée. Les filtrats réunis sont lyophilisés pour donner 0,85 g d'[(amino-2 thiazolyl-4)-2 méthoxyimino-2 acétamido]-7 carboxylato-2 [(carboxyméthyl-1 pyridinio-3)-2 thiazolyl-5]-3

oxo-8 thia-5 aza-1 bicyclo-[4.2.0]-octène-2 isomère syn sous la forme d'un lyophilisat orangé.

Spectre infrarouge (KBr, bandes caractéristiques en $cm^{-1}$) 3400, 1770, 1670, 1640, 1620, 1530, 1360, 1040.

Spectre RMN du proton (250 MHz, DMSO $d_6$, $\delta$ en ppm, J en Hz) 3,80 à 4 (Mt, 5H, $=N-O-CH_3$ et $-S-CH_2-$); 5,24 (D, J = 5 et Mf, 3H, H en 6 et $\geqslant \underset{+}{N}-CH_2-COO-$); 5,81 (DD, J = 8 et 5, 1H, H en 7); 6,77 (S, 1H, H en 5 du thiazole); 7,27 (Mf, 2H, $-NH_2$); 8,05 à 8,15 (S et Mt, 2H, H en 4 du thiazole et H en 5 du pyridinio); 8,85 (D, J = 8, 1H, H en 4 du pyridinio); 8,94 (Mf, 1H, H en 6 du pyridinio); 9,46 (S large, 1H, H en 2 du pyridinio); 9,69 (D, J = 8, 1H, $-CO-NH-$).

*Exemple 5:*

Une solution de 3,0 g de benzhydryloxycarbonyl-2 [méthoxyimino-2 (tritylamino-2 thiazolyl-4)-2 acétamido]-7 oxo-8 oxyde-5 [(pyridyl-3)-2 thiazolyl-5]-3 thia-5 aza-1 bicyclo-[4.2.0]-octène-2 isomère syn et de 0,74 g d'iodoacétamide dans 15 $cm^3$ de N,N-diméthylformamide est agitée pendant 48 h à 20° C puis 16 h à 35° C, puis versée dans 300 $cm^3$ d'acétate d'éthyle. Le précipité est essoré, lavé 5 fois par 20 $cm^3$ d'acétate d'éthyle et 3 fois par 20 $cm^3$ d'éther éthylique pour donner 3,2 g d'iodure de benzhydryloxycarbonyl-2 [(carbamoylméthyl-1 pyridinio-3)-2 thiazolyl-5]-3 [méthoxyimino-2 (tritylamino-2 thiazolyl-4)-2 acétamido]-7 oxo-8 oxyde-5 thia-5 aza-1 bicyclo-[4.2.0]-octène-2 isomère syn sous la forme d'un solide jaune.

Spectre infrarouge (KBr, bandes caractéristiques en $cm^{-1}$) 3380, 1800, 1730, 1695, 1520, 1495, 1040, 760, 705.

Spectre RMN du proton (250 MHz, DMSO $d_6$, $\delta$ en ppm, J en Hz) 3,87 (S, 3H, $=NOCH_3$); 3,88 et 4,40 (2D, J = 18, 2H, $-S-CH_2-$); 5,13 (D, J = 4,5, 1H, H en 6); 5,54 (S large, 2H, $\geqslant \underset{+}{N}-CH_2-$); 5,97 (DD, J = 8 et 4,5, 1H, H en 7); 6,81 (S, 1H, H en 5 du thiazole); 6,95 [S, 1H, $-COO-\underline{CH}(C_6H_5)_2$]; 6,90 à 7,4 (Mt, 25H, aromatiques); 7,80 et 8,09 (2S larges, 2H, $-CO-NH_2$); 8,03 (S, 1H, H en 4 du thiazole); 8,28 (DD, J = 7,5 et 6, 1H, H en 5 du pyridinio); 8,79 (S large, 1H, $-NH-$); 8,81 (D, J = 7,5, 1H, H en 4 du pyridinio); 9,05 (D, J = 6, 1H, H en 6 du pyridinio); 9,21 (D, J = 8, 1H, $-CO-NH-$); 9,47 (S, 1H, H en 2 du pyridinio).

Une solution de 3,2 g d'iodure de benzhydryloxycarbonyl-2 [(carbamoylméthyl-1 pyridinio-3)-2 thiazolyl-5]-3 [méthoxyimino-2 (tritylamino-2 thiazolyl-4)-2 acétamido]-7 oxo-8 oxyde-5 thia-5 aza-1 bicyclo-[4.2.0]-octène-2 isomère syn dans un mélange de 30 $cm^3$ de dichlorométhane et de 6 $cm^3$ de N,N-diméthylacétamide est refroidie à −5° C et traitée par 0,5 $cm^3$ de trichlorure de phosphore, puis traitée selon le mode opératoire de l'exemple 1 pour donner 3,2 g d'iodure de benzhydryloxycarbonyl-2 [(carbamoylméthyl-1 pyridinio-3)-2 thiazolyl-5]-3 [méthoxyimino-2 (tritylamino-2 thiazolyl-4)-2 acétamido]-7 oxo-8 thia-5 aza-1 bicyclo-[4.2.0]-octène-2 isomère syn sous la forme d'un solide jaune.

Spectre RMN du proton (250 MHz, DMSO $d_6$, $\delta$ en ppm, J en Hz) 3,9 (S, 3H, $=N-OCH_3$); 3,90 et 4 (2D, J = 18, 2H, $-S-CH_2-$); 5,33 (D, J = 5, 1H, H en 6); 5,61 (S large, 2H, $\geqslant \underset{+}{N}-CH_2-$); 5,94 (DD, J = 8 et 5, 1H, H en 7); 6,82 (S, 1H, H en 5 du thiazole); 6,93 [S, 1H, $-COO-\underline{CH}(C_6H_5)_2$]; 6,9 à 7,50 (Mt, 25H, aromatiques); 7,82 et 8,26 (2S larges, 2H, $-CONH_2$); 8,09 (S, 1H, H en 4 du thiazole); 8,29 (Mt, 1H, H en 5 du pyridinio); 8,82 (D, J = 8, 1H, H en 4 du pyridinio); 9,10 (D, J = 6, 1H, H en 6 du pyridinio); 9,50 (S, 1H, H en 2 du pyridinio); 9,67 (Mf, 1H, $-NH-$); 9,82 (D, J = 8, 1H, $-CONH-$).

Une solution de 3,3 g d'iodure de benzhydryloxycarbonyl-2 [(carbamoylméthyl-1 pyridinio-3)-2 thiazolyl-5]-3 [méthoxyimino-2 (tritylamino-2 thiazolyl-4)-2 acétamido]-7 oxo-8 thia-5 aza-1 bicyclo-[4.2.0]-octène-2 isomère syn dans un mélange de 50 $cm^3$ d'acide formique et 5 $cm^3$ d'anisole est traitée selon le mode opératoire de l'exemple 8 pour donner 2,35 g d'iodhydrate d'[(amino-2 thiazolyl-4)-2 méthoxyimino-2 acétamido]-7 carboxylato-2 [(carbamoylméthyl-1 pyridinio-3)-2 thiazolyl-5]-3 oxo-8 thia-5 aza-1 bicyclo-[4.2.0]-octène-2 isomère syn brut sous la forme d'un solide orangé. Le solide brut est repris dans un mélange de 200 $cm^3$ d'eau distillée et de 100 $cm^3$ d'acétate d'éthyle. La phase organique est lavée par 100 $cm^3$ d'eau distillée; les phases aqueuses sont décantées et rassemblées puis lavées par 100 $cm^3$ d'acétate d'éthyle puis agitée avec 30 $cm^3$ de résine IR 45 basique jusqu'à atteindre un pH de 3. La résine est éliminée par filtration et lavée 4 fois par 10 $cm^3$ d'eau distillée. Les filtrats réunis sont lyophilisés pour donner 1,22 g d'[(amino-2 thiazolyl-4)-2 méthoxyimino-2 acétamido]-7 carboxylato-2 [(carbamoylméthyl-1 pyridinio-3)-2 thiazolyl-5]-3 oxo-8 thia-5 aza-1 bicyclo-[4.2.0]-octène-2 isomère syn sous la forme d'un lyophilisat orangé.

Spectre RMN du proton (250 MHz, DMSO $d_6$, $\delta$ en ppm, J en Hz) 3,76 et 3,89 (2D, J = 18, 2H, $-S-CH_2-$); 3,88 (S, 3H, $=N-O-CH_3$); 5,18 (D, J = 5, 1H, H en 6); 5,54 (S large, 2H, $\geqslant \underset{+}{N}-CH_2-$); 5,68 (DD, J = 8 et 5, 1H, H en 7); 6,76 (S, 1H, H en 5 du thiazole); 7,25 (Mf, 2H, $-NH_2$); 7,74 et 8,28 (2S larges, 2H, $-CO-NH_2$); 8,10 (S, 1H, H en 4 du thiazole); 8,19 (Mt, 1H, H en 5 du pyridinio); 8,96 (Mt, 2H, H en 4 et H en 6 du pyridinio); 9,54 (S, 1H, H en 2 du pyridinio); 9,65 (D, J = 8, 1H, $-CONH-$).

*Exemple 6:*

Une solution de 3,3 g de benzhydryloxycarbonylamino-2 [(t-butoxycarbonyl-2 propyl-2 oxyimino)-2 (tritylamino-2 thiazolyl-4)-2 acétamido]-7 oxo-8 oxyde-5 [(pyridyl-3)-2 thiazolyl-5]-3 thia-5 aza-1 bicyclo-[4.2.0]-octène-2 isomère syn dans 10 $cm^3$ de N,N-diméthylformamide est traitée selon le mode opératoire décrit dans l'exemple 3 en remplaçant l'iodure de méthyle par un mé-

lange de 0,475 $cm^3$ de bromure de benzyle et de 0,05 g d'iodure de sodium pour donner 3,8 g de bromure de benzhydryloxycarbonyl-2 [(benzyl-1 pyridinio-3)-2 thiazolyl-5]-3 [(t-butoxycarbonyl-2 propyl-2 oxyimino)-2 (tritylamino-2 thiazolyl-4)-2 acétamido]-7 oxo-8 oxyde-5 thia-5 aza-1 bicyclo-[4.2.0]-octène-2 isomère syn sous la forme d'un solide jaune.

Spectre de RMN du proton (250 MHz, DMSO $d_6$, δ en ppm, J en Hz) 1,36 [S, 9H, $-C(CH_3)_3$]; 1,45 [S, 6H, $-C(CH_3)_2-$]; 3,92 et 4,49 (2d, J = 17,5, 2H, $-SOCH_2-$); 5,17 (d, J = 3,8, 1H, H en 6); 6,0 (S large, 2H, ⩾ $N^{\oplus}-\underline{CH_2}C_6H_5$); 6,08 (dd, J = 3,8 et 10, 1H, H en 7); 6,66 (t, J = 6, 1H, H en para du benzyle); 6,80 (S, 1H, H en 5 du thiazole); 6,88 (t, J = 6, 2H, H en méta du benzyle); 6,94 (S, 1H, $-CO_2\underline{C}HAr_2$); 7,11 (d, J = 6, 2H en ortho du benzyle); 7,15 à 7,75 (Mf, 25H, aromatiques); 8,09 (S, 1H, H en 4 du thiazole); 8,28 (dd, J = 6 et 8,8, 1H en 5 du pyridinio); 8,45 (d, J = 8,8, 1H, H en 4 du pyridinio); 8,76 (d, J = 10, 1H, $-CONH-$); 8,78 (S, 1H, $-NH-$ thiazole); 9,30 (d, J = 6, 1H, H en 6 du pyridinio); 9,68 (S, 1H, H en 2 du pyridinio).

3,8 g de bromure de benzhydryloxycarbonyl-2 [(benzyl-1 pyridinio-3)-2 thiazolyl-5]-3 [(t-butoxycarbonyl-2 propyl-2 oxyimino)-2 (tritylamino-2 thiazolyl-4)-2 acétamido]-7 oxo-8 oxyde-5 thia-5 aza-1 bicyclo-[4.2.0]-octène-2 isomère syn sont réduits par 0,52 $cm^3$ de trichlorure de phosphore selon le mode opératoire décrit dans l'exemple 3 pour donner 3,1 g de bromure de benzhydryloxycarbonyl-2 [(benzyl-1 pyridinio-3)-2 thiazolyl-5]-3 [(t-butoxycarbonyl-2 propyl-2 oxyimino)-2 (tritylamino-2 thiazolyl-4)-2 acétamido]-7 oxo-8 thia-5 aza-1 bicyclo-[4.2.0]-octène-2 isomère syn brut sous la forme d'un solide jaune.

Spectre de RMN du proton (250 MHz, DMSO $d_6$, δ en ppm, J en Hz) 1,39 [S, 9H, $-C(CH_3)_3$]; 1,46 et 1,48 [2S, 2 × 3H, $-C(CH_3)_2-$]; 3,9 et 4,08 (2d, J = 18, 2H, $-SCH_2-$); 5,34 (d, J = 5, 1H, H en 6); 5,94 (dd, J = 8 et 5, H en 7); 6,02 (S, 2H, ⩾ $N^{\oplus}-\underline{CH_2}C_6H_5$); 6,62 (t, J = 7,5, 1H, para du benzyle); 6,78 (S, 1H, H en 5 du thiazole); 6,85 (t, J = 7,5, 2H, méta du benzyle); 6,9 (S, 1H, $CO_2-\underline{C}HAr_2$); 7,07 (d, J = 7,5, 2H ortho du benzyle); 7 à 7,75 (mf, 25H, aromatiques); 8,09 (S, 1H, H en 4 du thiazole); 8,28 (dd, J = 6 et 7,5, 1H, H en 5 du pyridinio); 8,75 (d, J = 7,5, 1H, H en 4 du pyridinio); 9,33 (d, J = 6, 1H, H en 6 du pyridinio); 9,59 (d, J = 8, 1H, $-CONH-$); 9,67 (S, 1H, H en 2 du pyridinio); 9,4 et 9,9 (signal large, 1H, $-NH-$ thiazole).

On traite 3 g de bromure de benzhydryloxycarbonyl-2 [(benzyl-1 pyridinio-3)-2 thiazolyl-5]-3 [(t-butoxycarbonyl-2 propyl-2 oxyimino)-2 (tritylamino-2 thiazolyl-4)-2 acétamido]-7 oxo-8 thia-5 aza-1 bicyclo-[4.2.0]-octène-2 isomère syn brut par un mélange de 30 $cm^3$ d'acide formique et de 7 $cm^3$ d'anisole selon le mode opératoire décrit dans l'exemple 3 pour obtenir 1,1 g de bromhydrate d'[(amino-2 thiazolyl-4)-2 (carboxy-2 propyl-2 oxyimino)-2 acétamido]-8 [(benzyl-1 pyridinio-3)-2 thiazolyl-5]-3 carboxylato-2 oxo-8 thia-5 aza-1 bicyclo-[4.2.0]-octène-2 isomère syn.

Spectre de RMN du proton (250 MHz, DMSO $d_6$, δ en ppm, J en Hz) 1,47 et 1,49 [2S, 2 × 3H, $-C(CH_3)_2-$]; 3,92 et 4,05 (2d, J = 18, 2H, $-SCH_2-$); 5,33 (d, J = 5, 1H, H en 6); 5,98 (Mf, 3H, H en 7 et ⩾ $N^{\oplus}\underline{CH_2}C_6H_5$); 6,78 (S, 1H, H en 5 du thiazole); 7,4 et 7,65 (Mf, 5H aromatiques du benzyle $+NH_2$); 8,21 (S, 1H, H en 4 du thiazole); 8,25 (dd, J = 6 et 7,5, 1H, H en 5 du pyridinio); 9,04 (d, J = 7,5, 1H, H en 4 de la pyridine); 9,21 (d, J = 6, 1H, H en 6 du pyridinio); 9,58 (d, J = 8, 1H, $-CONH-$); 9,86 (S large, 1H, H en 2 du pyridinio).

*Exemple 7:*

Une solution de 0,95 g de benzhydryloxycarbonyl-2 [(t-butoxycarbonyl-2 propyl-2) oxyimino-2 (tritylamino-2 thiazolyl-4)-2 acétamido]-7 oxo-8 oxyde-5 [(pyridyl-4)-2 thiazolyl-5]-3 thia-5 aza-1 bicyclo-[4.2.0]-octène-2 isomère syn dans 12 $cm^3$ de N,N-diméthylformamide contenant 0,065 $cm^3$ d'iodure de méthyle est traitée selon le mode opératoire décrit dans l'exemple 2, pour donner 0,86 g d'iodure de benzhydryloxycarbonyl-2 [(t-butoxycarbonyl-2 propyl-2) oxyimino-2 (tritylamino-2 thiazolyl-4)-2 acétamido]-7 [(méthyl-1 pyridinio-4)-2 thiazolyl-5]-3 oxo-8 oxyde-5 thia-5 aza-1 bicyclo-[4.2.0]-octène-2 isomère syn sous la forme d'un solide jaune.

Spectre infrarouge (KBr, bandes caractéristiques en $cm^{-1}$) 3400, 1800, 1730, 1685, 1640, 1520, 1495, 1450, 1370, 1145, 845, 755, 705.

Spectre RMN du proton (250 MHz, DMSO $d_6$, δ en ppm, J en Hz) 1,37 [S, 9H, $-C(CH_3)_3$]; 1,46 [S, 6H, > $C(CH_3)_2$]; 3,89 et 4,5 (2D, J = 18,5, 2H, $-S-CH_2-$); 4,38 (S, 3H, ⩾ $\overset{+}{N}-CH_3$); 5,17 (D, J = 5, 1H, H en 6); 6,09 (DD, J = 8 et 5, 1H, H en 7); 6,80 (S, 1H, H en 5 du thiazole); 6,97 [S, 1H, $-COO-\underline{C}H(C_6H_5)_2$]; 6,95 à 7,4 (Mt, 25H, aromatiques); 8,19 (S, 1H, H en 4 du thiazole); 8,33 (D, J = 6, 2H, H en 3 et H en 5 du pyridinio); 8,44 (D, J = 8, 1H, $-CO-NH-$); 8,77 [S, 1H, $-NH-C(C_6H_5)_3$]; 9,05 (D, J = 6, 2H, H en 2 et H en 6 du pyridinio).

Une solution de 0,8 g d'iodure de benzhydryloxycarbonyl-2 [(t-butoxycarbonyl-2 propyl-2) oxyimino-2 (tritylamino-2 thiazolyl-4)-2 acétamido]-7 [(méthyl-1 pyridinio-4)-2 thiazolyl-5]-3 oxo-8 oxyde-5 thia-5 aza-1 bicyclo-[4.2.0]-octène-2 isomère syn dans un mélange de 20 $cm^3$ de dichlorométhane et de 5 $cm^3$ de N,N-diméthylacétamide est traitée par 0,1 $cm^3$ de trichlorure de phosphore selon le mode opératoire de l'exemple 2 pour donner 0,9 g de benzhydryloxycarbonyl-2 [(t-butoxycarbonyl-2 propyl-2) oxyimino-2 (tritylamino-2 thiazolyl-4)-2 acétamido]-7 [(méthyl-1 pyridinio-4)-2 thiazolyl-5]-3 oxo-8 thia-5 aza-1 bicyclo-[4.2.0]-octène-2 isomère syn brut sous la forme d'un solide jaune.

Spectre infrarouge (KBr, bandes caractéristiques en $cm^{-1}$) 1790, 1730, 1685, 1640, 1520, 1495, 1450, 1370, 1145, 760, 705.

Spectre RMN du proton (250 MHz, DMSO $d_6$,

δ en ppm, J en Hz) 1,38 [S, 9H, $-C(CH_3)_3$]; 1,43 et 1,44 [2S, 6H, > $C(CH_3)_2$]; 3,89 et 4,09 (2D, J = 18,5, 2H, $-S-CH_2-$); 4,38 (S, 3H, ⩾ $\overset{+}{N}-CH_3$); 5,33 (D, J = 5, 1H, H en 6); 5,94 (DD, J = 9 et 5, 1H, H en 7); 6,74 (S, 1H, H en 5 du thiazole); 6,94 [S, 1H, $-COO-CH(C_6H_5)_2$]; 6,90 à 7,4 (Mt, 25H, aromatiques); 8,2 (S, 1H, H en 4 du thiazole); 8,30 (D, J = 6, 2H, H en 3 et H en 5 du pyridinio); 9,05 [D, J = 6 + Mf, 3H, H en 2 et H en 6 du pyridinio + $-NH-C(C_6H_5)_3$]; 9,58 (D, J = 9, 1H, $-CO-NH-$).

Une solution de 0,86 g de benzhydryloxycarbonyl-2 [(t-butoxycarbonyl-2 propyl-2) oxyimino-2 (tritylamino-2 thiazolyl-4)-2 acétamido]-7 [(méthyl-1 pyridinio-4)-2 thiazolyl-5]-3 oxo-8 thia-5 aza-1 bicyclo-[4.2.0]-octène-2 isomère syn dans 30 $cm^3$ d'acide formique contenant 2 $cm^3$ d'anisole est traitée selon le mode opératoire décrit dans l'exemple 2 pour donner 0,21 g d'[(amino-2 thiazolyl-4)-2 (carboxy-2 propyl-2) oxyimino-2 acétamido]-7 carboxylato-2 [(méthyl-1 pyridinio-4)-2 thiazolyl-5]-3 oxo-8 thia-5 aza-1 bicyclo-[4.2.0]-octène-2 isomère syn à l'état d'iodhydrate brut que l'on retraite par de la résine IR 45 basique selon le mode opératoire de l'exemple 2 pour obtenir 0,053 g d'[(amino-2 thiazolyl-4)-2 (carboxy-2 propyl-2) oxyimino-2 acétamido]-7 carboxylato-2 [(méthyl-1 pyridinio-4)-2 thiazolyl-5]-3 oxo-8 thia-5 aza-1 bicyclo-[4.2.0]-octène-2 isomère syn sous la forme d'un lyophilisat jaune.

Spectre infrarouge (KBr, bandes caractéristiques en $cm^{-1}$) 3380, 1770, 1670, 1640, 1605, 1520, 1380, 1140, 840.

Spectre RMN du proton (250 MHz, DMSO $d_6$, δ en ppm, J en Hz) 1,4 à 1,5 [Mf, > $C(CH_3)_2$]; 3,83 (Mf, 2H, $-SCH_2-$); 4,31 (Mf, 3H, ⩾ $\overset{+}{N}-CH_3$); 5,20 (D, J = 5, 1H, H en 6); 5,74 (DD, J = 9 et 5, 1H, H en 7); 6,73 (S, 1H, H en 5 du thiazole); 7,29 (Mf, 2H, $-NH_2$); 8,23 (S, 1H, H en 4 du thiazole); 8,42 (Mf, 2H, H en 3 et H en 5 du pyridinio); 8,96 (Mf, 2H, H en 2 et H en 6 du pyridinio); 9,48 (D, J = 9, 1H, $-CONH-$).

Le benzhydryloxycarbonyl-2 [(t-butoxycarbonyl-2 propyl-2) oxyimino-2 (tritylamino-2 thiazolyl-4)-2 acétamido]-7 oxo-8 oxyde-5 [(pyridyl-4)-2 thiazolyl-5]-3 thia-5 aza-1 bicyclo-[4.2.0]-octène-2 isomère syn peut être préparé de la manière suivante.

On ajoute, à une solution refroidie à 0° C de 4,3 g d'acide (t-butoxycarbonyl-2 propyl-2) oxyimino-2 (tritylamino-2 thiazolyl-4)-2 acétique isomère syn, 2,7 g d'amino-7 benzhydryloxycarbonyl-2 oxo-8 oxyde-5 [(pyridyl-3)-2 thiazolyl-5]-3 thia-5 aza-1 bicyclo-[4.2.0]-octène-2 dans 50 $cm^3$ de N,N-diméthylformamide, 1,015 g de N-hydroxybenzothiazole et 1,54 g de N,N'-dicyclohexylcarbodiimide. Le mélange réactionnel est agité 1 h à 0° C puis 16 h à 25° C. Le précipité est éliminé par filtration et lavé 2 fois par 30 $cm^3$ d'acétate d'éthyle. Les filtrats réunis sont dilués par 1 l d'acétate d'éthyle et lavés par 500 $cm^3$ d'eau contenant 50 $cm^3$ de solution saturée de bicarbonate de sodium, puis 2 fois par 500 $cm^3$ d'eau distillée et par 200 $cm^3$ de solution saturée de chlorure de sodium. Après séchage sur sulfate de magnésium et évaporation à sec du solvant sous pression réduite (30 mm de mercure; 4 kPa) à 40° C, on soumet le résidu à une chromatographie sur une colonne (hauteur = 30 cm, diamètre = 5 cm) de gel de silice (0,04-0,06 mm) en éluant sous une pression de 0,5 bar par de l'acétate d'éthyle et en recueillant des fractions de 100 $cm^3$. Les fractions 7 à 12 sont réunies et concentrées sous pression réduite (30 mm de mercure; 4 kPa) à 30° C pour donner 2 g de produit attendu, impur, que l'on chromatographie à nouveau sur une colonne (hauteur = 30 cm, diamètre = 40 cm) de gel de silice (0,04-0,06 mm) en éluant sous une pression de 0,5 bar par un mélange de cyclohexane et d'acétate d'éthyle (10/90 en volumes) et en recueillant des fractions de 100 $cm^3$. Les fractions 5 à 16 sont réunies et concentrées à sec sous pression réduite (30 mm de mercure; 4 kPa) à 30° C pour donner 1,05 g de benzhydryloxycarbonyl-2 [(t-butoxycarbonyl-2 propyl-2) oxyimino-2 (tritylamino-2 thiazolyl-4)-2 acétamido]-7 oxo-8 oxyde-5 [(pyridyl-4)-2 thiazolyl-5]-3 thia-5 aza-1 bicyclo-[4.2.0]-octène-2 isomère syn sous la forme d'une meringue beige.

Spectre infrarouge (KBr, bandes caractéristiques en $cm^{-1}$) 3400, 1805, 1730, 1685, 1595, 1510, 1495, 1450, 1370, 1220, 1145, 820, 755, 700.

Spectre RMN du proton (250 MHz, $CDCl_3$, δ en ppm, J en Hz) 1,44 [S, 9H, $-C(CH_3)_3$]; 1,59 et 1,62 [2S, 6H, > $C(CH_3)_2$]; 3,37 et 3,96 (DD, J = 18,5 et 1 et D, J = 18,5, 2H, $-S-CH_2-$); 4,7 (DD, J = 5 et 1, 1H, H en 6); 6,31 (DD, J = 10 et 5, 1H, H en 7); 6,72 (S, 1H, H en 5 du thiazole); 6,96 [S, 1H, $-COO-CH(C_6H_5)_2$]; 6,95 à 7,4 (Mt, aromatiques); 7,59 (DD, J = 5 et 1, 2H, H en 3 et H en 5 de la pyridine); 7,63 (S, 1H, H en 4 du thiazole); 7,97 (D, J = 10, 1H, $-CONH-$); 8,71 (DD, J = 5 et 1, H en 2 et H en 6 de la pyridine).

L'amino-7 benzhydryloxycarbonyl-2 oxo-8 oxyde-5 [(pyridyl-4)-2 thiazolyl-5]-3 thia-5 aza-1 bicyclo-[4.2.0]-octène-2 peut être préparé de la manière suivante.

6,4 g de benzhydryloxycarbonyl-2 t-butoxycarbonylamino-7 oxo-8 oxyde-5 [(pyridyl-4)-2 thiazolyl-5]-3 thia-5 aza-1 bicyclo-[4.2.0]-octène-2 sont traités par 6 $cm^3$ d'acide méthanesulfonique dans 60 $cm^3$ d'acétonitrile, selon le mode opératoire de l'exemple 2 pour donner 2,7 g d'amino-7 benzhydryloxycarbonyl-2 oxo-8 oxyde-5 [(pyridyl-4)-2 thiazolyl-5]-3 thia-5 aza-1 bicyclo-[4.2.0]-octène-2 sous la forme d'une meringue brune.

Rf = 0,14 (chromatoplaque de gel de silice, éluant: acétate d'éthyle/méthanol 85/15 en volumes).

Le benzhydryloxycarbonyl-2 t-butoxycarbonylamino-7 oxo-8 oxyde-5 [(pyridyl-4)-2 thiazolyl-5]-3 thia-5 aza-1 bicyclo-[4.2.0]-octène-2 peut être préparé de la manière suivante.

Une solution de 24 g de benzhydryloxycarbonyl-2 t-butoxycarbonylamino-7 oxo-8 [(pyridyl-4)-2 thiazolyl-5]-3 thia-5 aza-1 bicyclo-[4.2.0]-octène-2 dans 370 $cm^3$ de dichlorométhane est oxydée par 7,92 g d'acide métachloroperbenzoïque à 85% selon le mode opératoire décrit dans l'exemple 2 pour donner 23 g de benzhydryloxycarbonyl-2 t-butoxycarbonylamino-7 oxo-8 oxyde-5 [(pyridyl-4)-2 thiazolyl-5]-3 thia-5 aza-1 bicyclo-[4.2.0]-octène-2 sous la forme d'un solide jaune-brun.

Spectre infrarouge ($CHBr_3$, bandes caractéristiques en $cm^{-1}$) 3410, 1800, 1715, 1595, 1505, 1370, 1240, 1050, 820, 745.

Spectre RMN du proton (250 MHz, $CDCl_3$, δ en ppm, J en Hz) 1,5 [S, 9H, $-C(CH_3)_3$]; 3,38 et 4,04 (DD, J = 18,5 et 1 et D, J = 18,5, 2H, $-S-CH_2-$); 4,61 (DD, J = 5 et 1, 1H, H en 6); 5,8 (D, J = 10, 1H, $-CONH-$); 5,95 (DD, J 10 et 5, 1H, H en 7); 6,94 [S, 1H, $-COO-\underline{CH}(C_6H_5)_2$]; 6,95 à 7,4 (Mt, aromatiques); 7,60 (DD, J = 5 et 1, 2H, H en 3 et H en 5 de la pyridine); 7,65 (S, 1H, H en 4 du thiazole); 8,70 (DD, J = 5 et 1, 2H, H en 2 et H en 6 de la pyridine).

Le benzhydryloxycarbonyl-2 t-butoxycarbonylamino-7 oxo-8 [(pyridyl-4)-2 thiazolyl-5]-3 thia-5 aza-1 bicyclo-[4.2.0]-octène-2 peut être préparé de la manière suivante.

On ajoute en 1 h 40 min une solution de 94 g de benzhydryloxycarbonyl-2 (bromo-1 oxo-2 éthyl)-3 t-butoxycarbonylamino-7 oxo-8 thia-5 aza-1 bicyclo-[4.2.0]-octène-2 (mélange des diastéréoisomères) dans 250 $cm^3$ de tétrahydrofuranne à une solution à 40° C de 44 g de thioisonicotinamide dans 330 $cm^3$ de N,N-diméthylacétamide. Le mélange réactionnel est agité 90 min à 40-45° C puis refroidi à −15° C et traité par 18,5 $cm^3$ de chlorure de méthanesulfonyle puis 53 $cm^3$ de triéthylamine. On agite 1 h vers −10° C puis 16 h à 25° C, puis dilue par 1,5 l d'acétate d'éthyle et 1 l d'eau distillée. La phase organique est lavée successivement par 1,2 l d'acide chlorhydrique 0,2N, 2 fois par 1 l d'eau puis par un mélange de 500 $cm^3$ d'eau et 150 $cm^3$ de solution saturée de bicarbonate de sodium, et enfin par 2 fois 1 l d'eau et par 200 $cm^3$ de solution saturée de chlorure de sodium. Le résidu obtenu après évaporation à sec du solvant sous pression réduite (30 mm de mercure; 4 kPa) à 40° C est chromatographié sur une colonne (hauteur = 56 cm, diamètre = 80 cm) de gel de silice (0,2-0,6 mm) en éluant par un mélange de cyclohexane et d'acétate d'éthyle (50/50 en volumes) et en recueillant des fractions de 800 $cm^3$. Les fractions 8 à 12 sont réunies et concentrées à sec sous pression réduite (30 mm de mercure; 4 kPa) à 30° C pour donner 24 g de benzhydryloxycarbonyl-2 t-butoxycarbonylamino-7 oxo-8 [(pyridyl-4)-2 thiazolyl-5]-3 thia-5 aza-1 bicyclo-[4.2.0]-octène-2 sous la forme d'une meringue brune.

Rf = 0,47 (chromatoplaque de gel de silice, éluant acétate d'éthyle).

*Exemple 8:*

Une solution de 1,6 g de benzhydryloxycarbonyl-2 [méthoxyimino-2 (tritylamino-2 thiazolyl-4)-2 acétamido]-7 oxo-8 oxyde-5 [(pyridyl-4)-2 thiazolyl-5]-3 thia-5 aza-1 bicyclo-[4.2.0]-octène-2 isomère syn et de 0,12 $cm^3$ d'iodure de méthyle dans 16 $cm^3$ de N,N-diméthylformamide est agitée 70 h à 20° C, puis coulée sur 250 $cm^3$ d'acétate d'éthyle. Le précipité est essoré et lavé 2 fois par 20 $cm^3$ d'acétate d'éthyle et 3 fois par 20 $cm^3$ d'éther éthylique, puis séché pour donner 1,4 g d'iodure de benzhydryloxycarbonyl-2 [méthoxyimino-2 (tritylamino-2 thiazolyl-4)-2 acétamido]-7 [(méthyl-1 pyridinio-4)-2 thiazolyl-5]-3 oxo-8 oxyde-5 thia-5 aza-1 bicyclo-[4.2.0]-octène-2 isomère syn sous la forme d'une poudre jaune.

Spectre infrarouge (KBr, bandes caractéristiques en $cm^{-1}$) 1800, 1730, 1670, 1640, 1525, 1500, 1450, 1220, 1040, 760, 705.

Spectre RMN du proton (250 MHz, DMSO $d_6$, δ en ppm, J en Hz) 3,85 (S, 3H, $=NOCH_3$); 3,94 et 4,45 (2D, J = 15, $-S-CH_2-$); 4,38 (S, $\geq \overset{+}{N}-CH_3$); 5,14 (D, J = 4,5, 1H, H en 6); 5,99 (DD, J = 7 et 4,5, 1H, H en 7); 6,83 (S, 1H, H en 5 du thiazole); 6,96 [S, 1H, $-COO-\underline{CH}(C_6H_5)_2$]; 7 à 7,5 (Mt, aromatiques); 8,15 (S, 1H, H en 4 du thiazole); 8,33 (D, J = 6, 2H, H en 3 et H en 5 du pyridinio); 8,83 [S, 1H, $-NH-C(C_6H_5)_3$]; 9,05 (D, J = 6, 2H, H en 2 et H en 6 du pyridinio); 9,3 (D, J = 7, 1H, $-CO-NH-$).

Une solution de 1,3 g d'iodure de benzhydryloxycarbonyl-2 [méthoxyimino-2 (tritylamino-2 thiazolyl-4)-2 acétamido]-7 [(méthyl-1 pyridinio-4)-2 thiazolyl-5]-3 oxo-8 oxyde-5 thia-5 aza-1 bicyclo-[4.2.0]-octène-2 isomère syn dans un mélange de 40 $cm^3$ de dichlorométhane et de 10 $cm^3$ de N,N-diméthylacétamide, refroidie à −5° C, est traitée par 0,17 $cm^3$ de trichlorure de phosphore puis agitée à −5° C pendant 40 min et versée sur 200 $cm^3$ d'acétate d'éthyle. Le précipité est essoré, lavé 3 fois par 10 $cm^3$ d'acétate d'éthyle et 3 fois par 20 $cm^3$ d'éther éthylique puis repris dans 10 $cm^3$ de dichlorométhane et 2 $cm^3$ de méthanol. La solution est filtrée sur un filtre fritté garni de 5 $cm^3$ de gel de silice (0,06-0,2 mm) puis concentrée à sec sous pression réduite (30 mm de mercure; 4 kPa) à 30° C pour donner 1,14 g d'iodure de benzhydryloxycarbonyl-2 [méthoxyimino-2 (tritylamino-2 thiazolyl-4)-2 acétamido]-7 [(méthyl-1 pyridinio-4)-2 thiazolyl-5]-3 oxo-8 thia-5 aza-1 bicyclo-[4.2.0]-octène-2 isomère syn brut sous la forme d'un solide jaune.

Spectre RMN du proton (250 MHz, DMSO $d_6$, δ en ppm, J en Hz) 3,87 (S, 3H, $=N-OCH_3$); 3,96 (D, J = 19, 1H du $-S-CH_2-$), de 4 à 4,20 (l'autre H du $-S-CH_2-$); 4,38 (S, 3H, $\geq \overset{+}{N}-CH_3$); 5,32 (D, J = 5, 1H, H en 6); 5,92 (DD, J = 8 et 5, 1H, H en 7; 6,78 (S, 1H, H en 5 du thiazole); 6,92 [S, 1H, $-COO-\underline{CH}(C_6H_5)_2$]; 6,95 à 7,45 (Mt, aromatiques); 8,15 (S, 1H, H en 4 du thiazole); 8,30 (D, J = 6, 2H, H en 3 et H en 5 du pyridinio); 9,05 (D, J = 6, 2H, H en 2 et H en 6 du pyridinio); 9,25 [Mf, 1H, $-\underline{NH}-C(C_6H_5)_3$]; 9,76 (D, J = 8, 1H, $-CO-NH-$).

Une solution de 6,2 g d'iodure de benzhydryloxycarbonyl-2 [méthoxyimino-2 (tritylamino-2 thiazolyl-4)-2 acétamido]-7 [(méthyl-1 pyridinio-4)-2 thiazolyl-5]-3 oxo-8 thia-5 aza-1 bicyclo-[4.2.0]-octène-2 isomère syn dans un mélange de 25 $cm^3$ d'anisole et de 30 $cm^3$ d'acide formique est chauffée 30 min à 50° C puis concentrée à sec sous pression réduite (1 mm de mercure; 0,13 kPa) à 40° C. Le résidu est repris par 100 $cm^3$ d'éthanol que l'on évapore à sec sous pression réduite (30 mm de mercure; 4 kPa) à 40° C, cette opération étant répétée encore 2 fois. Le résidu est concrété par 150 $cm^3$ d'éthanol. Le solide est essoré, lavé 2 fois par 10 $cm^3$ d'éthanol, 3 fois par 20 $cm^3$ d'éther éthylique et séché. On obtient 3,72 g d'iodhydrate d'[(amino-2 thiazolyl-4)-2 méthoxyimino-2 acétamido]-7 carboxylato-2 [(méthyl-1 pyridinio-4)-2 thiazolyl-5]-3 oxo-8 thia-5 aza-1 bicyclo-[4.2.0]-octène-2 isomère syn brut sous la forme d'un solide brun clair que l'on reprend par 400 $cm^3$ d'eau distillée; après filtration, la phase aqueuse est lavée 2 fois par 50 $cm^3$ d'acétate d'éthyle et traitée par de la résine IR 45 basique jusqu'à atteindre un pH de 4,8. La résine est éliminée par filtration et la solution aqueuse est lyophilisée pour donner 1,22 g d'[(amino-2 thiazolyl-4)-2 méthoxyimino-2 acétamido]-7 carboxylato-2 [(méthyl-1 pyridinio-4)-2 thiazolyl-5]-3 oxo-8 thia-5 aza-1 bicyclo-[4.2.0]-octène-2 isomère syn sous la forme d'un lyophilisat orangé.

Spectre infrarouge (KBr, bandes caractéristiques en $cm^{-1}$) 3390, 1770, 1670, 1640, 1610, 1525, 1380, 1035.

Spectre RMN du proton (250 MHz, DMSO $d_6$, δ en ppm, J en Hz) 3,78 et 3,88 (2D, J = 18, 2H, $-S-CH_2-$); 3,86 (S, 3H, $=N-O-CH_3$); 4,30 (S large, 3H, $\geqslant \overset{+}{N}-CH_3$); 5,19 (D, J = 5, 1H, H en 6); 5,69 (DD, J = 8 et 5, 1H, H en 7); 6,76 (S, 1H, H en 5 du thiazole); 7,25 (S large, 2H, $-NH_2$); 8,23 (S, 1H, H en 4 du thiazole); 8,41 (D, J = 5,5, 2H, H en 3 et H en 5 du pyridinio); 8,95 (D, J = 5,5, 2H, H en 2 et H en 6 du pyridinio); 9,65 (D, J = 8, 1H, $-CO-NH-$); 14 à 10 (Mf très étalé, 1H, $-COOH$).

Le benzhydryloxycarbonyl-2 [méthoxyimino-2 (tritylamino-2 thiazolyl-4)-2 acétamido]-7 oxo-8 oxyde-5 [(pyridyl-4)-2 thiazolyl-5]-3 thia-5 aza-1 bicyclo-[4.2.0]-octène-2 peut être préparé de la manière suivante.

A une solution de 15 g d'amino-7 benzhydryloxycarbonyl-2 oxo-8 oxyde-5 [(pyridyl-4)-2 thiazolyl-5]-3 thia-5 aza-1 bicyclo-[4.2.0]-octène-2 dans 100 $cm^3$ de dichlorométhane sec on ajoute 12,4 g d'acide méthoxyimino-2 (tritylamino-2 thiazolyl-4)-2 acétique, isomère syn, 0,05 g de N,N-diméthylamino-4 pyridine puis, après refroidissement à +5° C, une solution de 6,93 g de N,N-dicyclohexylcarbodiimide dans 120 $cm^3$ de dichlorométhane (en 30 min). Le mélange réactionnel est ensuite agité 1 h à +5° C puis 4 h à 25° C puis partiellement concentré, redilué par 500 $cm^3$ d'acétate d'éthyle et filtré. Le filtrat est versé dans un mélange de 1 l d'eau distillée, 1 l d'acétate d'éthyle et 10 $cm^3$ d'acide acétique. La phase organique est lavée par 200 $cm^3$ de solution saturée de bicarbonate de sodium, 3 fois par 200 $cm^3$ d'eau distillée puis 200 $cm^3$ de solution saturée de chlorure de sodium et séchée sur sulfate de sodium puis concentrée à sec sous pression réduite (30 mm de mercure; 4 kPa) à 40° C pour donner 26,8 g de produit attendu brut sous la forme d'une meringue brune que l'on peut purifier de la façon suivante: on redissout 5 g dans 120 $cm^3$ d'éthanol au reflux, ajoute 0,5 g de noir décolorant et 0,5 g de silice, agite, filtre et refroidit dans un bain d'eau glacée. Le précipité est essoré, lavé par 2 fois 10 $cm^3$ d'éthanol puis 2 fois par 20 $cm^3$ d'éther pour donner 2 g de benzhydryloxycarbonyl-2 [méthoxyimino-2 (tritylamino-2 thiazolyl-4)-2 acétamido]-7 oxo-8 oxyde-5 [(pyridyl-4)-2 thiazolyl-5]-3 thia-5 aza-1 bicyclo-[4.2.0]-octène-2 sous la forme d'une poudre ocre.

Spectre infrarouge (KBr, bandes caractéristiques en $cm^{-1}$) 3390, 1805, 1690, 1605, 1515, 1500, 1450, 1220, 1050, 1040, 830, 760, 700.

Spectre RMN du proton (250 MHz, DMSO $d_6$, δ en ppm, J en Hz) 3,8 à 3,90 (Mf, 1H du $-S-CH_2-$); 3,85 (S, $=N-OCH_3$); 4,41 (D, J = 18,5, 1H du $-S-CH_2-$); 5,1 (D, J = 4,5 H en 6); 5,95 (Mt, H en 7); 6,83 (S, H en 5 du thiazole); 6,96 [S, $-COO-\underline{CH}(C_6H_5)_2$]; 7,60 à 7,7 (Mt, H en 3 et H en 5 de la pyridine); 7,96 (S, H en 4 du thiazole); 8,72 (Mt, H en 2 et H en 6 de la pyridine); 8,82 [Mf, $-N\underline{H}-C(C_6H_5)_3$]; 9,25 (D, J = 8, $-CONH-$).

*Exemple 9:*

On agite à 40° C pendant 2 h une solution de 3,47 g de benzhydryloxycarbonyl-2 [méthoxyimino-2 (tritylamino-2 thiazolyl-4)-2 acétamido]-7 oxo-8 oxyde-5 [(pyridyl-3 méthyl)-2 thiazolyl-5]-3 thia-5 aza-1 bicyclo-[4.2.0]-octène-2, isomère syn, 0,435 $cm^3$ d'iodure de méthyle dans 20 $cm^3$ de N,N-diméthylformamide. Le mélange est versé dans 200 $cm^3$ d'oxyde d'éthyle puis agité vivement pendant 15 min. Le précipité obtenu est filtré, lavé par 2 fois 20 $cm^3$ d'oxyde d'éthyle puis séché sous pression réduite (0,1 mm de mercure; 0,013 kPa). On obtient 3,9 g d'iodure de benzhydryloxycarbonyl-2 [méthoxyimino-2 (tritylamino-2 thiazolyl-4)-2 acétamido]-7 oxo-8 oxyde-5 [(méthyl-1 pyridinio-3 méthyl)-2 thiazolyl-5]-3 thia-5 aza-1 bicyclo-[4.2.0]-octène-2, isomère syn sous la forme d'une poudre crème.

Spectre infrarouge (KBr, bandes caractéristiques en $cm^{-1}$) 1795, 1730, 1665, 1570, 1450, 1220, 1040, 755, 700, 675.

Spectre RMN du proton (350 MHz, DMSO $d_6$, δ en ppm, J en Hz) 3,80 et 4,25 (2D, J = 18, 2H, $-S-CH_2-$); 3,85 (S, 3H, $=N-OCH_3$); 4,35 (S, 3H, $\geqslant \overset{+}{N}-CH_3$); 4,47 (S, 2H, $-CH_2-$); 5,09 (D, J = 4, 1H, H en 6); 5,92 (DD, J = 7,5 et 4, 1H, H en 7); 6,82 (S, 1H, H en 5 du thiazole); 6,91 [S, 1H, $-COO-\underline{CH}(C_6H_5)_2$]; 7,15 à 7,45 (Mt, aromatiques); 7,62 (S, 1H, H en 4 du thiazole); 8,12 (DD, J = 8 et 6, 1H, H en 5 du pyridinio); 8,48 (D, J = 8, 1H, H en 4 du pyridinio); 8,8 [S, 1H, $-N\underline{H}-C(C_6H_5)_3$]; 8,93 (D, J = 6, 1H, H en 6 du pyridinio); 9,03 (S, 1H, H en 2 du pyridinio); 9,17 (D, J = 7,5, 1H, $-CO-NH-$).

A une solution refroidie à −2° C de 3,9 g d'iodure de benzhydryloxycarbonyl-2 [méthoxyimino-2 (tritylamino-2 thiazolyl-4)-2 acétamido]-7 oxo-8 oxyde-5 [(méthyl-1 pyridinio-3 méthyl)-2 thiazolyl-5]-3 thia-5 aza-1 bicyclo-[4.2.0]-octène-2, isomère syn et de 1,39 $cm^3$ de N,N-diméthylacétamide dans 35 $cm^3$ de dichlorométhane, on ajoute goutte à goutte 0,612 $cm^3$ de trichlorure de phosphore en 5 min. On agite le mélange à 0° C pendant 25 min puis le mélange réactionnel est dilué par 200 $cm^3$ d'acétate d'éthyle. On agite pendant 15 min, filtre le précipité obtenu et lave par 4 fois 50 $cm^3$ d'acétate d'éthyle puis par 2 fois 50 $cm^3$ d'oxyde d'éthyle. La poudre est séchée sous pression réduite (0,1 mm de mercure, 0,013 kPa) et on obtient 3,8 g d'iodure de benzhydryloxycarbonyl-2 [méthoxyimino-2 (tritylamino-2 thiazolyl-4)-2 acétamido]-7 oxo-8 [(méthyl-8 pyridionio-3 méthyl)-2 thiazolyl-5]-3 thia-5 aza-1 bicyclo-[4.2.0]-octène-2, isomère syn, sous la forme d'un solide jaune.

Spectre infrarouge (KBr, bandes caractéristiques en $cm^{-1}$) 1785, 1730, 1680, 1510, 1450, 1220, 1040, 755, 700.

A une solution refroidie à +4° C de 3,8 g d'iodure de benzhydryloxycarbonyl-2 [méthoxyimino-2 (tritylamino-2 thiazolyl-4)-2 acétamido]-7 oxo-8 [(méthyl-1 pyridinio-3 méthyl)-2 thiazolyl-5]-3 thia-5 aza-1 bicyclo-[4.2.0]-octène-2, isomère syn dans 3,8 $cm^3$ d'anisole, on coule un mélange de 17 $cm^3$ d'acide trifluoracétique et 1,7 $cm^3$ d'eau distillée. On coule alors 3,8 $cm^3$ d'eau distillée au mélange agité maintenu à 4° C. On enlève le bain réfrigérant, et on agite pendant 30 min à 20° C. On ajoute alors 400 $cm^3$ d'oxyde d'éthyle et on décante la phase liquide. Le résidu huileux est repris dans 200 $cm^3$ d'acétone; un solide précipite. On filtre et on lave le précipité par 3 fois 20 $cm^3$ d'acétone. On obtient 2,11 g d'iodure d'[amino-2 thiazolyl-4)-2 méthoxyimino-2 acétamido]-7 carboxy-2 [(méthyl-1 pyridinio-3 méthyl)-2 thiazolyl-5]-3 oxo-8 thia-5 aza-1 bicyclo-[4.2.0]-octène-2, isomère syn, sous la forme d'une poudre jaune.

Spectre infrarouge (KBr, bandes caractéristiques en $cm^{-1}$) 3490, 3050, 1780, 1670, 1630, 1365, 1200, 1180, 1045, 985, 680, 600.

Spectre RMN du proton (250 MHz, DMSO $d_6$, δ en ppm, J en Hz) 3,88 (S, $-S-CH_2-$); 3,96 (S, $=N-OCH_3$); 4,40 (S, $\geqslant \overset{+}{N}-CH_3$); 4,65 (S, $-CH_2-$); 5,29 (D, J = 5, 1H, H en 6); 5,87 (DD, J = 9 et 5, 1H, H en 7); 6,95 (S, 1H, H en 5 du thiazole); 7,8 (S, 1H, H en 4 du thiazole); 6,90 à 7,70 (Mf, $-NH_2$); 8,16 (Mt, H en 5 du pyridinio); 8,59 (D, J = 7,5, 1H, H en 4 du pyridinio); 9,01 (Mf, 1H, H en 6 du pyridinio); 9,18 (S large, 1H, H en 2 du pyridinio); 9,92 (D, J = 9, 1H, −CONH−).

On lave une solution de 1,35 g d'iodure d'[(amino-2 thiazolyl-4)-2 méthoxyimino-2 acétamido]-7 carboxy-2 [(méthyl-1 pyridinio-3 méthyl)-2 thiazolyl-5]-3 oxo-8 thia-5 aza-1 bicyclo-[4.2.0]-octène-2, isomère syn, dans 60 $cm^3$ d'eau distillée par 2 fois 10 $cm^3$ d'acétate d'éthyle. On décante la phase aqueuse et on la concentre jusqu'à un volume résiduel de 50 $cm^3$ sous pression réduite (30 mm de mercure; 4 kPa). On filtre et on ajoute 28 $cm^3$ de résine Amberlite IR 45 basique à la solution. On suit l'évolution de l'acidité du mélange au pH-mètre. On filtre la résine à pH 4,4 et on lyophilise le filtrat. On obtient 0,65 g d'[(amino-2 thiazolyl-4)-2 méthoxyimino-2 acétamido]-7 carboxylato-2 [(méthyl-1 pyridinio-3 méthyl)-2 thiazolyl-5]-3 oxo-8 thia-5 aza-1 bicyclo-[4.2.0]-octène-2, isomère syn, sous la forme d'un lyophilisat jaune-brun.

Spectre infrarouge (KBr, bandes caractéristiques en $cm^{-1}$) 3500, 3000, 1770, 1670, 1610, 1385, 1335, 1040, 680.

Spectre RMN du proton (250 MHz, DMSO $d_6$, δ en ppm, J en Hz) 3,63 et 3,75 (2D, J = 17,5, $-S-CH_2-$); 3,86 (S, $=N-OCH_3$); 4,38 (Mf, 3H, $\geqslant \overset{+}{N}-CH_3$); 4,55 (Mf, 2H, $-CH_2-$); 5,11 (D, J = 4, 1H, H en 6); 5,63 (DD, J = 8 et 4, 1H, H en 7); 6,75 (S, 1H, H en 5 du thiazole); 7,25 (S large, $-NH_2$); 7,74 (S, 1H, H en 4 du thiazole); 8,1 (Mf, 1H, H en 5 du pyridinio); 8,55 (Mf, 1H, H en 4 du pyridinio); 8,95 (Mf, 1H, H en 6 du pyridinio); 9,14 (Mf, 1H, H en 2 du pyridinio); 9,59 (D, J = 8, 1H, −CONH−).

Le benzhydryloxycarbonyl-2 [méthoxyimino-2 (tritylamino-2 thiazolyl-4)-2 acétamido]-7 oxo-8 oxyde-5 [(pyridyl-3 méthyl)-2 thiazolyl-5]-3 thia-5 aza-1 bicyclo-[4.2.0]-octène-2, isomère syn peut être préparé de la manière suivante.

A une solution refroidie à 3° C de 8,30 g de benzhydryloxycarbonyl-2 [méthoxyimino-2 (tritylamino-2 thiazolyl-4)-2 acétamido]-7 oxo-8 [(pyridyl-3 méthyl)-2 thiazolyl-5]-3 thia-5 aza-1 bicyclo-[4.2.0]-octène-2, isomère syn dans 85 $cm^3$ de dichlorométhane, on ajoute goutte à goutte pendant 15 min une solution de 1,46 g d'acide m-chloroperbenzoïque dans 30 $cm^3$ de dichlorométhane. Le mélange est agité à 3° C pendant 4½ h, puis dilué par 100 $cm^3$ de dichlorométhane. La phase organique est lavée successivement par 150 $cm^3$ d'une solution demi-saturée de bicarbonate de sodium, par 100 $cm^3$ d'eau distillée puis 100 $cm^3$ d'une solution saturée de chlorure de sodium. Elle est alors séchée sur sulfate de sodium anhydre; le mélange est filtré et le filtrat est concentré à sec sous pression réduite (30 mm de mercure; 4 kPa) à 30° C. Le résidu est purifié par chromatographie sur une colonne (hauteur = 30 cm, diamètre = 2,5 cm) de gel de silice (0,05-0,2 mm) en éluant par 1,2 l d'acétate d'éthyle puis par 0,8 l d'un mélange acétate d'éthyle/méthanol (95/5) en volumes et en recueillant des fractions de 60 $cm^3$. Les fractions 19 à 33 contenant le produit pur sont réunies et concentrées sous pression réduite (30 mm de mercure; 4 kPa) à 30° C pour donner 5 g de benzhydryloxycarbonyl-2 [méthoxyimino-2 (tritylamino-2 thiazolyl-4)-2 acétamido]-7 oxo-8 oxyde-5 [(pyridyl-3 méthyl)-2 thiazolyl-5]-3 thia-5 aza-1 bicyclo-[4.2.0]-octène-2, isomère syn sous la forme d'une meringue jaune.

Spectre infrarouge ($CHCl_3$, bandes caractéristi-

ques en $cm^{-1}$) 3400, 1805, 1730, 1685, 1515, 1450, 1375, 1050.

Spectre RMN du proton (250 MHz, $CDCl_3$, δ en ppm, J en Hz) 3,27 et 3,85 (2D, J = 18, 2H, $-S-CH_2-$); 4 à 4,2 (Mt, 5H, $=N-O-CH_3$ et $-CH_2-$); 4,57 (D, J = 5, 1H, H en 6); 6,18 (DD, J = 10 et 5, 1H, H en 7); 6,71 (S, 1H, H en 5 du thiazole); 6,88 [S, 1H, $-COO-C\underline{H}(C_6H_5)_2$]; 7,15 [Mf, $-N\underline{H}-C(C_6H_5)_3$]; 7,25 (Mt, H en 5 de la pyridine); 7,05 à 7,35 (Mt, aromatiques); 7,4 (S, 1H, H en 4 du thiazole); 7,52 (DDD, J = 7,5-2 et 1,5, 1H, H en 4 de la pyridine); 7,62 (D, J = 10, $-CONH-$); 8,48 (S large, 1H, H en 2 de la pyridine); 8,50 (DD, J = 5 et 1,5, 1H, H en 6 de la pyridine).

Le benzhydryloxycarbonyl-2 [méthoxyimino-2 (tritylamino-2 thiazolyl-4)-2 acétamido]-7 oxo-8 [(pyridyl-3 méthyl)-2 thiazolyl-5]-3 thia-5 aza-1 bicyclo-[4.2.0]-octène-2, isomère syn peut être préparé de la manière suivante.

A une solution refroidie à +5° C de 5,4 g d'amino-7 benzhydryloxycarbonyl-2 oxo-8 [(pyridyl-3 méthyl)-2 thiazolyl-5]-3 thia-5 aza-1 bicyclo-[4.2.0]-octène-2 dans 100 $cm^3$ de N,N'-diméthylformamide, on ajoute 5,32 g d'acide méthoxyimino-2 (tritylamino-2 thiazolyl-4)-2 acétique, isomère syn, 1,62 g d'hydroxy-3 benzotriazole et 2,47 g de N,N-dicyclohexylcarbodiimide. On agite pendant 1 h à 5° C, puis on laisse la température remonter et on agite 4 h à 23° C. On ajoute 0,5 $cm^3$ d'acide acétique, agite pendant 5 min et on filtre la suspension. Le filtrat est versé dans 500 $cm^3$ d'un mélange eau/acétate d'éthyle 50/50 (en volumes). La phase organique est décantée, puis lavée successivement par 100 $cm^3$ d'une solution 0,1N d'acide chlorhydrique, 100 $cm^3$ d'une solution saturée de bicarbonate de sodium, 100 $cm^3$ d'eau distillée et 100 $cm^3$ d'une solution saturée de chlorure de sodium. Elle est séchée sur sulfate de sodium anhydre, filtrée et le filtrat est concentré à sec sous pression réduite (20 mm de mercure; 2,7 kPa) à 30° C. Le résidu est purifié par chromatographie sur gel de silice (0,05-0,2 mm) (hauteur de silice: 77 cm, diamètre de la colonne: 3,5 cm) en éluant par 1 l de mélange cyclohexane/acétate d'éthyle 20/80 (en volumes) puis 3,8 l d'un mélange cyclohexane/acétate d'éthyle 10/90 (en volumes) et en recueillant des fractions de 60 $cm^3$. Les fractions 39 à 80 sont réunies et concentrées à sec sous pression réduite (20 mm de mercure; 2,7 kPa). On obtient 5,43 g de benzhydryloxycarbonyl-2 [méthoxyimino-2 (tritylamino-2 thiazolyl-4)-2 acétamido]-7 oxo-8 [(pyridyl-3 méthyl)-2 thiazolyl-5]-3 thia-5 aza-1 bicyclo-[4.2.0]-octène-2, isomère syn, sous la forme d'une meringue jaune.

Spectre infrarouge ($CHBr_3$, bandes caractéristiques en $cm^{-1}$) 3390, 1785, 1725, 1680, 1510, 1445, 1220, 1040, 750.

Spectre RMN du proton (250 MHz, $CDCl_3$, δ en ppm, J en Hz) 3,45 et 3,65 (2D, J ≃ 18, 2H, $-S-CH_2-$); 4,05 (AB limite, 2H, $-CH_2-$); 4,11 (S, 3H, $=N-O-CH_3$); 5,11 (D, J ≃ 5, 1H, H en 6); 6 (DD, J ≃ 9 et 5, 1H, H en 7); 6,72 (S, 1H, H en 5 du thiazole); 6,90 [S, 1H, $-COO-C\underline{H}(C_6H_5)_2$]; 7 à 7,35 (Mt, aromatiques, H en 5 de la pyridine, $-CONH-$, $-NH-$); 7,38 (S, 1H, H en 4 du thiazole); 7,52 (D large, J ≃ 8, H en 4 de la pyridine); 8,50 (Mt, 1H, H en 2 de la pyridine); 8,51 (DD, J ≃ 5 et 1, 1H, H en 6 de la pyridine).

L'amino-7 benzhydryloxycarbonyl-2 oxo-8 [(pyridyl-3 méthyl)-2 thiazolyl-5]-3 thia-5 aza-1 bicyclo-[4.2.0]-octène-2 peut être préparé de la manière suivante.

A une solution de 10 g de benzhydryloxycarbonyl-2 t-butoxycarbonylamino-7 oxo-8 [(pyridyl-3 méthyl)-2 thiazolyl-5]-3 thia-5 aza-1 bicyclo-[4.2.0]-octène-2 dans 100 $cm^3$ d'acétonitrile, on ajoute 10 $cm^3$ d'une solution d'acide méthanesulfonique. On agite le mélange pendant 5 min et on le verse dans 500 $cm^3$ d'un mélange 50/50 (en volumes) d'une solution saturée de bicarbonate de sodium et d'acétate d'éthyle. La phase organique est décantée, puis lavée successivement par 200 $cm^3$ d'eau distillée, puis 200 $cm^3$ d'une solution saturée de chlorure de sodium. Elle est séchée sur sulfate de magnésium anhydre, filtrée et le filtrat est concentré à sec sous pression réduite (30 mm der mercure; 4 kPa) à 30° C. On obtient 6,36 g d'amino-7 benzhydryloxycarbonyl-2 oxo-8 [(pyridyl-3 méthyl)-2 thiazolyl-5]-3 thia-5 aza-1 bicyclo-[4.2.0]-octène-2 sous la forme d'une meringue marron.

Spectre infrarouge ($CHBr_3$, bandes caractéristiques en $cm^{-1}$) 3400, 3340, 1780, 1725, 1620, 1495, 1480, 1450, 1425, 1220, 760.

Spectre de RMN du proton (250 MHz, DMSO $d_6$, δ en ppm, J en Hz) 3,72 et 3,83 (2d, J = 18, 2H, $-S-CH_2-$); 4,13 et 4,23 (2d, J = 16, 2H, $-CH_2-$); 4,89 (d, J = 5, 1H, H en 7); 5,08 (d, J = 5, 1H, H en 6); 6,8 [s, 1H, $-COO-C\underline{H}(C_6H_5)_2$]; 7,05 à 7,4 (mt, aromatiques, $-NH_2$ et H en 5 de la pyridine); 7,53 (s, 1H, H du thiazole); 7,64 (ddd, J = 8-2,5 et 1, 1H, H en 4 de la pyridine); 8,47 (dd, J = 5 et 1, 1H, H en 6 de la pyridine); 8,49 (d, J = 2, 1H, H en 2 de la pyridine).

Le benzhydryloxycarbonyl-2 t-butoxycarbonylamino-7 oxo-8 [(pyridyl-3 méthyl)-2 thiazolyl-5]-3 thia-5 aza-1 bicyclo-[4.2.0]-octène-2 peut être préparé de la manière suivante.

On ajoute une solution de 88,1 g de benzhydryloxycarbonyl-2 (bromo-1 oxo-2 éthyl)-3 t-butoxycarbonylamino-7 oxo-8 thia-5 aza-1 bicyclo-[4.2.0]-octène-2 (mélange des bromaldéhydes épimères) dans 150 $cm^3$ de tétrahydrofuranne anhydre à une solution de 25 g de pyridyl-3 thioformamide dans 200 $cm^3$ de N,N'-diméthylacétamide. On agite le mélange pendant 6 h à 20° C, puis on le coule dans 2 l d'un mélange eau/acétate d'éthyle 50/50 (en volumes). La phase organique est décantée puis lavée successivement par 500 $cm^3$ d'une solution saturée de bicarbonate de sodium, 250 $cm^3$ d'eau et 250 $cm^3$ d'une solution saturée de chlorure de sodium. Elle est séchée sur sulfate de magnésium anhydre, filtrée et le filtrat est concentré à sec sous pression réduite (30 mm de mercure; 4 kPa). Le résidu est purifié par chromatographie sur une colonne (hauteur: 60 cm, diamètre: 6 cm) de gel de silice (0,05-0,2 mm) en éluant par 7 l d'acétate d'éthyle et en recueillant des fractions

de 120 $cm^3$. Le contenu des fractions 18 à 56 est concentré à sec sous pression réduite (30 mm de mercure; 4 kPa) à 30° C et on obtient 26,74 g de benzhydryloxycarbonyl-2 t-butoxycarbonylamino-7 oxo-8 [(pyridyl-3 méthyl)-2 thiazolyl-5]-3 thia-5 aza-1 bicyclo-[4.2.0]-octène-2 sous la forme d'une meringue crème.

Spectre infrarouge ($CHBr_3$, bandes caractéristiques en $cm^{-1}$) 3420, 1785, 1720, 1500, 1455, 1425, 1390, 1240, 760.

Spectre de RMN du proton (250 MHz, $CDCl_3$, δ en ppm, J en Hz) 1,47 [s, 9H, $-C(CH_3)_3$]; 3,48 et 3,67 (2D, J = 18, 2H, $-S-CH_2-$); 4,1 (AB limite, J = 16, 2H, $-CH_2-$); 5,04 (d, J = 5, 1H, H en 6); 5,41 (d, J = 9, 1H, $-\underset{\underset{O}{\|}}{C}-NH-$); 5,71 (dd, J = 5 et 9, 1H, H en 7); 6,9 [s, 1H, $-COO-\underline{CH}-(C_6H_5)_2$]; 7,05 à 7,4 (mt, aromatiques et H en 5 de la pyridine); 7,39 (s, 1H, H du thiazole); 7,53 (ddd, J = 8-2,5 et 1, 1H, H en 4 de la pyridine); 8,50 (d, J = 2,5, 1H, H en 2 de la pyridine); 8,53 (dd, J = 5 et 1, 1H, H en 6 de la pyridine).

Le pyridyl-3 thioacétamide peut être préparé de la manière suivante.

Dans une solution de 50 g de pyridyl-3 acétonitrile, 54 $cm^3$ de triéthylamine dans 500 $cm^3$ d'éthanol absolu, on fait barboter un courant d'acide sulfhydrique pendant 6 h. On laisse reposer pendant 48 h puis on fait passer un courant d'azote. Le mélange est concentré à sec sous pression réduite (30 mm de mercure; 4 kPa) et l'huile résiduelle est reprise dans 150 $cm^3$ d'éthanol. On chauffe au reflux, filtre et la cristallisation se développe en refroidissant. Les cristaux sont filtrés, lavés par 10 $cm^3$ d'éthanol puis séchés sous pression réduite (0,1 mm de mercure; 0,013 kPa). On obtient 52 g de pyridyl-3 thioacétamide sous la forme d'une poudre cristalline blanche.

Pf. inst. 135-136° C Kofler.

Spectre de RMN du proton (60 MHz, DMSO $d_6$, δ en ppm, J en Hz) 3,9 (s, 2H, $-CH_2-$); 7,38 (q, J = 8 et 5, 1H, H en 5); 7,75 (dd, J = 8 et 2, 1H, H en 4); 8,50 (dd, J = 5 et 2, 1H, H en 6); 8,60 (d, J = 2, 1H, H en 2); 9,50 (s large, 2H, $-NH_2$).

*Exemple 10:*

On agite pendant 24 h à 23° C une solution de 13,06 g de benzhydryloxycarbonyl-2 [méthoxyimino-2 (tritylamino-2 thiazolyl-4)-2 acétamido]-7 [(pyridyl-3 amino)-2 thiazolyl-5]-3 oxo-8 thia-5 aza-1 bicyclo-[4.2.0]-octène-2, isomère syn, et de 2,11 g d'iodure de méthyle dans 135 $cm^3$ de N,N-diméthylformamide. Le mélange réactionnel est dilué par 270 $cm^3$ d'oxyde d'isopropyle et 540 $cm^3$ d'éther éthylique. La liqueur surnageante est décantée et le résidu huileux est repris dans 500 $cm^3$ d'éther éthylique. Le produit précipite. Le précipité est filtré et séché sous pression réduite (0,1 mm de mercure; 0,013 kPa) à 20° C. On obtient 11,55 g d'iodure de benzhydryloxycarbonyl-2 [méthoxyimino-2 (tritylamino-2 thiazolyl-4)-2 acétamido]-7 [(méthyl-1 pyridinio-3 amino)-2 thiazolyl-5]-3 oxo-8 thia-5 aza-1 bicyclo-[4.2.0]-octène-2, isomère syn, sous la forme d'une poudre jaune.

Spectre infrarouge (KBr, bandes caractéristiques en $cm^{-1}$) 3120, 2500, 1785, 1730, 1665, 1600, 1530, 1505, 1450, 1035, 755, 700, 670.

Spectre RMN du proton (350 MHz, DMSO $d_6$, δ en ppm, J en Hz) 3,85 (AB limite, J = 18, 2H, $-S-CH_2-$); 3,87 (S, 3H, $=N-OCH_3$); 4,42 (S, 3H, $\geqslant \underset{+}{N}-CH_3$); 5,28 (D, J = 5, 1H, H en 6); 5,84 (DD, J = 9 et 5, 1H, H en 7); 6,76 (S, 1H, H en 5 du thiazole); 6,92 [S, 1H, $-COO\underline{CH}(C_6H_5)_2$]; 7 à 7,5 (Mt, aromatiques et H en 4 du thiazole); 8,06 (DD, J = 9 et 6, 1H, H en 5 du pyridinio); 8,43 (D large, J = 9, 1H, H en 4 du pyridinio); 8,62 (D, J = 6, 1H, H en 6 du pyridinio); 8,85 [S, 1H, $-\underline{NH}-C(C_6H_5)_3$]; 9,2 (S large, 1H, H en 2 du pyridinio); 9,67 (D, J = 9, 1H, $-CO-NH-$); 11,30 (S, 1H, $-NH-$).

A une solution refroidie à 5° C de 0,78 g d'iodure de benzhydryloxycarbonyl-2 [méthoxyimino-2 (tritylamino-2 thiazolyl-4)-2 acétamido]-7 [(méthyl-1 pyridinio-3 amino)-2 thiazolyl-5]-3 oxo-8 thia-5 aza-1 bicyclo-[4.2.0]-octène-2, isomère syn dans 0,7 $cm^3$ d'anisole, on ajoute 4,6 $cm^3$ d'acide trifluoracétique et 0,46 $cm^3$ d'eau distillée. Le mélange est agité à 5° C pendant 5 min, puis à 23° C pendant 1 h 15 min. La solution est diluée successivement par 3 $cm^3$ d'acétone puis par 20 $cm^3$ d'éther. Le mélange hétérogène est agité pendant 15 min puis filtré et le précipité recueilli séché sous pression réduite (0,1 mm de mercure; 0,013 kPa). On obtient 0,51 g de ditrifluoracétate d'[(amino-2 thiazolyl-4)-2 méthoxyimino-2 acétamido]-7 carboxy-2 [(méthyl-1 pyridinio-3 amino)-2 thiazolyl-5]-3 oxo-8 thia-5 aza-1 bicyclo-[4.2.0]-octène-2, isomère syn, sous la forme d'une poudre ocre jaune.

Spectre infrarouge (KBr, bandes caractéristiques en $cm^{-1}$) 3400, 2200, 1775, 1675, 1530, 1510, 1200, 1140, 1050, 800, 725, 670.

Spectre RMN du proton (350 MHz, DMSO $d_6$, δ en ppm, J en Hz) 3,83 et 3,93 (2D, J = 18, 2H, $-S-CH_2-$); 3,92 (S, 3H, $=NOCH_3$); 4,38 (S, 3H, $\geqslant \underset{+}{N}-CH_3$); 5,27 (D, J = 5, 1H, H en 6); 5,87 (DD, J = 8 et 5, 1 H, H en 7); 6,83 (S, 1H, H en 5 du thiazole); 7,52 (S, 1H, H en 4 du thiazole); 8,02 (DD, J = 8,5 et 5, 1H, H en 5 du pyridinio); 8,47 (D, J = 8,5, 1H, H en 4 du pyridinio); 8,57 (D, J = 5, 1H, H en 6 du pyridinio); 8,5 à 5 (Mf très étalé, $-NH_2$ et $-COOH$); 9,39 (S, 1H, H en 2 du pyridinio); 9,76 (D, J = 8, 1H, $-CO-NH-$); 11,6 (Mf, 1H, $-NH-$).

A une suspension de 0,35 g de ditrifluoracétate d'[(amino-2 thiazolyl-4)-2 méthoxyimino-2 acétamido]-7 carboxy-2 [(méthyl-1 pyridinio-3 amino)-2 thiazolyl-5]-3 oxo-8 thia-5 aza-1 bicyclo-[4.2.0]-octène-2, isomère syn, dans 7 $cm^3$ d'eau distillée, on ajoute 0,9 $cm^3$ de résine Amberlite LA-2 en solution dans 2,6 $cm^3$ de méthylisobutylcétone. On suit l'évolution de l'acidité du mélange au pH-mètre. A la neutralité (pH = 6,8), on filtre la suspension et on lave le précipité obtenu successivement par 2 fois 2 $cm^3$ de méthyl-

isobutylcétone, 2 fois 2 $cm^3$ d'eau distillée, 3 fois 2 $cm^3$ d'éthanol, 3 fois 2 $cm^3$ d'acétone et 3 fois 10 $cm^3$ d'éther éthylique. Le solide est séché sous pression réduite (0,1 mm de mercure; 0,013 kPa) et on obtient 0,2 g d'[(amino-2 thiazolyl-4)-2 méthoxyimino-2 acétamido]-7 carboxylato-2 [(méthyl-1 pyridinio-3 amino)-2 thiazolyl-5]-3 oxo-8 thia-5 aza-1 bicyclo-[4.2.0]-octène-2, isomère syn, sous la forme d'une poudre jaune.

Spectre infrarouge (KBr, bandes caractéristiques en $cm^{-1}$) 3500, 2000, 1760, 1670, 1530, 1030, 670.

Spectre RMN du proton (250 MHz, DMSO + 1 goutte de $CF_3COOD$; δ en ppm, J en Hz) 3,83 et 3,93 (2D, J = 18, 2H, $-S-CH_2-$); 3,94 (S, 3H, $=N-OCH_3$); 4,37 (S, 3H, $\geqslant \overset{+}{N}-CH_3$); 5,27 (D, J = 5, 1H, H en 6); 5,87 (DD, J = 8 et 5, 1H, H en 7); 6,91 (S, 1H, H en 5 du thiazole); 7,53 (S, 1H, H en 4 du thiazole); 8,02 (DD, J = 6 et 8, 1H, H en 5 du pyridinio); 8,47 (D large, J = 8, 1H, H en 4 du pyridinio); 8,57 (D, J = 6, 1H, H en 6 du pyridinio); 9,38 (S large, 1H, H en 2 du pyridinio); 9,82 (D, J = 8, 1H, $-CO-NH-$).

Le benzhydryloxycarbonyl-2 [méthoxyimino-2 (tritylamino-2 thiazolyl-4)-2 acétamido]-7 [(pyridyl-3 amino)-2 thiazolyl-5]-3 oxo-8 thia-5 aza-1 bicyclo-[4.2.0]-octène-2, isomère syn peut être préparé de la manière suivante.

A une solution refroidie à 5° C de 10,83 g d'amino-7 benzhydryloxycarbonyl-2 [(pyridyl-3 amino)-2 thiazolyl-5]-3 oxo-8 thia-5 aza-1 bicyclo-[4.2.0]-octène-2, 10,65 g d'acide méthoxyimino-2 (tritylamino-2 thiazolyl-4)-2 acétique, isomère syn, dans 200 $cm^3$ de N,N'-diméthylformamide sec, on ajoute 3,24 g d'hydroxy-1 benzothiazole et 4,95 g de N,N'-dicyclohexylcarbodiimide. Le mélange est agité 30 min à +5° C puis 4 h à 20° C. On ajoute 1 $cm^3$ d'acide acétique au mélange réactionnel, puis on filtre le précipité. Le filtrat est dilué par 900 $cm^3$ d'acétate d'éthyle, et la phase organique est lavée successivement par 2 fois 500 $cm^3$ d'eau distillée, 500 $cm^3$ d'une solution d'acide chlorhydrique décinormale, 500 $cm^3$ d'une solution demi-saturée de bicarbonate de sodium, 2 fois 500 $cm^3$ d'eau distillée et 500 $cm^3$ d'une solution demi-saturée de chlorure de sodium. Après séchage de la phase organique sur sulfate de magnésium, filtration et concentration sous pression réduite (30 mm de mercure; 4 kPa), on obtient une merigue jaune qui est purifiée par chromatographie sur colonne (hauteur: 61 cm; diamètre: 4,8 cm) de gel de silice (0,06-0,20 mm) en éluant par 2 l d'un mélange de cyclohexane et d'acétate d'éthyle 25/75 (en volumes) et 500 $cm^3$ d'acétate d'éthyle en recueillant des fractions de 250 $cm^3$. On réunit les fractions 5 à 14 que l'on concentre à sec sous pression réduite (30 mm de mercure; 4 kPa) à 30° C. On obtient 14,6 g de benzhydryloxycarbonyl-2 [méthoxyimino-2 (tritylamino-2 thiazolyl-4)-2 acétamido]-7 [(pyridyl-3 amino)-2 thiazolyl-5]-3 oxo-8 thia-5 aza-1 bicyclo-[4.2.0]-octène-2, isomère syn sous la forme d'une meringue jaune vif.

Spectre infrarouge (KBr, bandes caractéristiques en $cm^{-1}$) 3380, 3180, 1785, 1730, 1680, 1530, 1490, 1040, 755, 700.

Spectre de RMN du proton (350 MHz, DMSO $d_6$, δ en ppm, J en Hz) 3,84 (AB limite, J = 18, 2H, $-S-CH_2-$); 3,85 (S, 3H, $=N-OCH_3$); 5,26 (D, J = 5, 1H, H en 6); 5,81 (DD, J = 8 et 5, 1H, H en 7); 6,76 (S, 1H, H en 5 du thiazole); 6,89 [S, 1H, $-COO-C\underline{H}(C_6H_5)_2$]; 7 à 7,50 (Mt, aromatiques, H en 4 du thiazole et H en 5 de la pyridine); 8,07 (DDD, J = 8, 2 et 1,5, 1H, H en 4 de la pyridine); 8,19 (DD, J = 6 et 1,5, 1H, H en 6 de la pyridine); 8,70 (D, J = 2, 1H, H en 2 de la pyridine); 8,85 [S, 1H, $-N\underline{H}-C(C_6H_5)_3$]; 9,68 (D, J = 8, 1H, $-CON\underline{H}-$); 10,46 (S large, 1H, $-NH-$).

L'amino-7 benzhydryloxycarbonyl-2 [(pyridyl-3 amino)-2 thiazolyl-5]-3 oxo-8 thia-5 aza-1 bicyclo-[4.2.0]-octène-2 peut être préparé de la manière suivante.

A une solution de 37,22 g de benzhydryloxycarbonyl-2 t-butoxycarbonylamino-7 [(pyridyl-3 amino)-2 thiazolyl-5]-3 oxo-8 thia-5 aza-1 bicyclo-[4.2.0]-octène-2 dans 372 $cm^3$ d'acétonitrile on ajoute en 5 min 37,7 $cm^3$ d'acide méthanesulfonique. Le mélange est agité pendant 5 min puis on ajoute la solution réactionnelle dans un mélange de 870 $cm^3$ d'une solution saturée de bicarbonate de sodium, 1740 $cm^3$ d'eau distillée et 580 $cm^3$ de chlorure de méthylène. On agite pendant 10 min et on filtre le précipité que l'on lave par 4 fois 100 $cm^3$ d'eau distillée. Le produit est séché et on obtient 27,66 g d'amino-7 benzhydryloxycarbonyl-2 [(pyridyl-3 amino)-2 thiazolyl-5]-3 oxo-8 thia-5 aza-1 bicyclo-[4.2.0]-octène-2 sous la forme d'une poudre cristalline jaune pâle.

Spectre infrarouge (KBr, bandes caractéristiques en $cm^{-1}$) 3380, 3150, 2000, 1780, 1730, 1670, 1590, 1550, 1530, 1490, 1225, 800, 760, 750, 700.

Spectre de RMN du proton (250 MHz, DMSO $d_6$, δ en ppm, J en Hz) 2,4 (mf, 2H, $-NH_2$); 3,78 (AB limite, J = 18, 2H, $-S-CH_2-$); 4,88 (d large, J = 5, 1H, H en 7); 5,1 (d, J = 5, 1H, $-H$ en 6); 6,85 [s, 1H, $-COO-C\underline{H}(C_6H_5)_2$]; 7 à 7,35 (mt, 11H aromatiques et $-\underline{H}$ du thiazole); 7,34 (mt, 1H, $-H$ en 5 de la pyridine); 8,06 (ddd, J = 8-2,5 et 1, 1H, $-H$ en 4 de la pyridine); 8,17 (dd, J = 5 et 1, 1H, $-H$ en 6 de la pyridine); 8,68 (d, J = 2,5, 1H, $-H$ en 2 de la pyridine); 10,38 (mf, 1H, $-NH-$).

Le benzhydryloxycarbonyl-2 t-butoxycarbonylamino-7 [(pyridyl-3 amino)-2 thiazolyl-5]-3 oxo-8 thia-5 aza-1 bicyclo-[4.2.0]-octène-2 peut être préparé de la manière suivante.

A une solution refroidie à −75° C de 107,1 g de benzhydryloxycarbonyl-2 t-butoxycarbonylamino-7 (diméthylamino-2 vinyl)-3 oxo-8 thia-5 aza-1 bicyclo-[4.2.0]-octène-2, forme E, dans 600 $cm^3$ de tétrahydrofuranne sec, on ajoute goutte à goutte une solution de 35,96 g de brome dans 40 $cm^3$ de dichlorométhane sec. Le mélange est agité à cette température pendant 10 min, puis on ajoute 30,64 g de pyridyl-3 thio-urée en solution dans 400 $cm^3$ d'un mélange eau/tétrahydrofuranne (50/50 en volumes). On enlève le bain réfrigérant et on agite à 20° C pendant 17 h. La solution brune est diluée par 1,5 l d'acétate

d'éthyle puis lavée successivement par 1 l d'eau distillée, 1 l d'une solution de bicarbonate de sodium demi-saturée, 1 l d'eau distillée et 500 $cm^3$ d'une solution saturée de chlorure de sodium. La phase organique est séchée sur sulfate de magnésium anhydre. Après évaporation du solvant sous pression réduite (30 mm de mercure; 4 kPa) à 30° C, le résidu est chromatographié sur colonne (hauteur: 46 cm; diamètre: 8,1 cm) de gel de silice (0,06-0,20 mm) en éluant successivement par 5 l d'un mélange cyclohexane/acétate d'éthyle (50/50 en volumes), 5 l d'un mélange cyclohexane/acétate d'éthyle (25/75 en volumes) et 7 l d'acétate d'éthyle, en recueillant des fractions de 1 l. Les fractions 8 à 17 contenant le produit pur sont rassemblées et concentrées jusqu'à un volume résiduel de 250 $cm^3$. On obtient une suspension jaune conservée à 5° C pendant 12 h. Le solide est filtré, lavé par 3 fois 100 $cm^3$ d'éther éthylique et séché sous pression réduite (1 mm de mercure; 0,13 kPa). On obtient ainsi 14,5 g de benzhydryloxycarbonyl-2 t-butoxycarbonylamino-7 [(pyridyl-3 amino)-2 thiazolyl-5]-3 oxo-8 thia-5 aza-1 bicyclo-[4.2.0]-octène-2 sous la forme d'une poudre jaune.

Spectre infrarouge (KBr, bandes caractéristiques en $cm^{-1}$) 3330, 3100, 2500, 1790, 1720, 1590, 1530, 1500, 1370, 1160, 760, 745, 705.

Spectre de RMN du proton (250 MHz, DMSO $d_6$, δ en ppm, J en Hz) 1,45 [s, 9H, $-C(CH_3)_3$]; 3,76 et 3,88 (2d, J = 18, 2H, $-S-CH_2-$); 5,18 (d, J = 5, 1H, –H en 6); 5,59 (dd, J = 9 et 5, 1H, –H en 7); 6,88 [s, 1H, $-COO-\underline{CH}(C_6H_5)_2$]; 7 à 7,4 (mt, 11H, aromatiques et $-\overline{H}$ du thiazole); 7,34 (mt, 1H, –H en 5 de la pyridine); 8,07 (ddd, J = 8-2,5 et 1, 1H, –H en 4 de la pyridine); 8,11 (d, J = 9, 1H, –CONH–); 8,19 (dd, J = 5 et 1, 1H, –H en 6 de la pyridine); 8,69 (d, J = 2, 1H, –H en 2 de la pyridine); 10,45 (s, 1H, =N–H).

*Exemple 11:*

Une solution de 1,67 g de benzhydryloxycarbonyl-2 {[(t-butoxycarbonyl-2 propyl-2) oxyimino]-2 (tritylamino-2 thiazolyl-4)-2 acétamido}-7 oxo-8 oxyde-5 [(pyridyl-3 amino)-2 thiazolyl-5]-3 thia-5 aza-1 bicyclo-[4.2.0]-octène-2, isomère syn, et de 0,10 $cm^3$ d'iodure de méthyle dans 15 $cm^3$ de N,N-diméthylformamide est agitée pendant 24 h. On dilue le mélange en ajoutant 30 $cm^3$ d'oxyde d'isopropyle. Les liqueurs surnageantes sont décantées, et le résidu est agité avec 50 $cm^3$ d'oxyde d'éthyle. Le précipité est filtré et séché sous pression réduite (0,1 mm de mercure; 0,013 kPa) à 20° C. On obtient 1,53 g d'iodure de benzhydryloxycarbonyl-2 {[(t-butoxycarbonyl-2 propyl-2) oxyimino]-2 (tritylamino-2 thiazolyl-4)-2 acétamido}-7 [(méthyl-1 pyridinio-3 amino)-2 thiazolyl-5]-3 oxo-8 oxyde-5 thia-5 aza-1 bicyclo-[4.2.0]-octène-2, isomère syn.

Spectre infrarouge ($CHBr_3$, bandes caractéristiques en $cm^{-1}$) 3380, 3100, 2500, 1806, 1730, 1675, 1600, 1505, 1450, 1370, 1145, 755, 700, 675.

Spectre de RMN du proton (250 MHz, DMSO $d_6$, δ en ppm, J en Hz) 1,36 [S, 9H, $-C(CH_3)_3$]; 1,44 et 1,45 [2S, 6H, $> C(CH_3)_2$]; 3,83 et 4,37 (2D, J = 18,5, 2H, $-SCH_2-$); 4,39 (S, 3H, $\geqslant \overset{+}{N}-CH_3$); 5,12 (D, J = 5, 1H, –H en 6); 6,08 (DD, J = 9 et 5, 1H, –H en 7); 6,80 (S, 1H, –H en 5 du thiazole); 6,95 [S, 1H, $-COO-\underline{CH}(C_6H_5)_2$]; 7 à 7,4 (Mt, aromatiques et –H en 4 du thiazole); 8,05 (DD, J = 8 et 6, 1H, –H en 5 du pyridinio); 8,35 (D, J = 9, 1H, –CO–NH–); 8,41 (D large, J = 8, 1H, –H en 4 du pyridinio); 8,6 (D, J = 6, 1H, –H en 6 du pyridinio); 8,78 [S, 1H, $-N\underline{H}C(C_6H_5)_3$]; 9,19 (S large, 1H, –H en 2 du pyridinio); 11,33 (Mf, 1H, > NH).

A une solution refroidie à 5° C de 1,25 g de benzhydryloxycarbonyl-2 {[(t-butoxycarbonyl-2 propyl-2) oxyimino]-2 (tritylamino-2 thiazolyl-4)-2 acétamido}-7 [(méthyl-1 pyridinio-3 amino)-2 thiazolyl-5]-3 oxo-8 oxyde-5 thia-5 aza-1 bicyclo-[4.2.0]-octène-2, isomère syn dans 1 $cm^3$ d'anisole, on ajoute 6,5 $cm^3$ d'acide trifluoroacétique et 0,65 cm d'eau distillée. On agite à cette température pendant 10 min puis 1 h 15 min à 22° C. Le mélange réactionnel est ensuite dilué dans 4,3 $cm^3$ d'acétone puis 40 $cm^3$ d'oxyde d'éthyle. On agite pendant 5 min, filtre et lave le précipité par 5 fois 10 $cm^3$ d'oxyde d'éthyle. On obtient 0,78 g de ditrifluoracétate d'{[(amino-2 thiazolyl-4)-2 (carboxy-2 propyl-2) oxyimino]-2 acétamido}-7 carboxy-2 [(méthyl-1 pyridinio-3 amino)-2 thiazolyl-5]-3 oxo-8 oxyde-5 thia-5 aza-1 bicyclo-[4.2.0]-octène-2, isomère syn, sous la forme d'une poudre brun verdâtre.

Spectre infrarouge (KBr, bandes caractéristiques en $cm^{-1}$) 3700, 2200, 1790, 1675, 1635, 1525, 1510, 1200, 1145, 800, 720, 670.

Spectre de RMN du proton (250 MHz, DMSO $d_6$, δ en ppm, J en Hz) 1,51 et 1,52 [2S, 6H, $> C(CH_3)_2$]; 3,8 et 4,41 (2D, J = 18,5 2H, $-S-CH_2-$); 4,36 (S, 3H, $\geqslant \overset{+}{N}-CH_3$); 5,11 (D, J = 5, 1H, –H en 6); 6,08 (DD, J = 8 et 5, 1H, –H en 7); 6,86 (S, 1H, –H en 5 du thiazole); 7,10 à 7,8 (Mf, 3H, $-\overset{+}{N}H_3$); 7,54 (S, 1H, –H en 4 du thiazole); 8,01 (DD, J = 8,5 et 5, 1H, –H en 5 du pyridinio); 8,44 (D large, J = 8,5, 1H, –H en 4 du pyridinio); 8,56 (D, J = 5, 1H, –H en 6 du pyridinio); 8,63 (D, J = 8, 1H, –CONH–); 9,35 (S large, 1H, –H en 2 du pyridinio); 11,6 (Mf, 1H, > N–H); 14 à 11 (Mf étalé, –COOH).

A une suspension de 0,71 g de ditrifluoracétate d'{[(amino-2 thiazolyl-4)-2 (carboxy-2 propyl-2) oxyimino]-2 acétamido}-7 carboxy-2 [(méthyl-1 pyridinio-3 amino)-2 thiazolyl-5]-3 oxo-8 oxyde-5 thia-5 aza-1 bicyclo-[4.2.0]-octène-2, isomère syn dans 21,3 $cm^3$ d'eau distillée, on ajoute une solution de 1,75 $cm^3$ de résine Amberlite LA-2 dans 5,35 $cm^3$ de méthylisobutylcétone. Le mélange est agité jusqu'à pH 6,2 puis la phase liquide est filtrée et le filtrat est lyophilisé. Le lyophilisat est agité pendant 2 h dans 25 $cm^3$ d'éther anhydre, filtré et lavé par 2 fois 10 $cm^3$ d'éther, séché sous pression réduite (0,1 mm de mercure; 0,013 kPa) pour donner 0,12 g d'{(amino-2 thiazolyl-4)-2 [(carboxy-2 propyl-2) oxyimino]-2 acétamido}-7 carboxylato-2 [(méthyl-1 pyridinio-3 amino)-2 thiazolyl-5]-3 oxo-8 oxyde-5 thia-5 aza-1 bicyclo-[4.2.0]-octène-2, isomère syn, sous la forme d'une poudre jaune.

Spectre infrarouge (KBr, bandes caractéristiques en $cm^{-1}$) 1780, 1670, 1610, 1530, 1395, 1040, 675.

Spectre de RMN du proton (250 MHz, DMSO $d_6$, δ en ppm, J en Hz) vers 1,51 ppm [Mf, $>C(CH_3)_2$]; 3,78 et 4,25 (2D, J = 18,5, 2H, $-S-CH_2-$); 4,31 (S, 3H, $\geqslant \underset{+}{N}-CH_3$); 5,05 (D, J = 4, 1H, −H en 6); 5,9 (Mf, 1H, −H en 7); 6,81 (S, 1H, −H en 5 du thiazole); 7,23 (Mf, 2H, $-NH_2$); 7,49 (S, 1H, −H en 4 du thiazole); 7,87 (Mf, 1H, −H en 5 du pyridinio); 8,42 (Mf, 1H, −H en 6 du pyridinio); 9,05 (Mf, 1H, H en 4 du pyridinio); 5,20 (Mf, 1H, −H en 2 du pyridinio).

Le benzhydryloxycarbonyl-2 {[(t-butoxycarbonyl-2 propyl-2) oxyimino]-2 (tritylamino-2 thiazolyl-4)-2 acétamido}-7 oxo-8 oxyde-5 [(pyridyl-3 amino)-2 thiazolyl-5]-3 thia-5 aza-1 bicyclo-[4.2.0]-octène-2, isomère syn peut être préparé de la manière suivante.

A une solution refroidie à 0° C de 13,46 g de benzhydryloxycarbonyl-2 {[(t-butoxycarbonyl-2 propyl-2) oxyimino]-2 (tritylamino-2 thiazolyl-4)-2 acétamido}-7 oxo-8 [(pyridyl-3 amino)-2 thiazolyl-5]-3 thia-5 aza-1 bicyclo-[4.2.0]-octène-2, isomère syn dans 123 $cm^3$ de dichlorométhane, on coule pendant 30 min une solution de 2,59 g d'acide m-chloroperbenzoïque dans 52 $cm^3$ de chlorure de méthylène. On agite pendant 15 min à 0° C puis on dilue la solution réactionnelle par 200 $cm^3$ de chlorure de méthylène. Le mélange est lavé successivement par 200 $cm^3$ d'une solution demi-saturée de bicarbonate de sodium, puis 2 fois 200 $cm^3$ d'eau distillée. La phase organique est alors séchée sur sulfate de magnésium anhydre, filtrée puis concentrée sous pression réduite (100 mm de mercure; 13,3 kPa) à 30° C. Le résidu est purifié par chromatographie sur une colonne (hauteur: 29 cm; diamètres: 5,8 cm) de gel de silice (0,04-0,06 mm) en éluant sous une pression de 0,4 bar par 2 l d'un mélange cyclohexane/acétate d'éthyle (10/90 en volumes) et en recueillant des fractions de 125 $cm^3$. Les fractions 11 à 15 sont réunies et concentrées à sec sous pression réduite (30 mm de mercure; 4 kPa) à 30° C pour donner 0,70 g de benzhydryloxycarbonyl-2 {[(t-butoxycarbonyl-2 propyl-2) oxyimino]-2 (tritylamino-2 thiazolyl-4)-2 acétamido}-7 oxo-8 oxyde-5 [(pyridyl-3 amino)-2 thiazolyl-5]-3 thia-5 aza-1 bicyclo-[4.2.0]-octène-2, isomère syn, sous la forme d'une meringue jaune.

Spectre infrarouge ($CHBr_3$, bandes caractéristiques en $cm^{-1}$) 3390, 1800, 1725, 1680, 1530, 1495, 1450, 1370, 1050, 700.

Spectre de RMN du proton (250 MHz, $CDCl_3$, δ en ppm, J en Hz) 1,41 [S, 9H, $-C(CH_3)_3$]; 1,56 et 1,58 [2S, 6H, $>C(CH_3)_2$]; 3,25 et 3,84 (2D, J = 18,5, 2H, $-S-CH_2-$); 4,56 (D, J = 5, 1H, −H en 6); 6,19 (DD, J = 9,5 et 5, 1H, −H en 7); 6,72 (S, 1H, −H en 5 du thiazole); 6,92 [S, 1H, $-COO-C\underline{H}(C_6H_5)_2$]; 7,01 (S, 1H, −H en 4 du thiazole); 7,11 (DD, J = 5 et 7,5, −H en 5 de la pyridine); 7,05 à 7,45 (Mt, aromatiques); 7,9 (DDD, J = 7,5, 2 et 1,5, 1H, −H en 4 de la pyridine); 8,0 (D, J = 9,5, 1H, −CONH−); 8,21 (DD, J = 5 et 1,5, 1H, −H en 6 de la pyridine); 8,6 (D, J = 2, 1H, −H en 2 de la pyridine); 8,73 (Mf, 1H, $-N\underline{H}C(C_6H_5)_3$].

Le benzhydryloxycarbonyl-2 {[t-butoxycarbonyl-2 propyl-2 oxyimino]-2 (tritylamino-2 thiazolyl-4)-2 acétamido}-7 oxo-8 [(pyridyl-3 amino)-2 thiazolyl-5]-3 thia-5 aza-1 bicyclo-[4.2.0]-octène-2, isomère syn, peut être préparé de la manière suivante.

A une solution refroidie à +5° C de 10,83 g d'amino-7 benzhydryloxycarbonyl-2 oxo-8 [(pyridyl-3 amino)-2 thiazolyl-5]-3 thia-5 aza-1 bicyclo-[4.2.0]-octène-2 et de 13,72 g d'acide (tritylamino-7 thiazolyl-4)-2 [(t-butoxycarbonyl-2 propyl-2) oxyimino]-2 acétique, isomère syn, dans 200 $cm^3$ de N,N-diméthylformamide anhydre, on ajoute 3,24 g d'hydroxy-1 benzotriazole et 4,95 g de N,N'-dicyclohexylcarbodiimide. Le mélange est agité pendant 30 min à 5° C et 3½ h à 22° C. On ajoute 1 $cm^3$ d'acide acétique, puis filtre le mélange, lave le précipité par 20 $cm^3$ de diméthylformamide et dilue le filtrat par 900 $cm^3$ d'acétate d'éthyle. La phase organique est lavée par 2 fois 500 $cm^3$ d'eau distillée, 500 $cm^3$ d'une solution aqueuse 0,1N d'acide chlorhydrique, 500 $cm^3$ d'une solution demi-saturée de bicarbonate de sodium, 2 fois 500 $cm^3$ d'eau distillée et 500 $cm^3$ d'une solution saturée de chlorure de sodium. Elle est séchée sur sulfate de magnésium anhydre; le mélange est filtré et le filtrat est concentré à sec sous pression réduite (30 mm de mercure; 4 kPa) à 30° C. Le résidu est purifié par chromatographie sur une colonne (hauteur: 63 cm; diamètre: 4,8 cm) de gel de silice (0,06-0,20 mm) en éluant sous une pression de 0,4 bar par 4 l d'un mélange cyclohexane/acétate d'éthyle (50/50 en volumes) et en recueillant des fractions de 250 $cm^3$. Les fractions 8 à 14 contenant le produit pur sont réunis et concentrés sous pression réduite (30 mm de mercure; 4 kPa) à 30° C pour donner 4,65 g de benzhydryloxycarbonyl-2 {[(t-butoxycarbonyl-2 propyl-2) oxyimino]-2 (tritylamino-2 thiazolyl-4)-2 acétamido}-7 oxo-8 [(pyridyl-3 amino)-2 thiazolyl-5]-3 thia-5 aza-1 bicyclo-[4.2.0]-octène-2, isomère syn, sous la forme d'une meringue jaune.

Spectre infrarouge ($CHBr_3$, bandes caractéristiques en $cm^{-1}$) 3400, 1790, 1725, 1680, 1525, 1495, 1450, 1370, 750, 740.

Spectre de RMN du proton (250 MHz, DMSO $d_6$, δ en ppm, J en Hz) 1,37 [S, 9H, $-C(CH_3)_3$]; 1,43 [S, 6H, $>C(CH_3)_2$]; 3,77 et 3,89 (2D, J = 17,5, 2H, $-S-CH_2-$); 5,28 (D, J = 5, 1H, −H en 6); 5,83 (DD, J = 8 et 5, 1H, −H en 7); 6,72 (S, 1H, −H en 5 du thiazole); 6,89 [S, 1H, $-COO-C\underline{H}(C_6H_5)_2$]; 7 à 7,4 (Mt, aromatiques, −H en 4 du thiazole et −H en 5 de la pyridine); 8,07 (D large, J = 7,5, 1H, −H en 4 de la pyridine);

8,19 (D large, J = 5, 1H, −H en 6 de la pyridine); 8,68 (D, J = 2, 1H, −H en 2 de la pyridine); 8,83 [S, 1H, $-NH-C(C_6H_5)_3$]; 9,48 (D, J = 8, 1H, −CO−NH−); 10,44 (S, 1H, > N−H).

*Exemple 12:*

L'iodure de benzhydryloxycarbonyl-2 [(t-butoxycarbonyl-2 propyl-2) oxyimido-2 (tritylamino-2 thiazolyl-4)-2 acétamido]-7 [(méthyl-1 pyridinio-3 amino)-2 thiazolyl-5]-3 oxo-8 oxyde-5 thia-5 aza-1 bicyclo-[4.2.0]-octène-2, isomère syn obtenu comme à l'exemple 11 peut être traité de la manière suivante.

A une solution refroidie à 0° C de 3,76 g d'iodure de benzhydryloxycarbonyl-2 [(t-butoxycarbonyl-2 propyl-2) oxyimino-2 (tritylamino-2 thiazolyl-4)-2 acétamido]-7 [(méthyl-1 pyridinio-3 amino)-2 thiazolyl-5]-3 oxo-8 oxyde-5 thia-5 aza-1 bicyclo-[4.2.0]-octène-2, isomère syn dans 30 cm³ de chlorure de méthylène, on ajoute 2,4 cm³ de N,N-diméthylacétamide, puis 1,06 cm³ de trichlorure de phosphore. On agite pendant 45 min à 0° C puis on dilue la masse réactionnelle dans 120 cm³ d'acétate d'éthyle. Un produit précipite, on filtre le précipité et on le lave par 3 fois 25 cm³ d'acétate d'éthyle, et 2 fois 25 cm³ d'éther éthylique. Le produit est dissous dans 100 cm³ de chlorure de méthylène, puis traité au noir 3S. Le filtrat est concentré à sec sous pression réduite (100 mm de mercure; 13,3 kPa) et le résidu est repris par 50 cm³ d'éther éthylique. Le mélange hétérogène est filtré et le solide lavé par 3 fois 15 cm³ d'éther éthylique. Il est séché sous pression réduite (0,1 mm de mercure; 0,013 kPa) et on obtient 2,95 g d'iodure de benzhydryloxycarbonyl-2 [(t-butoxycarbonyl-2 propyl-2) oxyimino-2 (tritylamino-2 thiazolyl-4)-2 acétamido]-7 [(méthyl-1 pyridinio-3 amino)-2 thiazolyl-5]-3 oxo-8 thia-5 aza-1 bicyclo-[4.2.0]-octène-2, isomère syn.

Spectre infrarouge (KBr, bandes caractéristiques en $cm^{-1}$) 2980, 2940, 1790, 1728, 1698, 1595, 1575, 1530, 1510, 1450, 1375, 1225, 1142, 1003, 760, 705.

Spectre de RMN du proton (250 MHz, DMSO $d_6$, δ en ppm, J en Hz) 1,38 [S, 9H, $-COO-C(CH_3)_3$]; 1,42 [S, 6H, $>C(CH_3)_2$]; 3,79 et 3,94 (2D, J = 17,5, 2H, $-S-CH_2-$); 4,38 (S, 3H, $\geqslant \overset{+}{N}-CH_3$); 5,27 (D, J = 5, 1H, −H en 6); 5,85 (DD, J = 8 et 5, 1H, −H en 7); 6,72 (S, 1H, −H en 5 du thiazole); 6,90 [S, 1H, $-COO-CH-(C_6H_5)_2$]; 6,95 à 7,4 (Mt, aromatiques + H en 4 du thiazole); 8,05 (DD, J = 8 et 5,5, 1H, −H en 5 du pyridinio); 8,39 (D, J = 8, 1H, −H en 4 du pyridinio); 8,59 (D, J = 5,5, 1H, −H en 6 du pyridinio); 8,83 [S, 1H, $-NHC(C_6H_5)_3$]; 9,16 (S large, 1H, −H en 2 du pyridinio); 9,48 (D, J = 8, 1H, −CO−NH−); 11,29 (Mf, 1H, −NH−).

On chauffe à 50° C pendant 30 min une solution de 2,85 g d'iodure de benzhydryloxycarbonyl-2 [(t-butoxycarbonyl-2 propyl-2) oxyimino-2 (tritylamino-2 thiazolyl-4)-2 acétamido]-7 [(méthyl-1 pyridinio-3 amino)-2 thiazolyl-5]-3 oxo-8 thia-5 aza-1 bicyclo-[4.2.0]-octène-2, isomère syn dans 42,8 cm³ d'acide formique et 4,3 cm³ d'anisole. Le mélange est alors dilué par 14,3 cm³ d'eau distillée et agité à 50° C pendant 15 min. La solution réactionnelle est concentrée à sec sous pression réduite (0,1 mm de mercure; 0,013 kPa) à 40° C. Le résidu est repris dans 100 cm³ d'éthanol que l'on évapore sous pression réduite (0,1 mm de mercure; 0,013 kPa) jusqu'à un volume résiduel d'environ 40 cm³. On répète l'opération et on filtre le produit que l'on lave par 2 fois 25 cm³ d'éthanol, 2 fois 25 cm³ d'acétate d'éthyle et 2 fois 25 cm³ d'éther éthylique. Le produit (0,95 g) est séché sous pression réduite (0,1 mm de mercure; 0,013 kPa). On en prélève 0,58 g que l'on dissout dans 0,75 cm³ d'anisole. On obtient une solution marron que l'on refroidit vers 5° C. On ajoute une solution froide de 4,90 cm³ d'acide trifluoracétique et 0,49 cm³ d'eau distillée. Le mélange est agité 15 min à 5° C puis 1 h 15 min à 25° C. Le mélange réactionnel est dilué par 3,2 cm³ d'acétone, puis on coule 13 cm³ d'éther éthylique. Le mélange est agité pendant 10 min, filtré et le précipité est lavé par 2 fois 10 cm³ d'éther éthylique et séché sous pression réduite (0,1 mm de mercure; 0,013 kPa). On obtient 0,60 g de ditrifluoracétate d'{(amino-2 thiazolyl-4)-2 [(carboxy-2 propyl-2) oxyimino]-2 acétamido}-7 carboxy-2 [(méthyl-1 pyridinio-3 amino)-2 thiazolyl-5]-3 oxo-8 thia-5 aza-1 bicyclo-[4.2.0]-octène-2, isomère syn, sous la forme d'une poudre marron clair.

A 0,62 g de ditrifluoracétate d'{(amino-2 thiazolyl-4)-2 [(carboxy-2 propyl-2) oxyimino]-2 acétamido}-7 carboxy-2 [(méthyl-1 pyridinio-3 amino)-2 thiazolyl-5]-3 oxo-8 thia-5 aza-1 bicyclo-[4.2.0]-octène-2, isomère syn dans 62 cm³ d'eau distillée, on ajoute 12 cm³ de résine Amberlite IR 45 (OH). On agite le mélange pendant 40 min, on filtre la résine et on extrait le filtrat par 25 cm³ d'acétate d'éthyle. La phase aqueuse est concentrée à l'évaporateur rotatif sous pression réduite (30 mm de mercure; 4 kPa) jusqu'à un volume résiduel de 50 cm³. Elle est alors lyophilisée pour donner 0,15 g d'un lyophilisat jaune pâle d'{(amino-2 thiazolyl-4)-2 [(carboxy-2 propyl-2) oxyimido]-2 acétamido}-7 carboxylato-2 [(méthyl-1 pyridinio-3 amino)-2 thiazolyl-5]-3 oxo-8 thia-5 aza-1 bicyclo-[4.2.0]-octène-2, isomère syn.

Spectre infrarouge (KBr, bandes caractéristiques en $cm^{-1}$) 3420, 3100, 2985, 1765, 1670, 1600, 1575, 1525, 1470, 1390, 1360, 1335, 1290, 1220, 1150, 1075, 1030, 980, 910, 830, 810, 765, 670, 610, 570, 530, 430, 375.

Spectre de RMN du proton (250 MHz, DMSO $d_6$, δ en ppm, J en Hz) 1,47 et 1,50 [2S, 6H, $>C(CH_3)_2$]; 3,75 et 3,90 (2D, J = 17,5, 2H, $-S-CH_2-$); 4,30 (S large, 3H, $\geqslant \overset{+}{N}CH_3$); 5,23 (D, J = 4, 1H, −H en 6); 5,78 (DD, J = 4 et 8, 1H, −H en 7); 6,76 (S, 1H, −H en 5 du thiazole); 7,26 (S large, 2H, $-NH_2$); 7,50 (S, 1H, −H en 5 du thiazole); 7,83 (Mf, 1H, H en 5 du pyridinio); 8,35 (D, 1H, H en 6 du pyridinio); 9,02 (D, 1H, H en 4

du pyridinio); 9,17 (S large, 1 H, –H en 2 du pyridinio).

*Exemple 13:*

On traite 3,03 g de benzhydryloxycarbonyl-2 [méthoxyimino-2 (tritylamino-2 thiazolyl-4)-2 acétamido]-7 oxo-8 oxyde-5 (nicotinoylamino-2 thiazolyl-5)-3 thia-5 aza-1 bicyclo-[4.2.0]-octène-2, isomère syn par 0,25 $cm^3$ d'iodure de méthyle dans 10 $cm^3$ de diméthylformamide selon le mode opératoire décrit dans l'exemple 8 pour obtenir 3 g d'iodure de benzhydryloxycarbonyl-2 [méthoxyimino-2 (tritylamino-2 thiazolyl-4)-2 acétamido]-7 [(méthyl-1 pyridinio-3 carbonylamino)-2 thiazolyl-5]-3 oxo-8 oxyde-5 thia-5 aza-1 bicyclo-[4.2.0]-octène-2, isomère syn sous la forme d'un solide crème.

Spectre infrarouge (KBr, bandes caractéristiques en $cm^{-1}$) 1800, 1730, 1670, 1510, 1450, 1035, 755, 700, 670.

Spectre de RMN du proton (350 MHz, DMSO $d_6$, δ en ppm, J en Hz) 3,80 et 4,25 (2d, J = 18,5, 2H, $-SOCH_2-$); 3,86 (s, 3H, $=NOCH_3$); 4,47 (s, 3H, $\geqslant N^+-CH_3$); 5,08 (d, J = 3,5, 1H, –H en 6); 5,91 (dd, J = 3,5 et 9, 1H, –H en 7); 6,79 (s, 1H, –H en 5 du thiazole); 6,89 (s, 1H, $-COOCHAr_2$); 7,05 à 7,45 (Mf, 25H, aromatiques); 7,49 (s, 1H, –H en 4 du thiazole); 8,30 (dd, J = 6 et 7, 1H, –H en 5 du pyridinio); 8,73 [s, 1H, $-NHC(C_6H_5)_3$]; 9,06 (d, J = 7, 1H, –H en 4 du pyridinio); 9,10 (d, J = 9, $-CONH-C_7$); 9,17 (d, J = 6, 1H, H en 6 du pyridinio); 9,57 (s, 1H, –H en 2 du pyridinio).

3 g d'iodure de benzhydryloxycarbonyl-2 [méthoxyimino-2 (tritylamino-2 thiazolyl-4)-2 acétamido]-7 [(méthyl-1 pyridinio-3 carbonylamino)-2 thiazolyl-5]-3 oxo-8 oxyde-5 thia-5 aza-1 bicyclo-[4.2.0]-octène-2, isomère syn sont réduits par 0,5 $cm^3$ de trichlorure de phosphore dans un mélange de 50 $cm^3$ de dichlorométhane et de 5 $cm^3$ de N,N-diméthylacétamide selon le mode opératoire décrit dans l'exemple 1 pour donner 3 g d'iodure de benzhydryloxycarbonyl-2 [méthoxyimino-2 (tritylamino-2 thiazolyl-4)-2 acétamido]-7 [(méthyl-1 pyridinio-3 carbonylamino)-2 thiazolyl-5]-3 oxo-8 thia-5 aza-1 bicyclo-[4.2.0]-octène-2, isomère syn brut sous la forme d'un solide jaune crème.

Spectre de RMN du proton (350 MHz, DMSO $d_6$, δ en ppm, J en Hz) 3,90 (Mf, 5H, $=N OCH_3$ et $-SCH_2-$); 4,48 (s, 3H, $\geqslant N^+-CH_3$); 5,27 (d, J = 4, 1H, –H en 6); 5,85 (dd, J = 4 et 7, 1H, –H en 7); 6,81 (s, 1H, –H en 5 du thiazole); 6,85 (s, 1H, $-COO-CHAr_2$); 7,05 à 7,50 (Mf, 25H, aromatiques); 7,52 (s, 1H, –H en 4 du thiazole); 8,3 (dd, J = 5 et 8, –H en 5 du pyridinio); 9,09 (d, J = 8, 1H, –H en 4 du pyridinio); 9,21 (d, J = 5, 1H, –H en 6 du pyridinio); 9,60 (s, 1H, –H en 2 du pyridinio); 9,75 (d, J = 7, 1H, $-CONH-C_7$).

3 g d'iodure de benzhydryloxycarbonyl-2 [méthoxyimino-2 (tritylamino-2 thiazolyl-4)-2 acétamido]-7 [(méthyl-1 pyridinio-3 carbonylamino)-2 thiazolyl-5]-3 oxo-8 thia-5 aza-1 bicyclo-[4.2.0]-octène-2, isomère syn brut sont traités par un mélange de 60 $cm^3$ d'acide formique et de 7 $cm^3$ d'anisole selon le mode opératoire décrit dans l'exemple 8 pour donner 2,1 g d'iodhydrate d'[(amino-2 thiazolyl-4)-2 méthoxyimino-2 acétamido]-7 carboxylato-2 [(méthyl-1 pyridinio-3 carbonylamino)-2 thiazolyl-5]-3 oxo-8 thia-5 aza-1 bicyclo-[4.2.0]-octène-2, isomère syn brut sous la forme d'un solide crème que l'on reprend par un mélange de 210 $cm^3$ d'eau distillée et de 100 $cm^3$ d'acétate d'éthyle; après filtration et lavage de l'insoluble 4 fois par 25 $cm^3$ d'eau, la phase aqueuse est décantée et traitée par 40 $cm^3$ de résine IR 45 (basique) jusqu'à atteindre un pH de 4,6, puis elle est filtrée et lyophilisée pour donner 0,7 g d'[(amino-2 thiazolyl-4)-2 méthoxyimino-2 acétamido]-7 carboxylato-2 [(méthyl-1 pyridinio-3 carbonylamino)-2 thiazolyl-5]-3 oxo-8 thia-5 aza-1 bicyclo-[4.2.0]-octène-2, isomère syn, sous la forme d'un lyophilisat jaune.

Spectre infrarouge (KBr, bandes caractéristiques en $cm^{-1}$) 3600-2400, 1765, 1670, 1610, 1530, 1390, 1040, 675.

Spectre de RMN du proton (250 MHz, DMSO $d_6$, δ en ppm, J en Hz) 3,85 (s, 3H, $=NOCH_3$); 4,42 (s, 3H, $\geqslant N^+-CH_3$); 5,17 (d, J = 4, 1H, –H en 6); 5,74 (dd, J = 4 et 7,5, 1H, –H en 7); 6,75 (s, 1H, –H en 5 du thiazole); 7,24 (s large, 2H, $-NH_2$); 7,51 (s, 1H, –H en 4 du thiazole); 8,12 (Mf, 1H), 9,01 (Mf, 2H) et 9,50 (s, H: protons du pyridinio); 9,64 (d, J = 7,5, 1H, $-CONH-C_7$).

Le benzhydryloxycarbonyl-2 [méthoxyimino-2 (tritylamino-2 thiazolyl-4)-2 acétamido]-7 oxo-8 oxyde-5 (nicotinoylamino-2 thiazolyl-5]-3 thia-5 aza-1 bicyclo-[4.2.0]-octène-2, isomère syn peut être préparé de la manières suivante.

A une solution refroidie à +5° C de 2,8 g d'amino-7 benzhydryloxycarbonyl-2 oxo-8 oxyde-5 (nicotinoylamino-2 thiazolyl-5)-3 thia-5 ata-1 bicyclo-[4.2.0]-octène-2, de 2,66 g d'acide méthoxyimino-2 (tritylamino-2 thiazolyl-4)-2 acétique isomère syn et de 0,82 g d'hydroxy-1 benzotriazole dans 20 $cm^3$ de N,N-diméthylformamide on ajoute 1,24 g de N,N'-dicyclohexylcarbodiimide puis agite le mélange réactionnel 1 h à +5° C puis 64 h à 20° C; après filtration le mélange réactionnel est dilué par 200 $cm^3$ de chloroforme et lavé par 100 $cm^3$ de solution à 5% de bicarbonate de sodium, par 100 $cm^3$ d'eau puis par 100 $cm^3$ de solution saturée de chlorure de sodium. Après séchage sur sulfate de magnésium, la phase organique est concentrée à sec sous pression réduite (30 mm de mercure; 4 kPa) à 30° C pour donner 5 g de produit brut. 10 g de ce produit brut sont chromatographiés sur une colonne de 250 $cm^3$ de gel de silice (0,2-0,06 mm) en éluant successivement par 1 l de mélange de dichlorométhane et d'acétate d'éthyle (75/25 en volumes) puis 1 l de mélange de dichlorométhane et d'acétate d'éthyle (50/50 en volumes) puis par 2 l d'acétate d'éthyle et par 1 l de mélange d'acétate d'éthyle et de méthanol (80/20 en volumes) et en recueillant des fractions de 100 $cm^3$. Les fractions 19 à 33 sont réunies et concentrées à sec sous pression réduite (30 mm de mercure; 4 kPa) à 30° C pour donner 6,2 g de benzhydryloxycarbonyl-2 [(méthoxyimino)-2 (tritylamino-2 thiazolyl-4)-2 acétamido]-7 oxo-8 oxyde-5

(nicotinoylamino-2 thiazolyl-5)-3 thia-5 aza-1 bicyclo-[4.2.0]-octène-2, isomère syn sous la forme d'un solide orangé.

Spectre infrarouge (KBr, bandes caractéristiques en $cm^{-1}$) 3250, 1800, 1730, 1675, 1590, 1540, 1515, 1450, 1040, 755, 740, 700.

Spectre de RMN du proton (350 MHz, DMSO $d_6$, δ en ppm, J en Hz) 3,79 et 4,23 (2d, J = 19, 2H, $-SOCH_2-$); 3,86 (s, 3H, $=NOCH_3$); 5,07 (d, J = 4, 1H, H en 6); 5,89 (dd, J = 4 et 9, 1H, H en 7); 6,79 (s, 1H, H en 5 du thiazole); 6,88 (s, 1H, $-CO_2C\underline{H}Ar_2$); 7,0 à 7,50 (Mf, 25H, aromatiques); 7,42 (s, 1H, H en 4 du thiazole); 7,57 (dd, J = 5 et 8, 1H, H en 5 de la pyridine); 8,40 (m, 1H, H en 4 de la pyridine); 8,72 [s, 1H, $-NH-C(C_6H_5)_3$]; 8,79 (dd, J = 1 et 5, H en 6 de la pyridine); 9,04 (d, J = 9, $CONH-C_7$); 9,19 (d, J = 1, 1H, H en 2 de la pyridine); 12,87 (s large, 1H, $-NHCO-$).

L'amino-7 benzhydryloxycarbonyl-2 oxo-8 oxyde-5 (nicotinoylamino-2 thiazolyl-5)-3 thia-5 aza-1 bicyclo-[4.2.0]-octène-2 peut être préparé de la manière suivante.

Une solution de 9 g de benzhydryloxycarbonyl-2 t-butoxycarbonylamino-7 oxo-8 oxyde-5 (nicotinoylamino-2 thiazolyl-5)-3 thia-5 aza-1 bicyclo-[4.2.0]-octène-2 dans 90 $cm^3$ d'acétonitrile est traitée par 9 $cm^3$ d'acide méthanesulfonique pendant 8 min à 25° C puis versée dans 300 $cm^3$ de solution à 5% de bicarbonate de sodium. Le précipité est essoré, lavé 5 fois par 20 $cm^3$ d'eau distillée puis 3 fois par 20 $cm^3$ d'éther éthylique et séché. On obtient 6,55 g d'amino-7 benzhydryloxycarbonyl-2 oxo-8 oxyde-5 (nicotinoylamino-2 thiazolyl-5)-3 thia-5 aza-1 bicyclo-[4.2.0]-octène-2 sous la forme d'un solide crème.

Spectre infrarouge (KBr, bandes caractéristiques en $cm^{-1}$) 3410, 1780, 1730, 1670, 1590, 1550, 1020, 895, 705.

Spectre de RMN du proton (250 MHz, DMSO $d_6$, δ en ppm, J en Hz) 3,76 et 4,30 (2d, J = 17,5, 2H, $-SOCH_2-$); 4,87 et 4,96 (2d, J = 4, 2 × 1H, −H en 6 et 7); 6,89 (s, 1H, $-CO_2C\underline{H}Ar_2$); 7,0 à 7,4 (mf, 10H, aromatiques); 7,46 (s, 1H, −H en 4 du thiazole); 7,6 (dd, J = 9 et 5, 1H, −H en 5 de la pyridine); 8,42 (d, J = 9, 1H, −H en 4 de la pyridine); 8,80 (dd, J = 5 et 1, 1H, −H en 6 de la pyridine); 9,21 (d, J = 1, 1H, −H en 2 de la pyridine).

Le benzhydryloxycarbonyl-2 t-butoxycarbonylamino-7 oxo-8 oxyde-5 (nicotinoylamino-2 thiazolyl-5)-3 thia-5 aza-1 bicyclo-[4.2.0]-octène-2 peut être préparé de la manière suivante.

On obtient 18,5 g de benzhydryloxycarbonyl-2 t-butoxycarbonylamino-7 oxo-8 oxyde-5 (nicotinoylamino-2 thiazolyl-5)-3 thia-5 aza-1 bicyclo-[4.2.0]-octène-2 par oxydation de 18,4 g de sulfure correspondant par action à −10° C de 5,6 q d'acide métachloroperbenzoïque à 85% dans un mélange de 250 $cm^3$ de méthanol et de 870 $cm^3$ de chloroforme pendant 10 min, suivie de lavages du mélange réactionnel par un mélange de 250 $cm^3$ d'eau distillée et de 500 $cm^3$ de solution saturée de bicarbonate de sodium puis par 500 $cm^3$ d'eau distillée et enfin concentration à sec de la phase organique sous pression réduite (30 mm de mercure; 4 kPa) à 30° C et concrétion du solide obtenu par de l'éther éthylique.

Spectre de RMN du proton (250 MHz, DMSO $d_6$, δ en ppm, J en Hz) 1,44 [s, 9H, $(CH_3)_3C-$]; 3,82 et 4,47 (2d, J = 18, 2H, $-SOCH_2-$); 5,07 (d, J = 5, 1H, −H en 6); 5,86 (dd, J = 5 et 10, 1H H en 7); 6,47 (d, J = 10, 1H, $-CONHC_7$); 6,90 (s, 1H, $-CO_2C\underline{H}Ar_2$); 7,05 à 7,4 (mf, 10H, aromatiques); 7,51 (s, 1H, −H du thiazole); 7,62 (dd, J = 5 et 8, 1H, −H en 5 de la pyridine); 8,42 (ddd, J = 8, 2 et 1,5, 1H, H en 4 de la pyridine); 8,81 (dd, J = 5 et 1,5, 1H, −H en 6 de la pyridine); 9,2 (d, J = 2, 1H, −H en 2 de la pyridine); 12,92 (s large, 1H, $-NHCO-$).

Le benzhydryloxycarbonyl-2 t-butoxycarbonylamino-7 (nicotinoylamino-2 thiazolyl-5)-3 oxo-8 thia-5 aza-1 bicyclo-[4.2.0]-octène-2 peut être préparé de la manière suivante.

On ajoute à une solution de 11,3 g d'(amino-2 thiazolyl-5)-3 benzhydryloxycarbonyl-2 t-butoxycarbonylamino-7 oxo-8 thia-5 aza-1 bicyclo-[4.2.0]-octène-2 dans 300 $cm^3$ de tétrahydrafuranne refroidie à 3° C, 7,12 g de chlorhydrate de chlorure de nicotinoyle, puis en 10 min, 11,12 $cm^3$ de triéthylamine. On agite 1 h à 3° C, puis 2 h en laissant remonter la température à 20° C. On verse le mélange réactionnel dans 400 $cm^3$ d'acétate d'éthyle et lave 3 fois par 120 $cm^3$ d'eau distillée et 100 $cm^3$ de solution saturée de chlorure de sodium. La phase organique est séchée et concentrée à sec sous pression réduite (30 mm de mercure; 4 kPa) à 40° C. Le résidu est cristallisé dans 20 $cm^3$ d'acétate d'éthyle pour donner 7,4 g de benzhydryloxycarbonyl-2 t-butoxycarbonylamino-7 (nicotinoylamino-2 thiazolyl-5)-3 oxo-8 thia-5 aza-1 bicyclo-[4.2.0]-octène-2 sous la forme d'un solide jaune.

Rf = 0,24 (chromatoplaque de gel de silice, éluant: mélange de cyclohexane et d'acétate d'éthyle 20/80 en volumes).

*Exemple 14:*

Une suspension refroidie à −10° C de 2,46 g de chlorhydrate d'amino-7 carboxylato-2 [(méthyl-1 pyridinio-3)-2 thiazolyl-5]-3 oxo-8 thia-5 aza-1 bicyclo-[4.2.0]-octène-2, isomère syn dans un mélange de 30 $cm^3$ d'acétonitrile et de 30 $cm^3$ de N,N-diméthylacétamide contenant 3,9 $cm^3$ de triéthylamine, on ajoute en 10 min une solution de chlorure de l'acide (t-butoxycarbonyl-2 propyl-2 oxyimino)-2 (tritylamino-2 thiazolyl-4)-2 acétique dans du dichlorométhane (solution préparée selon le mode opératoire décrit dans le brevet belge N° 876538 à partir de 3,44 g de l'acide correspondant). Le mélange réactionnel est agité pendant 1 h entre 0 et −10° C puis pendant 1 h à 20° C. On ajoute 1 $cm^3$ de méthanol puis évapore le dichlorométhane sous pression réduite (30 mm de mercure; 4 kPa) à 30° C. La solution résiduelle est diluée par 300 $cm^3$ d'eau. Le précipité est essoré, lavé par 50 $cm^3$ d'eau distillée puis 2 fois par 50 $cm^3$ d'éther et redissous dans 80 $cm^3$ d'acétate d'éthyle. La solution est filtrée, diluée par 400 $cm^3$ d'éther éthylique. Le précipité est essoré, lavé par

de l'éther éthylique 2 fois 50 $cm^3$ et séché pour donner 3,1 g de chlorhydrate de [(t-butoxycarbonyl-2 propyl-2 oxyimino)-2 (tritylamino-2 thiazolyl-4)-2 acétamido]-7 carboxylato-2 [(méthyl-1 pyridinio-3)-2 thiazolyl-5]-3 oxo-8 thia-5 aza-1 bicyclo-[4.2.0]-octène-2, isomère syn sous la forme d'un solide jaune.

Spectre infrarouge (KBr, bandes caractéristiques en $cm^{-1}$) 1775, 1730, 1680, 1615, 1580, 1150.

Spectre de RMN du proton (350 MHz, DMSO $d_6$, δ en ppm, J en Hz) 1,42 [Mf, 16H, $-C(CH_3)_2CO_2C(CH_3)_3$]; 3,80 (AB, 2H, $-SCH_2-$); 4,43 (s, 3H, $\geqslant N^+-CH_3$); 5,16 (d, J = 5, 1H, −H en 6); 5,63 (dd, J = 5 et 8, 1H, −H en 7); 6,78 (s, 1H, −H en 4 du thiazole); 7,10 à 7,50 (Mf, 15H, aromatiques); 8,13 (mf, 2H, −H en 5 du thiazole et −H en 5 du pyridinio); 8,79 (s, 1H, −NH− thiazole); 8,88 (d, J = 7, 1H, −H en 4 du pyridinio); 8,97 (mf, 1H, −H en 6 du pyridinio); 9,35 (d, J = 8, 1H, −CONH−); 9,51 (s, 1H, −H en 2 du pyridinio).

3 g de chlorhydrate de [(t-butoxycarbonyl-2 propyl-2 oxyimino)-2 (tritylamino-2 thiazolyl-4)-2 acétamido]-7 carboxylato-2 [(méthyl-1 pyridinio-3)-2 thiazolyl-5]-3 oxo-8 thia-5 aza-1 bicyclo-[4.2.0]-octène-2, isomère syn sont agités pendant 1 h à 25° C avec 20 $cm^3$ d'acide formique contenant 1,2 $cm^3$ d'acide chlorhydrique concentré. L'insoluble est éliminé par filtration et lavé 2 fois par 0,5 $cm^3$ d'acide formique. Les filtrats rassemblés sont concentrés à sec sous pression réduite (0,3 mm de mercure; 0,04 kPa) à 30° C. Le résidu est repris par 40 $cm^3$ d'éthanol que l'on évapore à sec sous pression réduite (30 mm de mercure; 4 kPa) à 30° C. Cette opération est répétée une autre fois, puis le résidu est repris dans 50 $cm^3$ d'éthanol. Le solide est filtré, lavé 4 fois par 20 $cm^3$ d'éthanol, 2 fois par 20 $cm^3$ d'acétone et 2 fois par 40 $cm^3$ d'éther éthylique et séché pour donner 1,4 g de chlorhydrate d'[(amino-2 thiazolyl-4)-2 (carboxy-2 propyl-2 oxyimino)-2 acétamido]-7 carboxylato-2 [(méthyl-1 pyridinio-3)-2 thiazolyl-5]-3 oxo-8 thia-5 aza-1 bicyclo-[4.2.0]-octène-2, isomère syn, sous la forme d'un solide jaune dont les caractéristiques sont semblables à celles du produit décrit dans l'exemple 2.

Le chlorhydrate d'amino-7 carboxylato-2 [(méthyl-1 pyridinio-3)-2 thiazolyl-5]-3 oxo-8 thia-5 aza-1 bicyclo-[4.2.0]-octène-2 peut être obtenu de la manière suivante.

54 g d'iodure de benzhydryloxycarbonyl-2 t-butoxycarbonylamino-7 [(méthyl-1 pyridinio-3)-2 thiazolyl-5]-3 oxo-8 thia-5 aza-1 bicyclo-[4.2.0]-octène-2 sont traités par 500 $cm^3$ d'acide trifluoracétique à 25° C pendant 40 min. Le mélange réactionnel est concentré à sec sous pression réduite (2 mm de mercure; 0,27 kPa) à 35° C. Le résidu est repris par 100 $cm^3$ d'éthanol que l'on évapore sous pression réduite (30 mm de mercure; 4 kPa) à 35° C. Cette opération est répétée encore 2 fois puis le produit est concrété par 100 $cm^3$ d'éthanol, essoré, lavé 4 fois par 50 $cm^3$ d'éther éthylique puis repris dans 100 $cm^3$ d'acide chlorhydrique normal à 35° C. La solution est filtrée, diluée par 100 $cm^3$ d'isopropanol et portée à 4° C pendant 16 h. Les cristaux formés sont essorés, lavés par 20 $cm^3$ d'isopropanol puis 3 fois par 50 $cm^3$ d'éther éthyliquer pour donner 12 g de chlorhydrate d'amino-7 carboxylato-2 [(méthyl-1 pyridinio-3)-2 thiazolyl-5]-3 oxo-8 thia-5 aza-1 bicyclo-[4.2.0]-octène-2 sous la forme de cristaux jaunes.

Spectre de RMN du proton (250 MHz, DMSO $d_6$, δ en ppm, J en Hz) 3,88 et 4,0 (2d, J = 18, 2H, $-SCH_2-$); 4,43 (s, 3H, $\geqslant N^+-CH_3$); 4,97 (d, J = 5, 1H, −H en 7); 5,17 (d, J = 5, 1H, −H en 6); 8,17 (s, 1H, −H du thiazole); 8,21 (dd, J = 8 et 6, 1H, −H en 5 du pyridinio); 8,99 (d, J = 8, 1H, −H en 4 du pyridinio); 9,04 (d, J = 5, −H en 6 du pyridinio); 9,6 (s, 1H, −H en 2 du pyridinio).

L'iodure de benzhydryloxycarbonyl-2 t-butoxycarbonylamino-7 [(méthyl-1 pyridinio-3)-2 thiazolyl-5]-3 oxo-8 thia-5 aza-1 bicyclo-[4.2.0]-octène-2 peut être obtenu de la manière suivante.

Une solution de 50 g de benzhydryloxycarbonyl-2 t-butoxycarbonylamino-7 oxo-8 [(pyridyl-3)-2 thiazolyl-5]-3 thia-5 aza-1 bicyclo-[4.2.0]-octène-2 dans 200 $cm^3$ de N,N-diméthylformamide est traitée par 6,5 $cm^3$ d'iodure de méthyle selon le mode opératoire décrit dans l'exemple 2 pour donner 54,7 g d'iodure de benzhydryloxycarbonyl-2 t-butoxycarbonylamino-7 [(méthyl-1 pyridinio-3)-2 thiazolyl-5]-3 oxo-8 thia-5 aza-1 bicyclo-[4.2.0]-octène-2 sous la forme d'un solide jaune orangé.

Spectre de RMN du proton (250 MHz, DMSO $d_6$, δ en ppm, J en Hz) 1,46 [s, 9H, $(CH_3)_3C-$]; 3,43 et 4,08 (2d, J = 17,5, 2H, $-SCH_2-$); 4,50 (s, 3H, $\geqslant N^+-CH_3$); 5,27 (d, J = 5, 1H, −H en 6); 5,72 (dd, J = 5 et 9, 1H, −H en 7); 6,93 (s, 1H, $-CO_2CHAr_2$); 6,95 à 7,5 (mf, 10H, aromatiques); 8,1 (s, 1H, −H du thiazole); 8,16 (d, J = 9, 1H, −CONH−); 8,26 (2d, J = 6 et 8, 1H, −H en 5 du pyridinio); 8,74 (d, J = 8, 1H, −H en 4 du pyridinio); 9,08 (d, J = 6, 1H, −H en 6 du pyridinio); 9,42 (s, 1H, −H en 2 du pyridinio).

La présente invention concerne également les médicaments qui contiennent comme produit actif au moins un produit de formule générale I à l'état pur (sous forme libre ou sous forme de sel) ou sous forme d'une composition en association avec un ou plusieurs adjuvants pharmaceutiquement acceptables. Ces médicaments peuvent être utilisés par voie orale, parentérale (notamment intramusculaire ou intraveineuse) ou rectale.

Comme compositions solides pour administration orale peuvent être utilisés des comprimés, des pilules, des poudres (généralement présentées dans des capsules, par exemple en gélatine) ou des granulés. Dans ces compositions, le produit actif selon l'invention est mélangé à un ou plusieurs diluants ou adjuvants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration parentérale peuvent être des solutions stériles aqueuses ou non aqueuses, des suspensions ou des émulsions. Comme solvant ou véhicule, on peut employer le propylèneglycol, un polyéthylèneglycol, des huiles végétales, en particulier l'huile d'olive et des esters organiques injectables, par exemple l'oléate d'éthyle. Ces compositions peuvent également contenir des adjuvants, en particulier des agents mouillants, émulsifiants ou dispersants. La stérilisation peut se faire de plusieurs façons, par exemple à l'aide d'un filtre bactériologique, en incorporant à la composition des agents stérilisants, par irradiation ou par chauffage. Elles peuvent être également préparées sous forme de compositions solides stériles qui seront dissoutes au moment de l'emploi dans de l'eau stérile ou tout autre milieu stérile injectable.

Les compositions pour administration rectale sont des suppositoires qui peuvent contenir, outre le produit actif, des excipients tels que le beurre de cacao ou des glycérides semi-synthétiques.

En thérapeutique humaine, les médicaments selon la présente invention sont particulièrement utiles dans le traitement des infections d'origine bactérienne.

D'une façon générale, le médecin déterminera la posologie qu'il estime la plus appropriée en fonction de l'âge, du poids, du degré de l'infection et des autres facteurs propres au sujet à traiter. Généralement, les doses sont comprises entre 1 et 10 g par jour de produit actif par voie intramusculaire pour un adulte.

Les exemples suivants, donnés à titre non limitatif, illustrent une composition selon la présente invention.

*Exemple A:*

On prépare 100 cm³ d'une solution aqueuse isotonique contenant 1,40 g de bicarbonate de sodium et, comme produit actif, 10 g d'[(amino-2 thiazolyl-4)-2 méthoxyimino-2 acétamido]-7 carboxylato-2 [(carboxyméthyl-1 pyridinio-3)-2 thiazolyl-5]-3 oxo-8 thia-5 aza-1 bicyclo-[4.2.0]-octène-2, isomère syn. Après filtration sur filtre bactériologique, on répartit aseptiquement cette solution en ampoules (à raison de 10 cm³ par ampoule), on lyophilise et on scelle les ampoules.

Chaque ampoule contient l'équivalent de 1 g du produit actif, sous forme de son sel sodique.

*Exemple B:*

On prépare 100 cm³ d'une solution aqueuse isotonique contenant 1,33 g de bicarbonate de sodium et, comme produit actif, 10 g d'[(amino-2 thiazolyl-4)-2 (carboxy-2 propyl-2 oxyimino)-2 acétamido]-7 carboxylato-2 [(méthyl-1 pyridinio-3 amino)-2 thiazolyl-5]-3 oxo-8 thia-5 aza-1 bicyclo-[4.2.0]-octène-2, isomère syn. Après filtration sur filtre bactériologique, on répartit aseptiquement cette solution en ampoules (à raison de 10 cm³ par ampoule), on lyophilise et on scelle les ampoules.

Chaque ampoule contient l'équivalent de 1 g du produit actif, sous forme de son sel sodique.

**Revendications** pour les Etats contractants BE, CH, DE, FR, GB, IT, LI, LU, NL, SE

1. Un dérivé de la céphalosporine, caractérisé en ce qu'il répond à la formule générale:

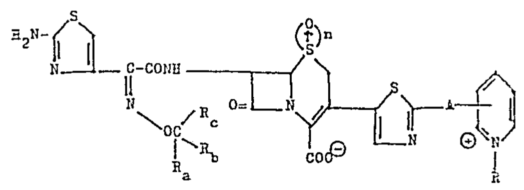

dans laquelle:

le symbole A représente une liaison simple ou un radical bivalent choisi parmi $-CH_2-$, $-NH-$ ou $-NHCO-$ fixé en position -3 ou -4 du radical pyridinio, le symbole R représente un radical méthyle, carboxyméthyle, carbamoylméthyle, benzyle ou allyle,

les symboles $R_a$, $R_b$ et $R_c$ représentent chacun un atome d'hydrogène, ou bien le symbole $R_a$ représente un radical carboxy et les symboles $R_b$ et $R_c$ sont identiques ou différentes et représentent des atomes d'hydrogène ou des radicaux alcoyle contenant 1 à 4 atomes de carbone, ou forment ensemble un radical alcoylène contenant 2 à 5 atomes de carbone et n est égal à 0 ou 1, sous sa forme syn, ainsi que ses sels d'addition avec les acides, et ses sels métalliques ou ses sels d'addition avec les bases azotées lorsqu'ils existent.

2. Un dérivé de la céphalosporine selon la revendication 1, caractérisé en ce que le symbole A représente une liaison simple ou un radical bivalent choisi parmi $-CH_2-$, $-NH-$ ou $-NHCO-$ fixé en position -3 ou -4 du radical pyridinio, le symbole R représente un radical méthyle, carboxyméthyle, carbamoylméthyle ou benzyle, les symboles $R_a$, $R_b$ et $R_c$ représentent chacun un atome d'hydrogène, ou bien le symbole $R_a$ représente un radical carboxy et les symboles $R_b$ et $R_c$ représentent des atomes d'hydrogène ou des radicaux méthyle, et n est égal à 0 ou 1, ainsi que ses sels d'addition avec les acides et ses sels métalliques ou ses sels d'addition avec les bases azotées lorsqu'ils existent.

3. Un dérivé de la céphalosporine selon la revendication 1, caractérisé en ce que le symbole A est une liaison simple ou un radical $-NH-$, fixé en position -3 du radical pyridinio, le symbole R représente un radical méthyle, carboxyméthyle ou carbamoylméthyle, les symboles $R_a$, $R_b$ et $R_c$ représentent des atomes d'hydrogène, ou bien le symbole $R_a$ représente un radical carboxy et les symboles $R_b$ et $R_c$ représentent des atomes d'hydrogène ou des radicaux méthyle et n égale 0, ainsi que ses sels d'addition avec les acides et ses sels métalliques ou ses sels d'addition avec les bases azotées lorsqu'ils existent.

4. Un procédé de préparation d'un produit selon la revendication 1, caractérisé en ce que l'on fait agir un acide de formule générale:

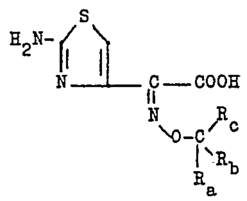

dans laquelle:

$R_a$, $R_b$ et $R_c$ sont définis comme dans la revendication 1, et dont la fonction amine et le cas échéant le radical carboxy représenté par $R_a$ sont préalablement protégés, ou un dérivé réactif de cet acide sur une amino-7 céphalosporine ou un de ses sels, de formule générale:

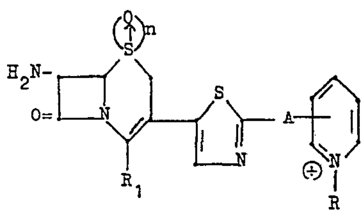

dans laquelle:

les symboles A et n sont définis comme dans la revendication 1, le symbole R est défini comme dans la revendication 1 ou représente un radical carboxyméthyle protégé et le symbole $R_1$ est un radical carboxylato ou un radical carboxy libre ou protégé (étant entendu que lorsque $R_1$ est autre que le radical carboxylato, le produit se trouve à l'état d'halogénure ou de sulfonate), puis on réduit éventuellement le sulfoxyde, élimine les radicaux protecteurs et transforme éventuellement le produit obtenu en sel d'addition avec un acide, ou lorsqu'ils existent en sel métallique ou en sel d'addition avec une base azotée.

5. Un procédé de préparation d'un produit selon la revendication 1, caractérisé en ce que l'on fait agir un halogénure de structure R−X dans laquelle R est défini comme dans la revendication 1 ou représente un radical carboxyméthyle protégé, et le symbole X représente un atome d'halogène ou un radical alcoylsulfonyloxy, dont la partie alcoyle qui contient 1 à 4 atomes de carbone peut être substituée par un ou plusieurs atomes d'halogène ou phénylsulfonyoxy, dont le radical phényle peut être éventuellement substitué par un ou plusieurs substituants choisis parmi les atomes d'halogène, ou les radicaux alcoyle ou nitro, sur un dérivé de la céphalosporine de formule générale:

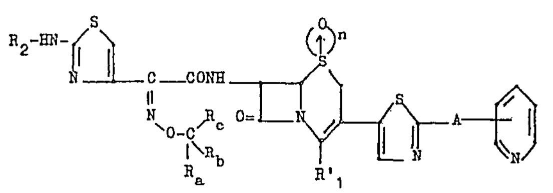

dans laquelle:

A, $R_a$, $R_b$, $R_c$ et n sont définis comme dans la revendication 1 étant entendu que lorsque $R_a$ représente un radical carboxy, il peut être libre ou protégé, le symbole $R'_1$ est un radical carboxy libre ou protégé et le symbole $R_2$ représente un atome d'hydrogène ou un radical protecteur d'amino, puis éventuellement on réduit le sulfoxyde obtenu, élimine les radicaux protecteurs et transforme éventuellement le produit obtenu en un sel d'addition avec un acide, ou lorsqu'ils existent en un sel métallique ou en sel d'addition avec une base azotée.

6. Un procédé de préparation d'un produit selon la revendication 1, caractérisé en ce que l'on fait agir une thio-urée de formule générale:

$$R_2NH-CS-NH_2$$

dans laquelle $R_2$ est un atome d'hydrogène ou un radical protecteur d'amino, sur un dérivé de céphalospórine de formule générale:

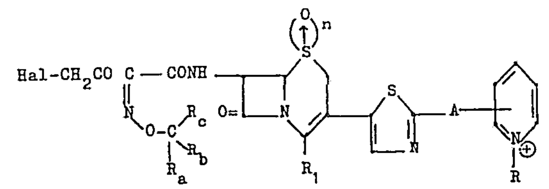

dans laquelle les symboles A, R, $R_1$ et n sont définis comme dans la revendication 4, étant entendu que lorsque $R_1$ est autre que carboxylato, le produit se trouve à l'état d'halogénure ou de sulfonate, les symboles $R_a$, $R_b$ et $R_c$ sont définis comme dans la revendication 1 ou $R_a$ est un radical carboxy protégé et Hal est un atome d'halogène, puis réduit éventuellement le sulfoxyde obtenu, élimine le cas échéant les radicaux protecteurs et transforme éventuellement le produit obtenu en un sel d'addition avec un acide, ou lorsqu'ils existent en un sel métallique ou en sel d'addition avec une base azotée.

7. Un procédé de préparation d'un produit selon la revendication 1, pour lequel A représente un radical −NHCO−, caractérisé en ce que l'on transforme l'amine correspondante de formule générale:

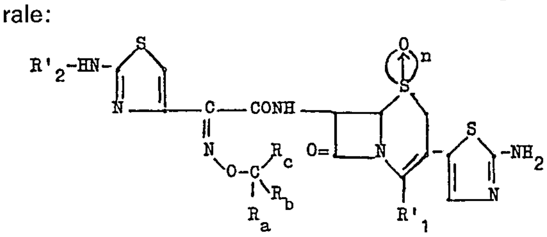

dans laquelle $R_a$, $R_b$, $R_c$, $R'_1$ et n sont définis comme dans la revendication 5 et $R'_2$ représente un radical protecteur d'amino, par toute méthode connue pour former une fonction amide, sans toucher au reste de la molécule, puis réduit éventuellement le sulfoxyde obtenu, élimine les radicaux protecteurs, et transforme éventuellement le produit obtenu en un sel d'addition avec un acide, ou lorsqu'ils existent en un sel métallique ou en sel d'addition avec une base azotée.

8. Composition pharmaceutique, caractérisée en ce qu'elle contient au moins un produit selon la revendication 1, à l'état pur, ou en association avec

un ou plusieurs diluants ou adjuvants compatibles et pharmaceutiquement acceptables.

**Revendication** pour l'Etat contractant: AT

Procédé de préparation d'un dérivé de la céphalosporine, caractérisé en ce qu'il répond à la formule générale:

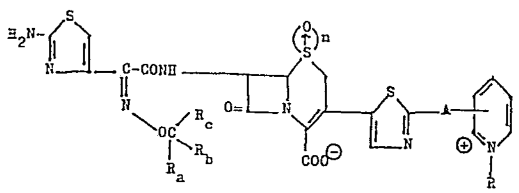

dans laquelle:

le symbole A représente une liaison simple ou un radical bivalent choisi parmi $-CH_2-$, $-NH-$ ou $-NHCO-$ fixé en position -3 ou -4 du radical pyridinio, le symbole R représente un radical méthyle, carboxyméthyle, carbamoylméthyle, benzyle ou allyle,

les symboles $R_a$, $R_b$ et $R_c$ représentent chacun un atome d'hydrogène, ou bien le symbole $R_a$ représente un radical carboxy et les symboles $R_b$ et $R_c$ sont identiques ou différents et représentent des atomes d'hydrogène ou des radicaux alcoyle contenant 1 à 4 atomes de carbone, ou forment ensemble un radical alcoylène contenant 2 à 5 atomes de carbone et n est égal à 0 ou 1, sous sa forme syn, ainsi que ses sels d'addition avec les acides, et ses sels métalliques ou ses sels d'addition avec les bases azotées lorsqu'ils existent, caractérisé en ce que l'on fait agir un acide de formule générale:

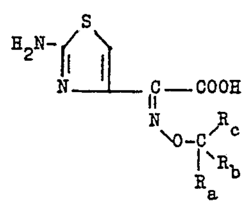

dans laquelle $R_a$, $R_b$ et $R_c$ sont définis comme ci-dessus, et dont la fonction amine et le cas échéant le radical carboxy représenté par $R_a$ sont préalablement protégés, ou un dérivé réactif de cet acide sur une amino-7 céphalosporine ou un de ses sels, de formule générale:

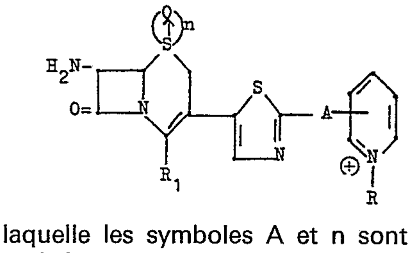

dans laquelle les symboles A et n sont définis comme ci-dessus, le symbole R est défini comme ci-dessus ou représente un radical carboxyméthyle protégé et le symbole $R_1$ est un radical carboxylato ou un radical carboxy libre ou protégé (étant entendu que lorsque $R_1$ est autre que le radical carboxylato, le produit se trouve à l'état d'halogénure ou de sulfonate), puis on réduit éventuellement le sulfoxyde, élimine les radicaux protecteurs et transforme éventuellement le produit obtenu en sel d'addition avec un acide, ou lorsqu'ils existent en sel métallique ou en sel d'addition avec une base azotée, ou bien on fait agir un halogénure de structure $R-X$ dans laquelle R est défini comme ci-dessus ou représente un radical carboxyméthyle protégé, et le symbole X représente un atome d'halogène ou un radical alcoylsulfonyloxy, dont la partie alcoyle qui contient 1 à 4 atomes de carbone peut être susbtituée par un ou plusieurs atomes d'halogène, ou phénylsulfonyloxy, dont le radical phényle peut être éventuellement substitué par un ou plusieurs substituants choisis parmi les atomes d'halogène ou les radicaux alcoyle ou nitro, sur un dérivé de la céphalosporine de formule générale:

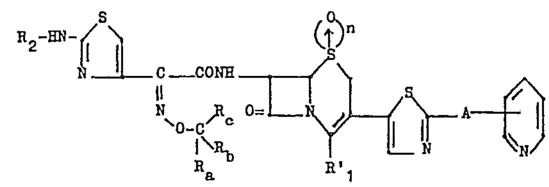

dans laquelle A, $R_a$, $R_b$, $R_c$ et n sont définis comme ci-dessus étant entendu que lorsque $R_a$ représente un radical carboxy, il peut être libre ou protégé, le symbole $R'_1$ est un radical carboxy libre ou protégé et le symbole $R_2$ représente un atome d'hydrogène ou un radical protecteur d'amino, puis éventuellement on réduit le sulfoxyde obtenu, élimine les radicaux protecteurs et transforme éventuellement le produit obtenu en un sel d'addition avec un acide, ou lorsqu'ils existent en un sel métallique ou en sel d'addition avec une base azotée, ou bien on fait agir une thio-urée de formule générale:

$$R_2NH-CS-NH_2$$

dans laquelle $R_2$ est un atome d'hydrogène ou un radical protecteur d'amino, sur un dérivé de céphalosporine de formule générale:

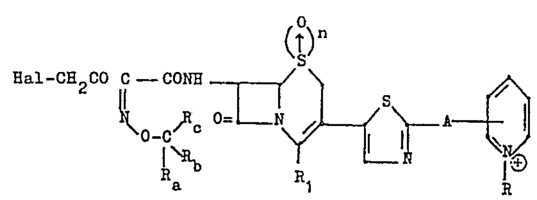

dans laquelle les symboles A, R, $R_1$ et n sont définis comme ci-dessus, étant entendu que lorsque $R_1$ est autre que carboxylato, le produit se trouve à l'état d'halogénure ou de sulfonate, les symboles $R_a$, $R_b$ et $R_c$ sont définis comme ci-dessus ou $R_a$ est un radical carboxy protégé et Hal est un atome d'halogène, puis réduit éventuellement le sulfoxyde obtenu, élimine le cas échéant les radicaux protecteurs et transforme éventuellement le produit obtenu en un sel d'addition avec un acide, ou lorsqu'ils existent en un sel métallique ou en sel d'addition avec une base azotée, ou bien lorsque A représente un radical $-NHCO-$, on transforme l'amine correspondante de formule générale:

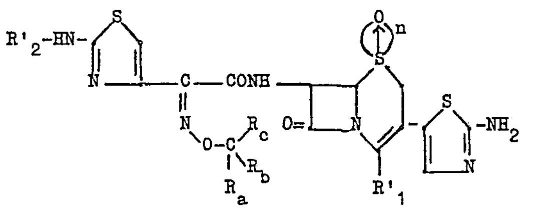

dans laquelle $R_a$, $R_b$, $R_c$, $R'_1$ et n sont définis comme ci-dessus et $R'_2$ représente un radical protecteur d'amino, par toute méthode connue pour former une fonction amide, sans toucher au reste de la molécule, puis réduit éventuellement le sulfoxyde obtenu, élimine les radicaux protecteurs, et transforme éventuellement le produit obtenu en un sel d'addition avec un acide, ou lorsqu'ils existent en un sel métallique ou en sel d'addition avec une base azotée.

**Patentansprüche** für die Vertragsstaaten BE, CH, DE, FR, GB, IT, LI, LU, NL, SE

1. Ein Derivat des Cephalosporins, dadurch gekennzeichnet, dass es der allgemeinen Formel

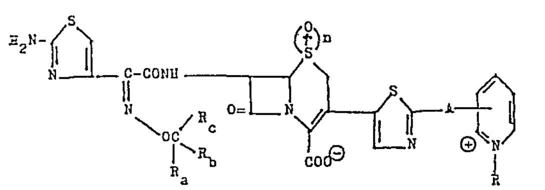

entspricht, worin das Symbol A eine Einfachbindung oder einen zweiwertigen Rest bedeutet, ausgewählt unter $-CH_2-$, $-NH-$ oder $-NHCO-$, der in 3- oder 4-Stellung des Pyridiniorestes steht, das Symbol R einen Methyl-, Carboxymethyl-, Carbamoylmethyl-, Benzyl- oder Allylrest bedeutet, die Symbole $R_a$, $R_b$ und $R_c$ jeweils ein Wasserstoffatom bedeuten, oder aber das Symbol $R_a$ einen Carboxyrest bedeutet, und die Symbole $R_b$ und $R_c$ identisch oder verschieden sind, und Wasserstoffatome oder Alkylreste mit 1 bis 4 Kohlenstoffatomen bedeuten oder zusammen einen Alkylenrest mit 2 bis 5 Kohlenstoffatomen bilden, und n ist 0 oder 1, in seiner syn-Form, sowie seine Additionssalze mit Säuren und seine Metallsalze oder seine Additionssalze mit Stickstoffbasen, falls sie existieren.

2. Ein Derivat des Cephalosporins gemäss Anspruch 1, dadurch gekennzeichnet, dass das Symbol A eine Einfachbindung oder einen zweiwertigen Rest bedeutet, ausgewählt unter $-CH_2-$, $-NH-$ oder $-NHCO-$, der in 3- oder 4-Stellung des Pyridiniorestes fixiert ist, das Symbol R einen Methyl-, Carboxymethyl-, Carbamoylmethyl- oder Benzylrest bedeutet, die Symbole $R_a$, $R_b$ und $R_c$ jeweils ein Wasserstoffatom bedeuten, oder aber das Symbol $R_a$ einen Carboxyrest bedeutet, und die Symbole $R_b$ und $R_c$ Wasserstoffatome oder Methylreste bedeuten, und n = 0 oder 1, sowie seine Additionssalze mit Säuren und seine Metallsalze oder Additionssalze mit Stickstoffbasen, falls sie existieren.

3. Ein Derivat des Cephalosporins gemäss Anspruch 1, dadurch gekennzeichnet, dass das Symbol A eine Einfachbindung oder ein $-NH-$Rest ist, der in 3-Stellung des Pyridiniorestes fixiert ist, das Symbol R einen Methyl-, Carboxymethyl- oder Carbamoylmethylrest bedeutet, die Symbole $R_a$, $R_b$ und $R_c$ Wasserstoffatome bedeuten, oder aber das Symbol $R_a$ einen Carboxyrest bedeutet, und die Symbole $R_b$ und $R_c$ Wasserstoffatome oder Methylreste bedeuten und n = 0, sowie seine Additionssalze mit Säuren und seine Metallsalze oder Additionssalze mit Stickstoffbasen, falls sie existieren.

4. Verfahren zur Herstellung eines Produkts gemäss Anspruch 1, dadurch gekennzeichnet, dass man eine Säure der allgemeinen Formel

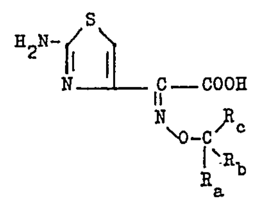

worin $R_a$, $R_b$ und $R_c$ wie in Anspruch 1 definiert sind und dessen Aminfunktion und ggf. der Carboxyrest bei der Bedeutung $R_a$ vorher geschützt wurden, oder ein reaktives Derivat dieser Säure auf ein 7-Aminocephalosporin oder eines seiner Salze der allgemeinen Formel

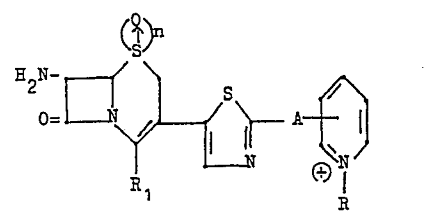

einwirken lässt, worin die Symbole A und n wie in Anspruch 1 definiert sind, das Symbol R wie in Anspruch 1 definiert ist oder einen geschützten Carboxymethylrest bedeutet, und das Symbol $R_1$ ein Carboxylatorest oder ein freier oder geschützter Carboxyrest ist (wobei, falls $R_1$ anders als der Carboxylatorest ist, das Produkt sich im Zustand des Halogenids oder Sulfonats befindet), und dass man dann ggf. das Sulfoxid reduziert, die Schutzgruppen entfernt und das erhaltene Produkt ggf. in ein Additionssalz mit einer Säure oder, falls sie existieren, ein Metallsalz oder ein Additionssalz mit einer Stickstoffbase überführt.

5. Verfahren zur Herstellung eines Produkts gemäss Anspruch 1, dadurch gekennzeichnet, dass man ein Halogenid der Struktur R−X, worin R wie in Anspruch 1 definiert ist oder einen geschützten Carboxymethylrest bedeutet, und das Symbol X ein Halogenatom oder einen Alkylsulfonyloxyrest, dessen Alkylteil, der 1 bis 4 Kohlenstoffatome enthält, ggf. durch ein oder mehrere Halogenatome substituiert sein kann, oder Phenylsulfonyloxy, dessen Phenylrest ggf. durch einen oder mehrere Substituenten, ausgewählt unter den Halogenatomen oder den Alkyl- oder Nitroresten, substituiert sein kann, bedeutet, auf ein Derivat des Cephalosporins der allgemeinen Formel

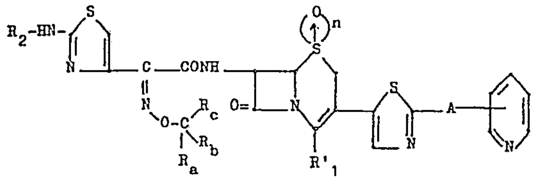

einwirken lässt, worin A, $R_a$, $R_b$, $R_c$ und n wie in Anspruch 1 definiert sind, wobei, falls $R_a$ einen Carboxyrest bedeutet, dieser frei oder geschützt sein kann, das Symbol $R'_1$ ein freier oder geschützter Carboxyrest ist, und das Symbol $R_2$ ein Wasserstoffatom oder einen Aminoschutzrest bedeutet, und dass man dann ggf. das erhaltene Sulfoxid reduziert, die Schutzgruppen entfernt und das erhaltene Produkt ggf. in ein Additionssalz mit einer Säure oder, falls sie existieren, in ein Metallsalz oder ein Additionssalz mit einer Stickstoffbase überführt.

6. Verfahren zur Herstellung eines Produkts gemäss Anspruch 1, dadurch gekennzeichnet, dass man einen Thioharnstoff der allgemeinen Formel

$$R_2NH-CS-NH_2$$

worin $R_2$ ein Wasserstoffatom oder ein Aminoschutzrest ist, auf ein Cephalosporinderivat der allgemeinen Formel

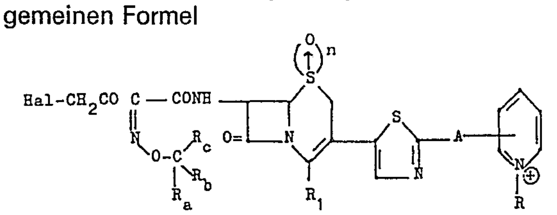

worin die Symbole A, R, $R_1$ und n wie in Anspruch 4 definiert sind, wobei, falls $R_1$ anders als Carboxylato ist, das Produkt sich im Zustand des Halogenids oder Sulfonats befindet, die Symbole $R_a$, $R_b$ und $R_c$ wie in Anspruch 1 definiert sind, oder $R_a$ ein geschützter Carboxyrest und Hal ein Halogenatom ist, einwirken lässt, und dass man dann ggf. das erhaltene Sulfoxid reduziert, ggf. die Schutzgruppen entfernt und das erhaltene Produkt ggf. in ein Additionssalz mit einer Säure oder, falls sie existieren, in ein Metallsalz oder ein Additionssalz mit einer Stickstoffbase überführt.

7. Verfahren zur Herstellung eines Produkts gemäss Anspruch 1, wobei A einen $-NHCO-$Rest bedeutet, dadurch gekennzeichnet, dass man das entsprechende Amin der allgemeinen Formel

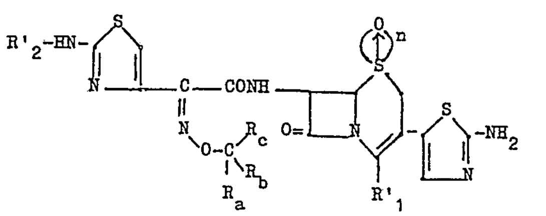

worin $R_a$, $R_b$, $R_c$, $R'_1$ und n wie in Anspruch 5 definiert sind, und $R'_2$ einen Aminoschutzrest bedeutet, nach jeder an sich bekannten Methode zur Bildung einer Amidfunktion, ohne dass der Rest des Moleküls beeinträchtigt wird, überführt, dass man dann ggf. das erhaltene Sulfoxid reduziert, die Schutzgruppen entfernt, und das erhaltene Produkt ggf. in ein Additionssalz mit einer Säure oder, falls sie existieren, in ein Metallsalz oder ein Additionssalz mit einer Stickstoffbase überführt.

8. Pharmazeutische Zusammensetzung, dadurch gekennzeichnet, dass sie mindestens ein Produkt gemäss Anspruch 1 in reinem Zustand oder in Assoziation mit einem oder mehreren verträglichen und pharmazeutisch annehmbaren Verdünnungs- oder Hilfsmitteln enthält.

**Patentaspruch** für den Vertragsstaat: AT

Verfahren zur Herstellung eines Cephalosporinderivats, das dadurch gekennzeichnet ist, dass es der allgemeinen Formel:

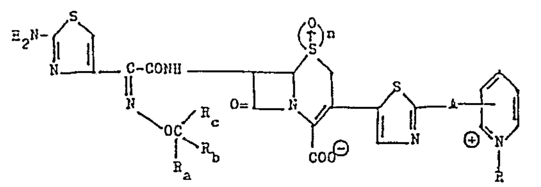

in welcher das Symbol A eine Einfachbindung oder einen zweiwertigen Rest, ausgewählt aus $-CH_2-$, $-NH-$ oder $-NHCO-$ darstellt, die in 3- oder 4-Stellung des Pyridiniorestes gebunden sind, das Symbol R einen Methyl-, Carboxymethyl-, Carbamoylmethyl-, Benzyl- oder Allylrest darstellt, die Symbole $R_a$, $R_b$ und $R_c$ jeweils ein Wasserstoffatom darstellen oder das Symbol $R_a$ einen Carboxyrest darstellt und die Symbole $R_b$ und $R_c$, die gleich oder voneinander verschieden sind, Wasserstoffatome oder Alkylreste mit 1 bis 4 Kohlenstoffatomen darstellen oder zusammen einen Alkylenrest mit 2 bis 5 Kohlenstoffatomen bilden und n gleich 0 oder 1 ist, in der syn-Form entspricht, sowie von seinen Additionssalzen mit Säuren und – falls es sie gibt – seinen Metallsalzen oder seinen Salzen mit Stickstoffbasen, dadurch gekennzeichnet, dass man eine Säure der allgemeinen Formel:

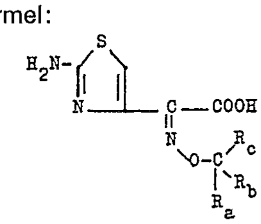

in welcher $R_a$, $R_b$ und $R_c$ die obige Bedeutung haben und deren Aminfunktion und zutreffendenfalls durch $R_a$ dargestellter Carboxyrest zuvor geschützt wurden, oder ein reaktionsfähiges Derivat dieser Säure auf ein 7-Amino-cephalosporin der allgemeinen Formel:

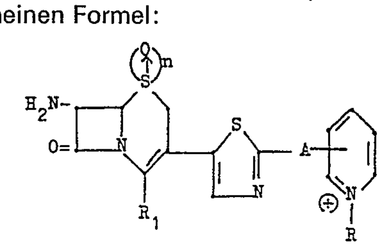

in welcher die Symbole A und n die obige Bedeutung haben, das Symbol R die obige Bedeutung

hat oder einen geschützten Carboxymethylrest darstellt und das Symbol $R_1$ ein Carboxylatrest oder ein freier oder geschützter Carboxyrest ist, oder eines seiner Salze (selbstverständlich befindet sich die Verbindung, wenn $R_1$ vom Carboxylatrest verschieden ist, im Zustand des Halogenids oder Sulfonats) einwirken lässt, dann ggf. das Sulfoxid reduziert, die Schutzgruppen entfernt und ggf. die erhaltene Verbindung in ein Additionssalz mit einer Säure oder, falls es sie gibt, in ein Metallsalz oder ein Additionssalz mit einer Stickstoffbase umwandelt, oder dass man ein Halogenid der Struktur R−X, worin R die obige Bedeutung hat oder einen geschützten Carboxymethylrest darstellt, und das Symbol X ein Halogenatom oder einen Alkylsulfonyloxyrest, dessen Alkylteil, der 1 bis 4 Kohlenstoffatome enthält, durch ein oder mehrere Halogenatome substituiert sein kann, oder Phenylsulfonyloxyrest, dessen Phenylrest ggf. durch einen oder mehrere Substituenten, ausgewählt aus den Halogenatomen oder den Alkyl- oder Nitroresten substituiert sein kann, darstellt, auf ein Cephalosporinderivat der allgemeinen Formel:

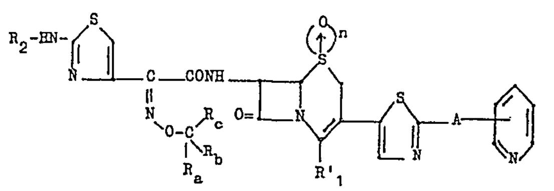

in welcher A, $R_a$, $R_b$, $R_c$ und n die obige Bedeutung haben, wobei ein von $R_a$ dargestellter Carboxyrest selbstverständlich frei oder geschützt sein kann, das Symbol $R'_1$ ein freier oder geschützter Carboxyrest ist und das Symbol $R_2$ ein Wasserstoffatom oder eine Aminoschutzgruppe darstellt, einwirken lässt und dann ggf. das erhaltene Sulfoxid reduziert, die Schutzgruppen entfernt und die erhaltene Verbindung ggf. in ein Additionssalz mit einer Säure oder, falls es diese gibt, in ein Metallsalz oder ein Salz mit einer Stickstoffbase umwandelt, oder dass man einen Thioharnstoff der allgemeinen Formel:

$$R_2NH-CS-NH_2$$

in welcher $R_2$ ein Wasserstoffatom oder eine Aminoschutzgruppe ist, auf eine Cephalosporinverbindung der allgemeinen Formel:

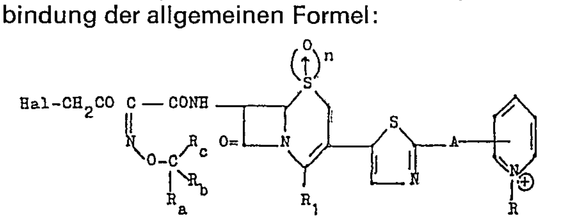

in welcher die Symbole A, R, $R_1$, und n die obige Bedeutung haben – selbstverständlich befindet sich, wenn $R_1$ von Carboxylat verschieden ist, die Verbindung im Zustand des Halogenids oder des Sulfonats –, die Symbole $R_a$, $R_b$ und $R_c$ die obige Bedeutung haben oder $R_a$ ein geschützter Carboxyrest ist und Hal ein Halogenatom ist, einwirken lässt und dann ggf. das erhaltene Sulfoxid reduziert, zutreffendenfalls die Schutzgruppen entfernt und ggf. die erhaltene Verbindung in ein Additionssalz mit einer Säure oder, falls es sie gibt, in ein Metallsalz oder ein Additionssalz mit einer Stickstoffbase umwandelt, oder dass man, wenn A einen Rest −NHCO− darstellt, das entsprechende Amin der allgemeinen Formel:

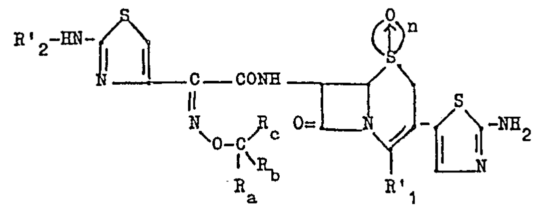

in welcher $R_a$, $R_b$, $R_c$, $R'_1$ und n die obige Bedeutung haben und $R'_2$ eine Aminschutzgruppe darstellt, nach irgendeiner bekannten Methode zur Bildung einer Aminfunktion, wobei der Rest des Moleküls nicht angegriffen wird, umwandelt, dann ggf. das erhaltene Sulfoxid reduziert, die Schutzgruppen entfernt und die erhaltene Verbindung ggf. in ein Additionssalz mit einer Säure oder, falls es sie gibt, in ein Metallsalz oder ein Additionssalz mit einer Stickstoffbase überführt.

**Claims** for the Contrating States BE, CH, DE, FR, GB, IT, LI, LU, NL, SE

1. A cephalosporin derivative, characterised in that it corresponds to the general formula:

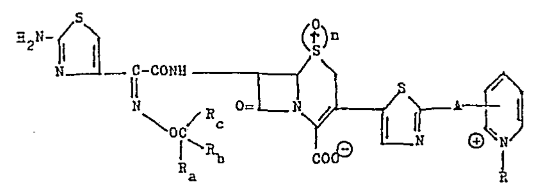

in which the symbol A denotes a single bond or a bivalent radical chosen from $-CH_2-$, −NH− or −NHCO− attached in position 3- or 4- of the pyridinium radical, the symbol R denotes a methyl, carboxymethyl, carbamoylmethyl, benzyl or allyl radical, the symbols $R_a$, $R_b$ and $R_c$ each denote a hydrogen atom, or alternatively the symbol $R_a$ denotes a carboxy radical and the symbols $R_b$ and $R_c$ are identical or different and denote hydrogen atoms or alkyl radicals containing 1 to 4 carbon atoms, or together form an alkylene radical containing 2 to 5 carbon atoms and n equals 0 or 1, in its syn form, as well as its addition salts with acids, and its metal salts or its addition salts with nitrogenous bases where these salts exist.

2. A cephalosporin derivative according to Claim 1, characterised in that the symbol A denotes a single bond or a bivalent radical chosen from $-CH_2-$, −NH− or −NHCO− attached in position 3- or 4- of the pyridinium radical, the symbol R denotes a methyl, carboxymethyl, carbamoylmethyl or benzyl radical, the symbols $R_a$, $R_b$ and $R_c$ each denote a hydrogen atom, or alternatively the symbol $R_a$ denotes a carboxy radical and the symbols $R_b$ and $R_c$ denote hydrogen atoms or methyl radicals, and n equals 0 or 1, as well as its addition salts with acids and its metal salts or its

addition salts with nitrogenous bases where these salts exist.

3. A cephalosporin derivative according to Claim 1, characterised in that the symbol A is a single bond or an −NH− radical attached in position 3- of the pyridinium radical, the symbol R denotes a methyl, carboxymethyl or carbamoylmethyl radical, the symbols $R_a$, $R_b$ and $R_c$ denote hydrogen atoms, or alternatively the symbol $R_a$ denotes a carboxy radical and the symbols $R_b$ and $R_c$ denote hydrogen atoms or methyl radicals and n equals 0, as well as its addition salts with acids and its metal salts or its addition salts with nitrogenous bases where these salts exist.

4. A process for preparing a product according to Claim 1, characterised in that an acid of general formula:

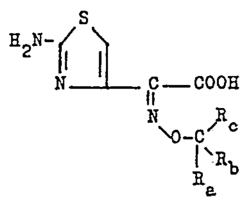

in which $R_a$, $R_b$ and $R_c$ are as defined in Claim 1, and in which the amine function and, where appropriate, the carboxy radical denoted by $R_a$ are protected beforehand, or a reactive derivative of this acid, is reacted with a 7-aminocephalosporin or one of its salts, of general formula:

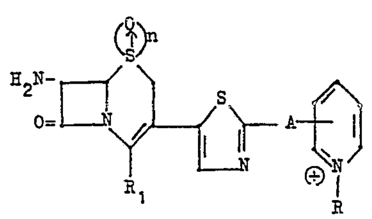

in which the symbols A and n are as defined in Claim 1, the symbol R is as defined in Claim 1 or denotes a protected carboxymethyl radical, and the symbol $R_1$ is a carboxylate radical or a free or protected carboxy radical (with the understanding that when $R_1$ is other than a carboxylate radical, the product is in the halide or sulphonate state), the sulphoxide is then optionally reduced, the protective radicals are removed and the product obtained is optionally converted to an addition salt with an acid or, where these exist, to a metal salt or an addition salt with a nitrogenous base.

5. A process for preparing a product according to Claim 1, characterised in that a halide of structure R−X, in which R is as defined in Claim 1 or denotes a protected carboxymethyl radical, and the symbol X denotes a halogen atom or an alkylsulphonyloxy radical (in which the alkyl part which contains 1 to 4 carbon atoms can be substituted with one or more halogen atoms) or phenylsulphonyloxy radical (in which the phenyl radical can optionally be substituted with one or more substituents chosen from halogen atoms or alkyl or nitro radicals), is reacted with a cephalosporin derivative of general formula:

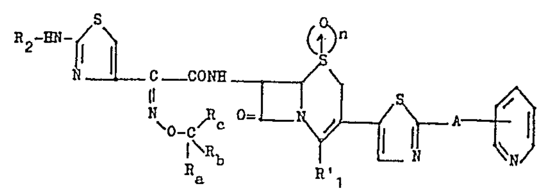

in which A, $R_a$, $R_b$, $R_c$ and n are as defined in Claim 1, with the understanding that when $R_a$ denotes a carboxy radical it can be free or protected, the symbol $R'_1$ is a free or protected carboxy radical and the symbol $R_2$ denotes a hydrogen atom or an amino-protective radical, the sulphoxide obtained is then optionally reduced, the protective radicals are removed and the product obtained is optionally converted to an addition salt with an acid or, where these exist, to a metal salt or an addition salt with a nitrogenous base.

6. A process for preparing a product according to Claim 1, characterised in that a thio-uree of general formula:

$$R_2NH-CS-NH_2$$

in which $R_2$ is a hydrogen atom or an amino-protective radical, is reacted with a cephalosporin derivative of general formula:

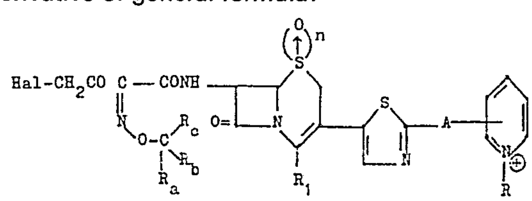

in which the symbols A, R, $R_1$ and n are as defined in Claim 4, with the understanding that, when $R_1$ is other than carboxylate, the product is in the halide or sulphonate state, the symbols $R_a$, $R_b$ and $R_c$ are as defined in Claim 1 or $R_a$ is a protected carboxy radical and Hal is a halogen atom, the sulphoxide obtained is optionally reduced, where appropriate the protective radicals are removed and the product obtained is optionally converted to an addition salt with an acid or, where these exist, to a metal salt or an addition salt with a nitrogenous base.

7. A process for preparing a product according to Claim 1, for which A denotes an −NHCO− radical, characterised in that the corresponding amine of general formula:

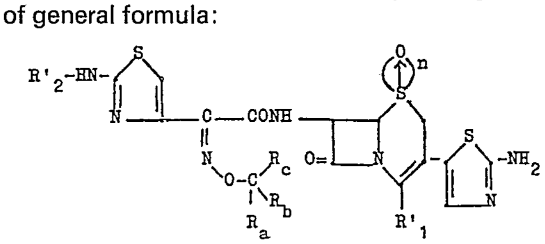

in which $R_a$, $R_b$, $R_c$, $R'_1$ and n are as defined in Claim 5 and $R'_2$ denotes an amino-protective radical, is converted by any known method for forming an amide group, without affecting the remainder of the molecule, the sulphoxide obtained is optionally reduced, the protective radicals are removed and the product obtained is optionally converted to an addition salt with an acid or, where these

exist, to a metal salt or an addition salt with a nitrogenous base.

8. Pharmaceutical composition, characterised in that it contains at least one product according to Claim 1, in the pure state, or in combination with one or more compatible and pharmaceutically acceptable diluents or adjuvants.

**Claim** for the Contracting State: AT

Process for preparing a cephalosporin derivative, characterised in that it corresponds to the general formula:

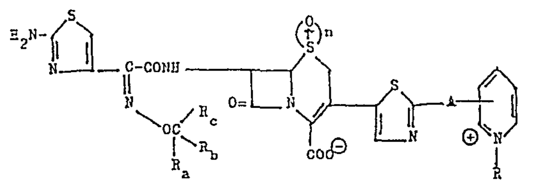

in which the symbol A denotes a single bond or a bivalent radical chosen from $-CH_2-$, $-NH-$ or $-NHCO-$ attached in position 3- or 4- of the pyridinium radical, the symbol R denotes a methyl, carboxymethyl, carbamoylmethyl, benzyl or allyl radical, the symbols $R_a$, $R_b$ and $R_c$ each denote a hydrogen atom, or alternatively the symbol $R_a$ denotes a carboxy radical and the symbols $R_b$ and $R_c$ are identical or different and denote hydrogen atoms or alkyl radicals containing 1 to 4 carbon atoms, or together form an alkylene radical containing 2 to 5 carbon atoms and n equals 0 or 1, in its syn form, as well as its addition salts with acids, and its metal salts or its addition salts with nitrogenous bases where these salts exist, characterised in that an acid of the general formula:

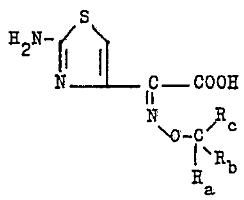

in which $R_a$, $R_b$ and $R_c$ are as defined above, and in which the amine function and, where appropriate, the carboxy radical denoted by $R_a$ are protected beforehand, or a reactive derivative of this acid, is reacted with a 7-aminocephalosporin or one of its salts, of general formula:

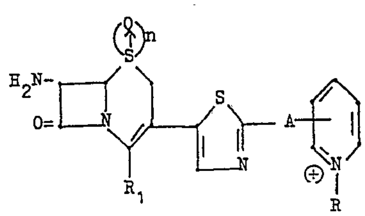

in which the symbols A and n are as defined above, the symbol R is as defined above or denotes a protected carboxymethyl radical and the symbol $R_1$ is a carboxylate radical or a free or protected carboxy radical (with the understanding that when $R_1$ is other than a carboxylate radical, the product is in the halide or sulphonate state), the sulphoxide is then optionally reduced, the protective radicals are removed and the product obtained is optionally converted to an addition salt with an acid or, where these exist, to a metal salt or an addition salt with a nitrogenous base, or alternatively a halide of structure R−X, in which R is as defined above or denotes a protected carboxymethyl radical, and the symbol X denotes a halogen atom or an alkylsulphonyloxy radical (in which the alkyl part which contains 1 to 4 carbon atoms can be substituted with one ore more halogen atoms) or phenylsulphonyloxy radical (in which the phenyl radical can optionally be substituted with one or more substituents chosen from halogen atoms or alkyl or nitro radicals), is reacted with a cephalosporin derivative of general formula:

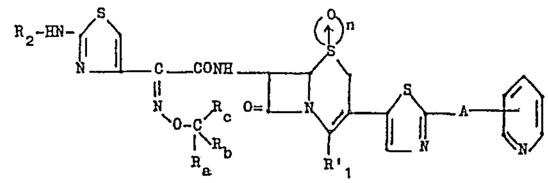

in which A, $R_a$, $R_b$, $R_c$ and n are as defined above, with the understanding that when $R_a$ denotes a carboxy radical it can be free or protected, the symbol $R'_1$ is a free or protected carboxy radical and the symbol $R_2$ denotes a hydrogen atom or an amino-protective radical, the sulphoxide obtained is then optionally reduced, the protective radicals are removed and the product obtained is optionally converted to an addition salt with an acid or, where these exist, to a metal salt or an addition salt with a nitrogenous base, or alternatively a thio-urea of general formula:

$$R_2NH-CS-NH_2$$

in which $R_2$ is a hydrogen atom or an amino-protective radical, is reacted with a cephalosporin derivative of general formula:

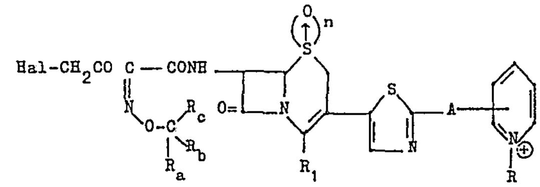

in which the symbols A, R, $R_1$ and n are as defined above, with the understanding that, when $R_1$ is other than carboxylate, the product is in the halide or sulphonate state, the symbols $R_a$, $R_b$ and $R_c$ are as defined above or $R_a$ is a protected carboxy radical and Hal is a halogen atom, the sulphoxide obtained is then optionally reduced, where appropriate the protective radicals are removed and the product obtained is optionally converted to an addition salt with an acid or, where these exist, to a metal salt or an addition salt with a nitrogenous base, or alternatively when A denotes a $-NHCO-$ radical, the corresponding amine of general formula:

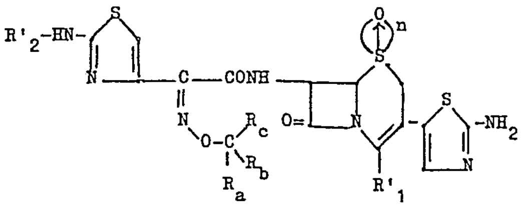

in which $R_a$, $R_b$, $R_c$, $R'_1$ and n are as defined above and $R'_2$ denotes an amino-protective radical, is converted by any known method for forming an amide group, without affecting the remainder of the molecule, the sulphoxide obtained is then optionally reduced, the protective radicals are removed and the product obtained is optionally converted to an addition salt with an acid or, where these exist, to a metal salt or an addition salt with a nitrogenous base.